# EUROPEAN PATENT SPECIFICATION

(11) **EP 2 799 537 B1**
(45) Date of publication and mention of the grant of the patent: **22.09.2021**
(21) Application number: 12862290.9
(22) Date of filing: 27.12.2012
(51) Int. Cl.: C12N 5/079, A61K 9/70, A61K 31/4409, A61K 31/4418, A61K 31/4439, A61K 31/4709, A61K 31/519, A61K 31/713, A61K 35/30, A01N 1/02, A61P 43/00

(54) **NORMALIZATION OF CULTURE OF CORNEAL ENDOTHELIAL CELLS**
NORMALISIERUNG DER ZÜCHTUNG VON HORNHAUTENDOTHELZELLEN
NORMALISATION D'UNE CULTURE DE CELLULES ENDOTHÉLIALES DE LA CORNÉE

(30) Priority: 28.12.2011 JP 2011289666; 15.02.2012 JP 2012030969; 05.07.2012 JP 2012151340
(43) Date of publication of application: 05.11.2014
(60) Divisional application: 19172604.1
(73) Proprietor: Kyoto Prefectural Public University Corporation, Kyoto 602-8566 (JP); ActualEyes Inc., Kyotanabe-city, Kyoto (JP); CorneaGen, Inc., Seattle, WA 98101 (US)
(72) Inventor: KINOSHITA, Shigeru, Kyoto-shi Kyoto 602-8566 (JP); KOIZUMI, Noriko, Kyotanabe-shi Kyoto 610-0394 (JP); OKUMURA, Naoki, Kyotanabe-shi Kyoto 610-0394 (JP)
(74) Representative: Engelhard, Markus
(86) International application number: PCT/JP2012/084320
(87) International publication number: WO 2013/100208

(56) References cited:
- EP-A1- 1 726 639
- EP-A1- 2 193 806
- WO-A1-2005/038015
- US-A1- 2006 234 911
- OKUMURA N ET AL: "Inhibition of TGF-[beta] Signaling Enables Human Corneal Endothelial Cell Expansion In Vitro for Use in Regenerative Medicine", PLOS ONE, vol. 8, no. 2, 25 February 2013 (2013-02-25), page e58000, XP055229718, DOI: 10.1371/journal.pone.0058000
- OKUMURA N ET AL: "Cell-based approach for treatment of corneal endothelial dysfunction", CORNEA, vol. 33, no. Suppl. 11, November 2014 (2014-11), pages S37-S41, XP008178140, ISSN: 1536-4798, DOI: 10.1097/ICO.0000000000000229
- FUNAKI T. ET AL.: 'Smad7 suppresses the inhibitory effect of TGF-p2 on corneal endothelial cell proliferation and accelerates corneal endothelial wound closure in vitro.' CORNEA vol. 22, no. 2, 2003, pages 153 - 159, XP008174412
- KOIZUMI N. ET AL.: 'Cultivated Corneal Endothelial Cell Sheet Transplantation in a Primate Model.' INVESTIGATIVE OPHTHALMOLOGY & VISUAL SCIENCE vol. 48, no. 10, 2007, pages 4519 - 4526, XP008164101
- OKUMURA N. ET AL.: 'Enhancement on Primate Corneal Endothelial Cell Survival in Vitro by a ROCK Inhibitor.' INVESTIGATIVE OPHTHALMOLOGY & VISUAL SCIENCE vol. 50, no. 8, 2009, pages 3680 - 3687, XP009144489
- NORIKO KOIZUMI: 'Cultivated Corneal Endothelial Cell Sheet Transplantation in a Primate Model' JOURNAL OF JAPANESE OPHTHALMOLOGICAL SOCIETY vol. 113, 2009, pages 1050 - 1059, XP008164101
- NAOKI OKUMURA: 'Shudankai Shoroku 3. Enhancement on Primate Corneal Endothelial Cell Survival in Vitro by a ROCK Inhibitor' JOURNAL OF KYOTO PREFECTURAL UNIVERSITY OF MEDICINE vol. 119, no. 2, 2010, pages 125 - 126, XP008174825
- PETROLL W.M. ET AL.: 'Myofibroblast transformation of cat corneal endothelium by transforming growth factor-beta1, -beta2, and -beta3.' INVESTIGATIVE OPHTHALMOLOGY & VISUAL SCIENCE vol. 39, no. 11, 1998, pages 2018 - 2032, XP055077410
- SUMIOKA T. ET AL.: 'Inhibitory effect of blocking TGF-§/Smad signal on injury-induced fibrosis of corneal endothelium.' MOLECULAR VISION vol. 14, 2008, pages 2272 - 2281, XP008148116
- SAIKA S. ET AL.: 'Inhibition of p38MAP kinase suppresses fibrotic reaction of retinal pigment epithelial cells.' LABORATORY INVESTIGATION vol. 85, 2005, pages 838 - 850, XP055077411
- JOYCE N.C.: 'Proliferative capacity of the corneal endothelium.' PROGRESS IN RETINAL AND EYE RESEARCH vol. 22, 2003, pages 359 - 389, XP055078038
- SAIKA S.: 'TGF beta pathobiology in the eye.' LABORATORY INVESTIGATION vol. 86, 2006, pages 106 - 115, XP008114631
- SAIKA S. ET AL.: 'Fibrotic disorders in the eye: Targets of gene therapy.' PROGRESS IN RETINAL AND EYE RESEARCH vol. 27, no. 2, 2008, pages 177 - 196, XP022518381
- None

## Description

### [Technical Field]

The present invention is directed to a technique and a method for culturing a corneal endothelial cell in a normal state.

### [Background Art]

Visual information is recognized in such a manner that light into a cornea, which is a transparent tissue at the forefront of an eyeball, reaches a retina, stimulating the nerve cell of the retina, and an electric signal generated is transferred through the optic nerve to the visual cortex of the cerebrum. In order to obtain favorable eyesight, the cornea needs to be transparent. The transparency of the cornea is retained by the corneal endothelial cells which functions as a pump and barrier to maintain constant moisture content.

Human corneal endothelial cells are present at the density of about 3,000 per 1 square millimeter at birth. However, once the corneal endothelial cells are damaged, they do not have the capability to regenerate themselves. In endothelial corneal dystrophy or bullous keratopathy, which is caused by dysfunction of the corneal endothelium due to various causes, the cornea becomes opaque due to edema, resulting in significant loss of vision. Currently, perforating keratoplasty is performed on bullous keratopathy, where all the three layers, i.e., epidermis, stroma and endothelium, of the cornea are transplanted. However, donation of corneas is insufficient in Japan, and the number of corneal transplant performed in the country is about 1,700 per year while there are about 2,600 patients who are on the waiting list for corneal transplant.

In recent years, with the objective of reducing the risk of rejection response or postoperative complications and obtaining better visual performance, the concept of "part transplant" is gaining attention, where only a damaged tissue is transplanted. Among the types of corneal transplants, a transplant of stroma tissues, i.e., Deep Lamellar Keratoplasty, a transplant of corneal endothelial tissues, i.e., Descemet's Stripping Automated Endothelial Keratoplasty, and the like are starting to be performed. Further, cultured mucosal epithelium transplantation has already been clinically applied, where corneal epithelium or oral mucous membrane that is cultured ex vivo is transplanted instead of corneal epithelium. A method for transplanting corneal endothelium cultured ex *vivo* is also taken into consideration. Corneal endothelium-like sheets, consisting of a corneal endothelial layer which is cultured on a collagen layer, are known for use in the transplant of corneal endothelium (see Patent Literature 1) . However, as to corneal endothelial cells, and in particular human-derived corneal endothelial cells, the donors of corneas are limited, and the culturing is difficult *in vitro.* Thus, time and cost are required to obtain the amount of cultured cells necessary for transplant.

Human embryonic stem (ES) cells have both high ability for self-replication and multipotency, gaining attention as a form of medical application. However, human ES cells easily cause cell death due to an operation of dispersing the cells during a culturing process. Thus, there has been a problem of significant reduction in the number of cells on the practical side. In recent years, it has been found that cell death caused when human ES cells are cultured is caused by activation of Rho kinase (ROCK), and that inhibition of ROCK greatly suppresses cell death; and it has been reported that it is possible to mass culture human ES cells and produce cerebral cells using a ROCK inhibitor, Y-27632, or the like (Non Patent Literature 1). Accordingly, the inventors have disclosed a method of mass culture of corneal endothelial cells using Y-27632 or the like (Patent Literature 2).

Besides this, Patent Literature 3 discloses a neurosphere method using corneal endothelial precursor cells.

Patent Literature 4 discloses use of a TGF-β kinase inhibitor and a p38 MAPK inhibitor for culturing epithelial cells.

In addition, Non Patent Literatures 2 and 4 describe involvement of TGF-β, p38 MAPK and Smad in a specific severe corneal endothelial disease. Non Patent Literature 3 describes prospects of the growth of human corneal endothelial cells using a ROCK inhibitor. Non Patent Literature 5 indicates that fibrosis during a severe disorder of cornea is due to IL-1β, and due to activation of p38 MAPK during the course thereof. Non Patent Literature 6 indicates that fibrosis present at excess external injury due to freezing with rabbits is suppressed using an inhibitor with activation of p38 MAPK. Non Patent Literature 7 describes that in conventional corneal endothelial cell culture media, if subculturing occurs, growth while maintaining a normal state becomes impossible. Non Patent Literature 8 discloses a culture medium for corneal endothelial cells. It is described that this culture medium includes FBS, EGF and NGF, and no favorable culturing can be performed in this culture medium if cells to be cultured are not derived from organisms at their young age. Non Patent Literature 9 discloses a culture medium for corneal endothelial cells using basic FGF. Non Patent Literature 10 discloses a culture medium for corneal endothelial cells using collagenase. Non Patent Literature 11 discloses a culture medium for corneal endothelial cells using a conditioned culture medium. Various types of culture media are developed as in Non Patent Literatures 8 to 11; however, as indicated in Non Patent Literature 7, it is known that in conventional corneal endothelial cell culture media, if subculturing occurs, growth becomes impossible while maintaining a normal state. Non Patent Literatures 12 to 14 also describe manufacture of a cultured corneal endothelial sheet. Non Patent Literatures 9 to 12 and 15 disclose human ocular tissue-derived stem cell and autocorneal endothelial transplantation. Non Patent Literatures 16 and 17 also describe manufacture of a cultured corneal endothelial sheet.

### [Citation List]

### [Patent Literature]

Patent Literature 1: Japanese Laid-Open Publication No. 2005-229869
Patent Literature 2: International Publication No. WO 2009/28631
Patent Literature 3: Japanese Laid-Open Publication No. 2006-187281
Patent Literature 4: US Patent Application Publication No. 2006/0234911

### [Non Patent Literature]

Non Patent Literature 1: Watanabe K., et al., Nat Biotechnol.2007, 25, pp 681
Non Patent Literature 2: Saika, Dai 324 Kai Kansai Ganshikkan Kenkyukai Tokubetsu Koen [Special Lecture in 324th Kansai Ocular Disease Research Group] "Jouhi-Kanyoukei Ikou To Ganshikkan [Epithelium-Mesenchymal System Transition and Ocular Disease]" June 23, 2010, http://ohpth.kpu-m.ac.jp/wp-content/uploads/2010/07/tit le20100623.doc
Non Patent Literature 3: Koizumi, Saisentan • Jisedai Kenkyu Kaihatsu Shien Program [Leading-edge • Next-Generation Research and Development Support Program http://www.jsps.go.jp/j-jisedai/data/life/LS117_outline .pdf
Non Patent Literature 4 : Sumioka T., et al., Molecular Vision 2008; 14:2272-2281
Non Patent Literature 5: Lee JG., et al., Invest Ophthalmol Vis Sci. 2009; 50: 2067-2076
Non Patent Literature 6: Song SK., et al, Invest Ophthalmol Vis Sci.2010; 51: 822-829)
Non Patent Literature 7: Peh GS., et al., ARVO 2011, 561 Corenal Endothelium: Health and Diseases 6595 (May 1-5,2011) Non Patent Literature 8: Nancy C. Joyce, et al., Cornea 2004; 23(Suppl.1) : S8-S19)
Non Patent Literature 9: MiyataK., et al., Cornea 20(1):59-63,2001
Non Patent Literature 10: Wei Li, et al., Invest Ophthalmol Vis Sci. 2007; 48:614-620
Non Patent Literature 11: Xiaoyan Lu, et al., Molecular Vision 2010; 16:611-622
Non Patent Literature 12: Mimura T., et al. , Invest Ophthalmol Vis Sci. 2004 Sep; 45(9): 2992-2997
Non Patent Literature 13: Mimura T., et al., Exp Eye Res. 2003 Jun; 76(6): 745-751
Non Patent Literature 14: Yokoo S., et al., Invest Ophthalmol Vis Sci. 2005 May; 46(5): 1626-1631
Non Patent Literature 15: Amano S et al., Curr Eye Res. 2003 Jun; 26(6):313-318
Non Patent Literature 16: Ide T et al., Biomaterials. 2006 Feb; 27(4): 607-14. Epub 2005 Aug 15.
Non Patent Literature 17: Sumide T et al., FASEB J. 2006 Feb; 20(2):392-4. Epub 2005 Dec 9.

### [Summary of Invention]

### [Solution to Problem]

The inventors have found a technique which makes it possible to grow corneal endothelial cells while maintaining their normal functions by inhibiting tumor necrosis factor β (TGF-β) pathway. As a result, it has become possible to grow a relatively large amount of corneal endothelial cells which have normal functions.

### [Advantageous Effects of Invention]

The present invention provides a technique that is capable of growing a corneal endothelial cell while maintaining its normal functions, which was difficult to achieve before. The normal functions include biochemical functions of corneal endothelial cells such as ZO-1 and Na⁺/K⁺-ATPase, transplantability to primates and the like, and encompass functions for achieving corneal transplant.

### [Brief Description of Drawings]

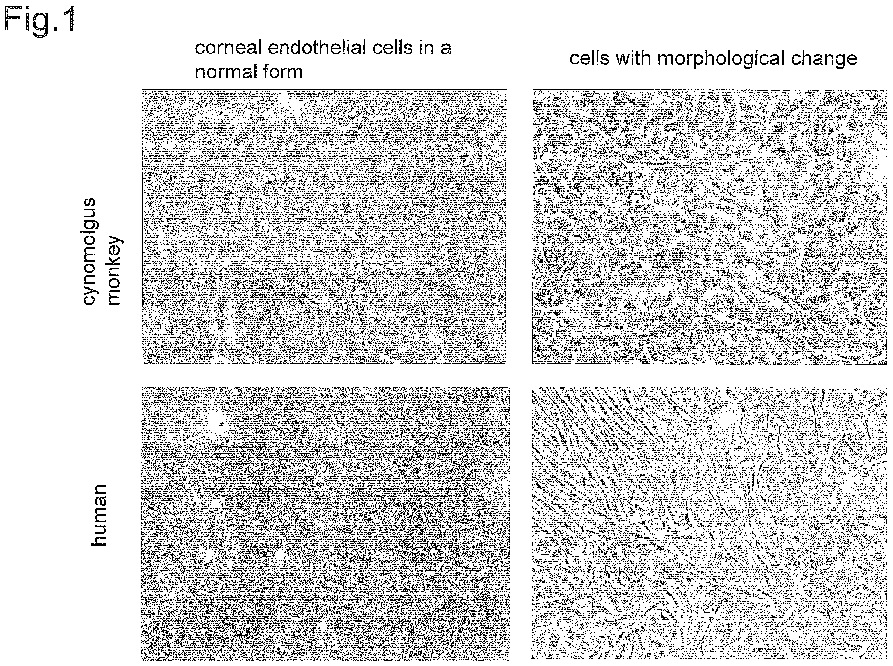

[Figure 1] Figure **1** shows a morphological change in culturing cells of cynomolgus monkey and humans in a conventional method. The upper side shows the morphological change in cynomolgus monkeys and the lower side shows the morphological change in humans. The culturing result is under the conditions of DMEM + 10% FBS + 2 ng/ml basic FGF for the cynomolgus monkeys, while the culturing result for the humans is under the conditions of Opti-MEM I Reduced-Serum Medium, Liquid + 8% FBS + 200 mg/ml CaCl₂•2H₂O+0.08% chondroitin sulfuric acid +20µg/ml ascorbic acid + 50µg/ml gentamicin + 5ng/ml EGF. The left side shows corneal endothelium cells in a normal form, but a morphological change is easily produced by long-term culturing or subculturing as shown on the right side.

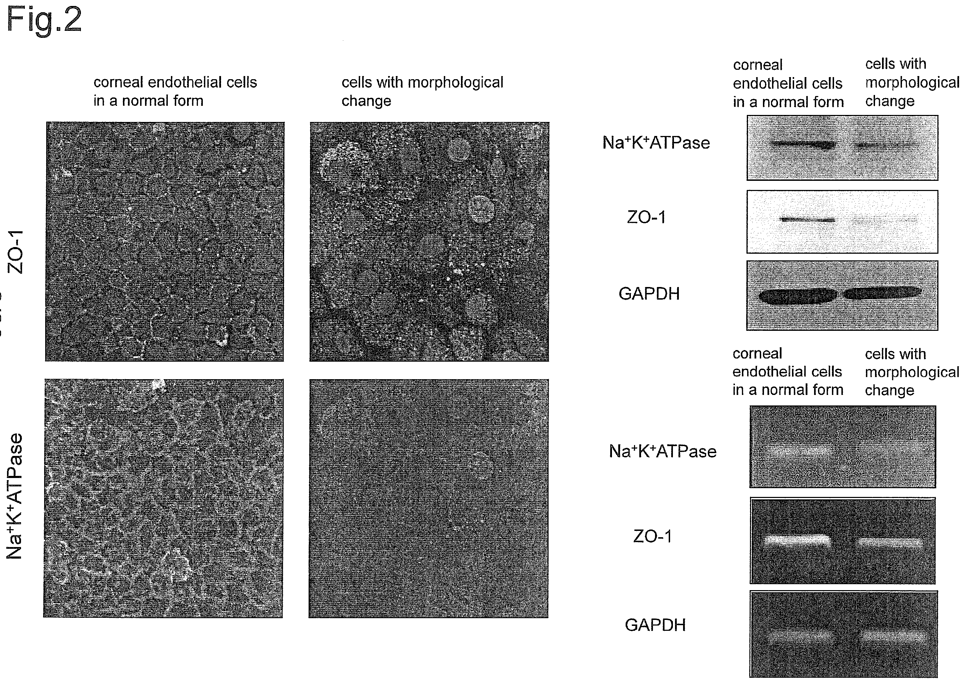

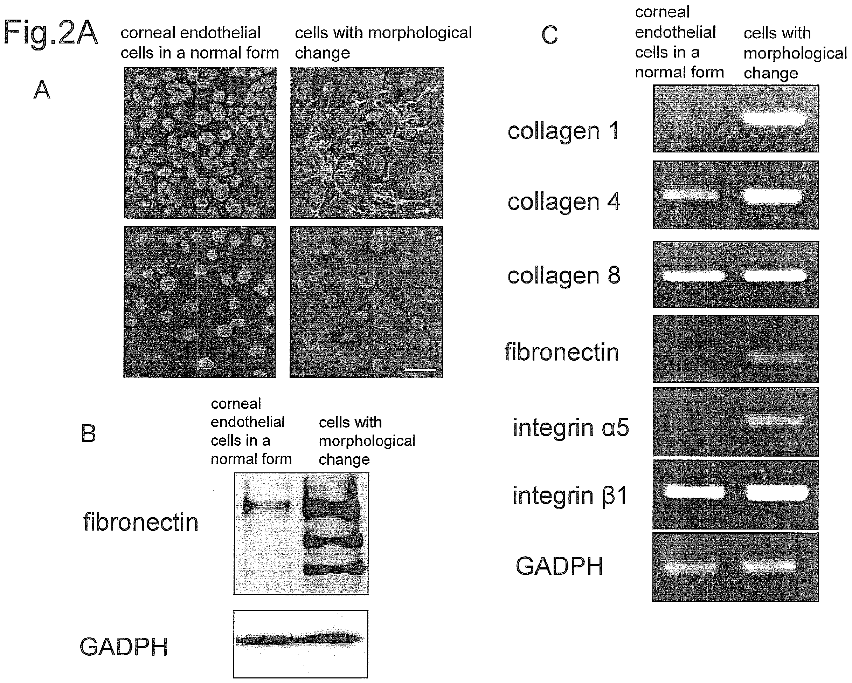

[Figure 2] Figure **2** shows that a normal function is lost in culturing based on prior art. The left two panels show immunostaining result. The far left panel shows a monkey corneal endothelial cell (MCEC) which was cultured into a normal form, and second panel from the left shows a MCEC that was morphologically changed into a fibroblastic phenotype due to long-term culturing. The top two images of the immunostaining results show staining with ZO-1while the bottom two images show staining with Na⁺/K⁺-ATPase. The top right panel shows results obtained using Western blot. The bottom right panel shows results using real-time PCR. In both the top right panel and bottom right panel, the left side show a MCEC which was cultured normally, while the right side shows a MCEC morphologically changed to a fibroblastic phenotype by conventional long-term culturing. For Western blot and real-time PCR results, staining is shown with an antibody or probe directed to Na⁺/K⁺-ATPase, ZO-1 and GAPDH, starting from the top.
[Figure 2A] Figure **2A** shows a fibroblast primate corneal endothelial cell (CEC) generating an abnormal extracellular matrix, that is, the normal function is lost when cultured based on prior art. (A) shows the expression of fibronectin and collagen type 1 in cells with a fibroblast phenotype and a normal phenotype. The upper row shows fibronectin, and the lower row shows collagen type 1. The left side shows a normal cell phenotype, and the right side shows a fibroblast phenotype. The fibroblast phenotype indicated an excess extracellular matrix such as fibronectin and collagen type 1. On the other hand, the normal cell phenotype has completely lost its staining capacity. (B) shows Western blot showing the expression of fibronectin protein in cells with fibroblast phenotype and normal phenotype. The GAPDH is used as a control. The protein expression level of the fibronectin was more up-regulated in cells with the fibroblast phenotype than in cells with the normal phenotype. (C) shows semi-quantitative PCR results of the expression of collagen type 1, type 4 and type 8, fibronectin, integrin α5, and integrin β1 (listed in order from the top)in cells cells of a fibroblast phenotype (right) and a normal phenotype (left) of. GAPDH is used as a control. Through the semi-quantitative PCR analysis, type 1 collagen transcripts (α1(I)mRNA) were abundantly expressed in cells of the fibroblast phenotype, while the expression of α1(I)mRNAwas decreased in cells of the normal phenotype. While α1 (IV)mRNA and α1 (VIII) mRNA, which were a basement membrane collagen phenotype, were expressed in cells of the normal phenotype and the fibroblast phenotype, the degree of expression was smaller in cells of the normal phenotype than in the cells of the fibroblast phenotype. While mRNA of fibronectin and integrin α5 was observed in the fibroblast phenotype, the mRNA of the two types was not expressed in the cells of the normal phenotype. As for the mRNA of β1 integrin, a similar level of expression was observed in both phenotypes.

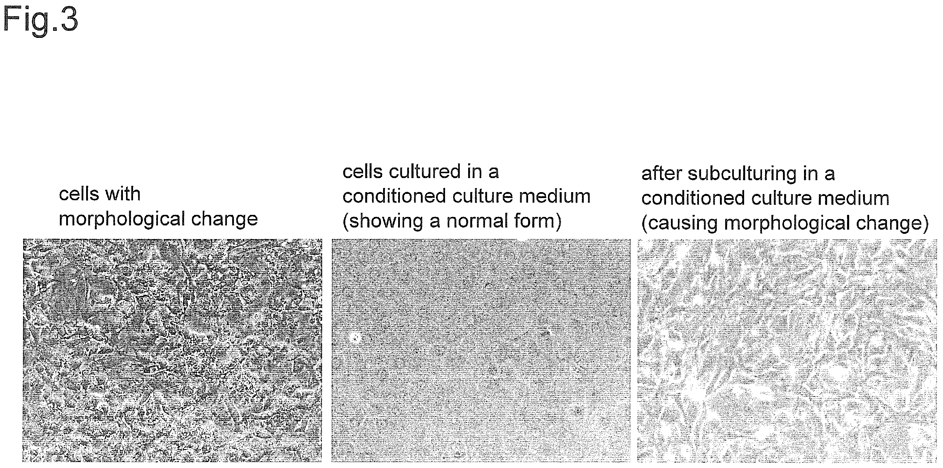

[Figure 3] Figure **3** shows fibrosis in a conventional method. A conditioned culture medium (Conditioned medium) for 3T3 feeder cells suppresses fibroblastic change (transformation) (center), but it is indicated that subculturing results in transformation after all (right) . The left side shows that when a human corneal endothelial cell was cultured under the conditions of Opti-MEM I Reduced-Serum Medium, Liquid + 8% FBS + 200mg/ml CaCl₂•2H₂O + 0.08% chondroitin sulfuric acid + 20µg/ml ascorbic acid + 50µg/ml gentamicin + 5ng/ml epithelial growth factor (EGF), the cell was transformed in a fibroblastic manner. The center shows a result with a similar culture medium as a conditioned culture medium using 3T3, which is a mouse-derived fibroblast. The right side is a photograph (obtained using a phase-contrast microscope) of a human corneal endothelial cell using 3T3 7 days after culturing and subculturing in a conditioned culture medium. The cells are an enlarged cells which were transformed to a fibroblast phenotype and are arranged in a multi-layered manner. As such, it is understood that fibrosis is generated when subculturing is performed in a conventional culture medium.

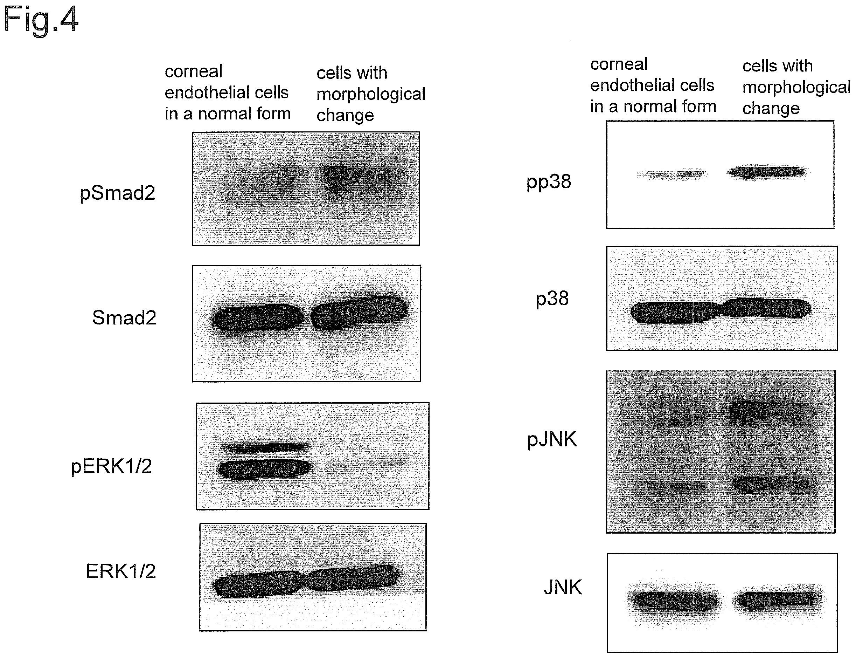

[Figure 4] Figure **4** shows Western blot results demonstrating that a Smad pathway, p38 MAPK pathway and JNK pathway are activated in a fibrotic cell of a monkey corneal endothelial cell. In each panel, the left side shows a monkey corneal endothelial cell (MCEC) in a normal phenotype, and the right side shows a MCEC which is morphologically changed to a fibroblast phenotype. The left panel shows Western blot results with antibodies directed to pSmad2, Smad2, pERK1/2 and ERK1/2 (listed from the top) . The right panel shows Western blot results with antibodies directed to pp38, p38, pJNK and JNK (listed from the top) . It is confirmed that different results maybe obtained in accordance with the state of growth of cells since the phosphorylation of ERK is influenced by not only the change due to fibrosis, but is also influenced by cell growth.

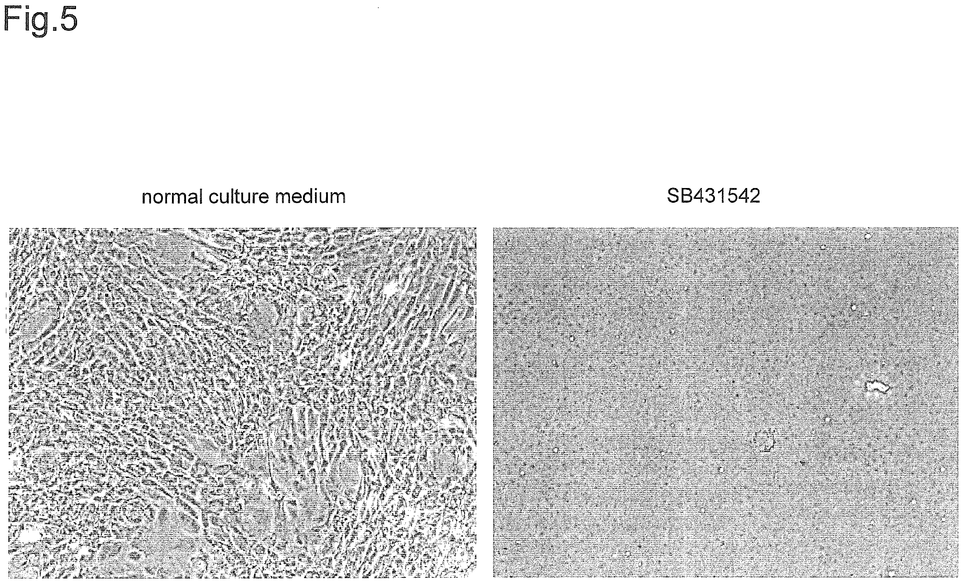

[Figure 5] Figure **5** shows that TGF-β signaling was inhibited by a phosphorylation inhibitor of a receptor, which was able to suppress the transformation of monkey corneal endothelium. The left image shows cell culture of a corneal endothelium from a cynomolgus monkey with DMEM + 10%FBS + 2ng/ml basic FGF (also referred to as a normal culture medium herein), which was morphologically changed to the fibroblast phenotype . The right image shows cell culture of a cornea from the same subject as the normal culture medium but with a phosphorylation inhibitor of a receptor(i.e.SB431542), where TGF-β signaling was inhibited, . A layer of polygonal cells with little difference in size are recognized, which allows one to understand that morphological change is suppressed in cells of a fibroblast phenotype.

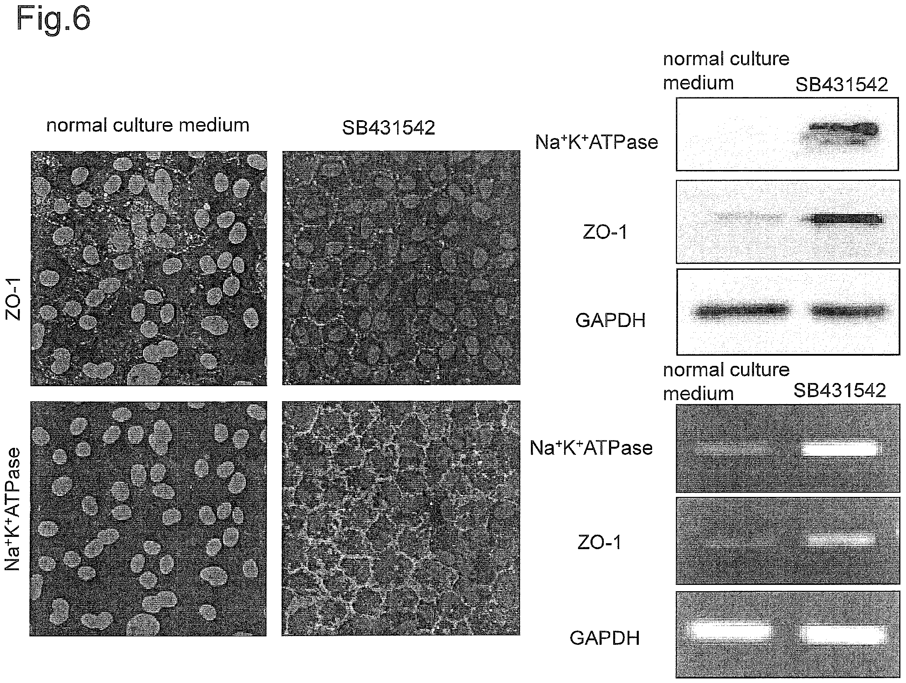

[Figure 6] Figure **6** shows that the loss of function-associated protein due to fibrosis of a monkey corneal endothelial cell was suppressed by TGF-β signal inhibition. The left panel shows a MCEC which was morphologically changed to the fibroblast phenotype when a corneal endothelium of a cynomolgus monkey was cultured with DMEM + 10%FBS + 2ng/ml basic FGF (normal culture medium). The second panel from the left of the immunostaining images show an immunostain by ZO-1 (top image) and Na⁺/K⁺-ATPase (bottom image), which are function-associated markers of MCEC that was treated with SB431542 and cultured into a normal phenotype. The upper right panel shows Western blot results . The lower right panel shows real-time PCR results . In both of the upper right panel and lower right panel, the left side shows a MCEC, which was treated with SB431542 and cultured into a normal phenotype, and the right side shows a MCEC which was morphologically changed to a fibroblast phenotype. For both Western blots and real-time PCR experiments, staining was conducted with an antibody or a probe directed to Na⁺/K⁺-ATPase, ZO-1, and GAPDH (listed in order from the top).

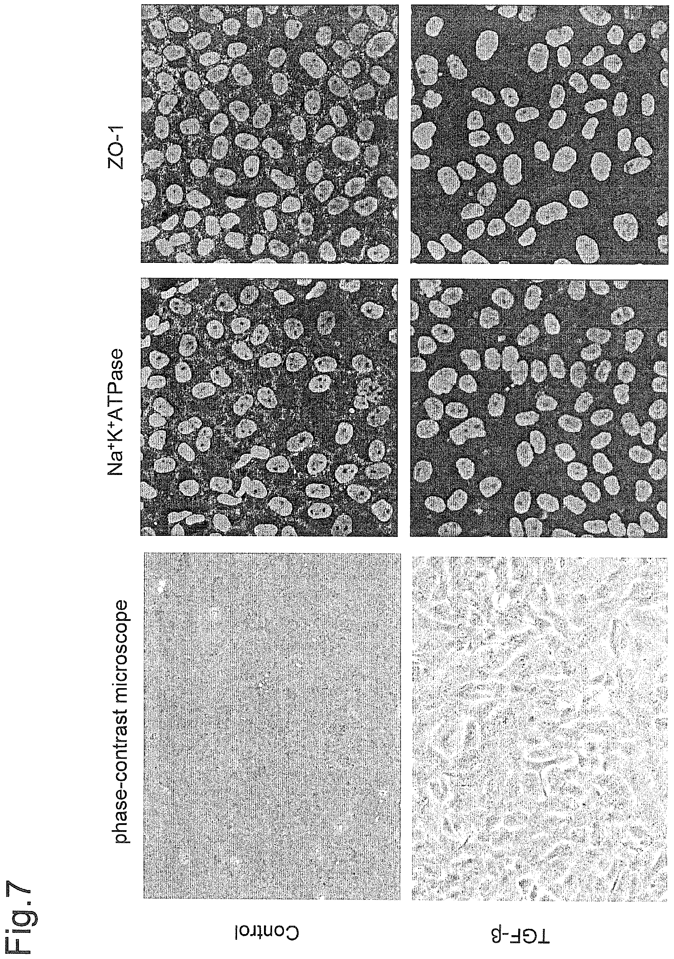

[Figure 7] Figure **7** is a diagram showing addition of TGF-β to induce transformation, thus losing a function-associated protein, in order to confirm that a TGF-β signal is associated with the transformation of a monkey corneal endothelium. Images in the top row shows MCEC of a control; and the bottom images shows result of cells treated with TGF-β. The left panel shows photographs of a cell form using a phase-contrast microscope. The center shows staining results with an antibody directed to Na⁺/K⁺-ATPase. The right side shows staining results with an antibody directed to ZO-1.

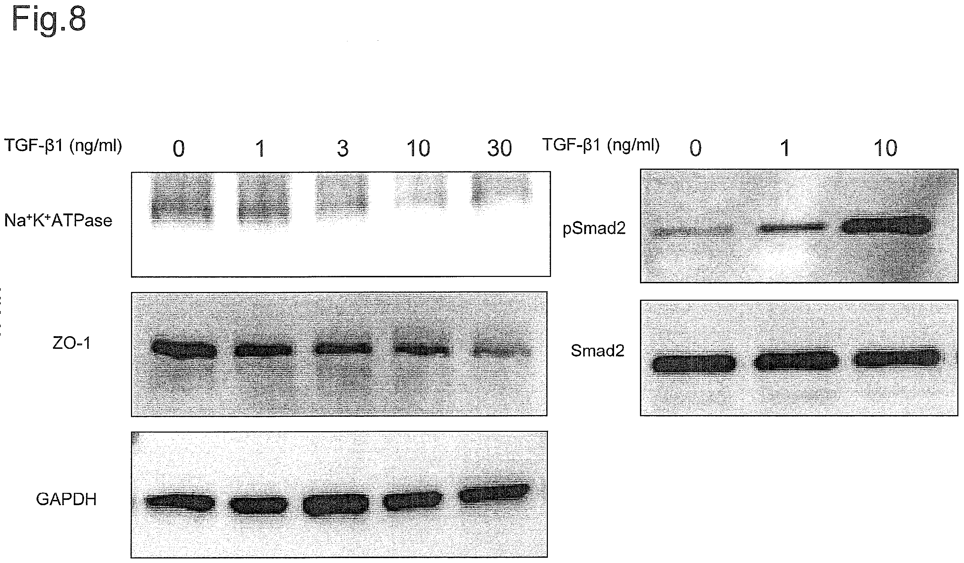

[Figure 8] Figure **8** is a diagram showing a monkey corneal endothelial cell becoming fibrotic and morphologically changed by TGF-β, thus losing a function-associated protein. The change is directly related to the concentration of TGF-β (in the left side panel, shown are 0ng/ml, 1ng/ml, 3ng/ml, 10ng/ml, and 30ng/ml from the left. In the right side panel, shown are 0ng/ml, 1ng/ml, and 10ng/ml from the left) . From the upper left panel, Western blot results are shown with antibodies directed to Na⁺/K⁺-ATPase, ZO-1, and GAPDH. On the right side, staining results are shown with antibodies directed to pSmad2 and Smad2 from the top.

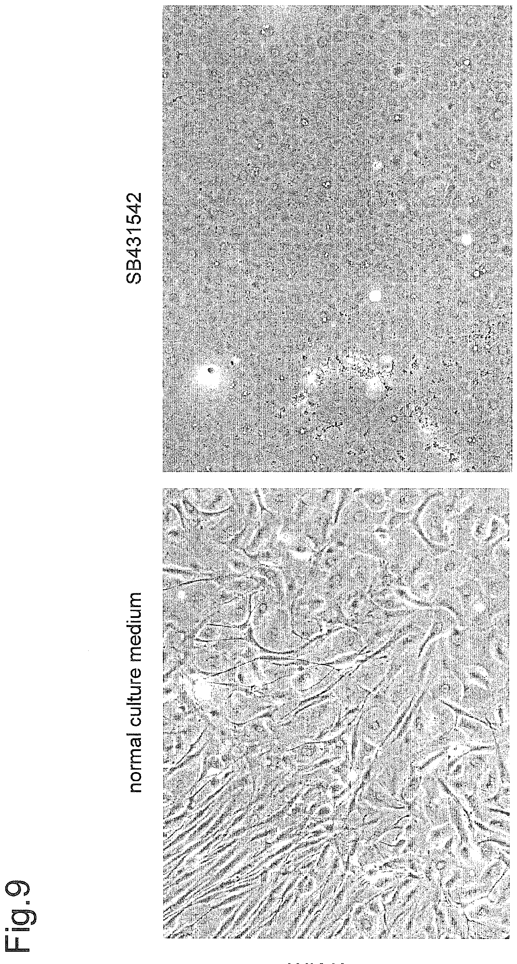

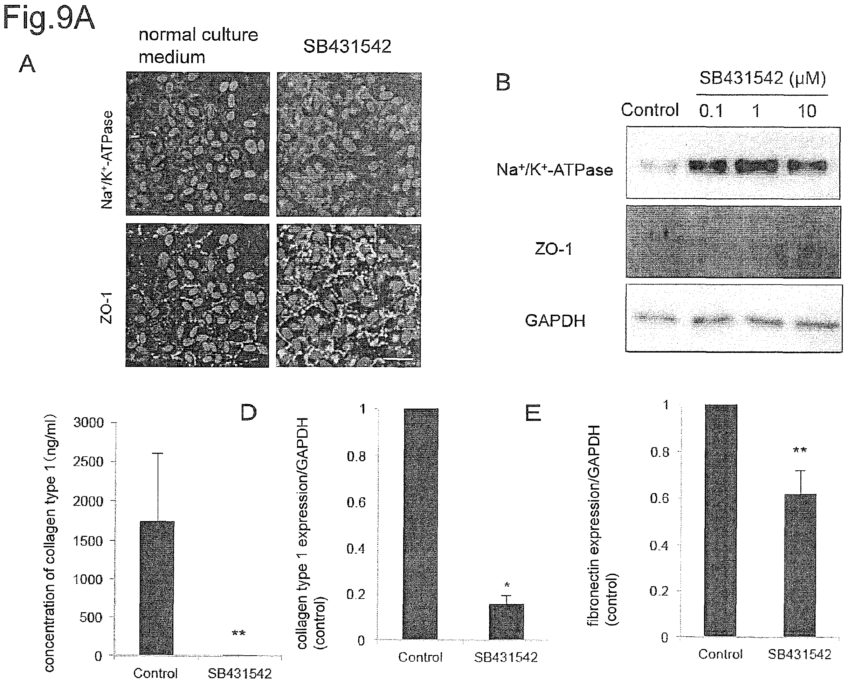

[Figure 9] Figure **9** shows results indicating that transformation is suppressed by inhibiting TGF-β signaling using a phosphorylation inhibitor of a receptor in a human corneal endothelium, thereby culturing a normal endothelium. The left side is a control (normal culture medium), and the right side shows a staining result with SB431542.
[Figure 9A] Figure **9A** shows that SB431542 maintains functions of a human corneal endothelial cell (HCEC) and suppresses the change in the human corneal endothelial cell to the fibroblast phenotype (A, B). In (A), the left side shows human corneal endothelial cells which was cultured in a normal culture medium, and the right side shows the same type of cells which was cultured in a normal culture medium with the addition of 1µM SB431542 to a. When immunostaining was performed with Na⁺/K⁺-ATPase(pumping function) and ZO-1(barrier function) which are function-associated markers for corneal endothelial cells, the expression of these markers was recognized in a subpopulation of cells in a normal culture medium, while the expression was recognized in all the cells in a culture medium to which SB431542 was added. The scale bar shows 100µm. In (B), results of Western blot are shown with three concentrations of SB431542 (10µM, 1µM and 0.1µM from the right) . The control indicates a culture medium to which nothing was added. The expression of Na⁺/K⁺-ATPase, ZO-1, and GAPDH (control) are shown (listed from the top). By blocking TGF receptor signaling using SB431542, intracellular localization became possible for Na⁺/K⁺-ATPase and ZO-1 in a cell membrane, and it became possible to maintain their protein expression. (C) shows ELISA assay. The concentration of collagen type 1 secreted in cell supernatant was measured in the absence (control, used as a reference) and presence of SB431542. The ELISA assay indicated that SB431542 significantly down-regulated the secretion of the collagen type 1 to the cell supernatant. **P<0.05. (D and E) show quantitative PCR results. In these graphs, the expression of collagen type 1 (D) and fibronectin (E) in the presence and absence of SB43152 (1µM) were normalized with GAPDH and the ratios are expressed relative to the control (i.e. control is 1) . These show that SB431542 significantly decreased the expression of the collagen type 1 and fibronectin at the mRNA level. *P<0.01, **P<0.05.

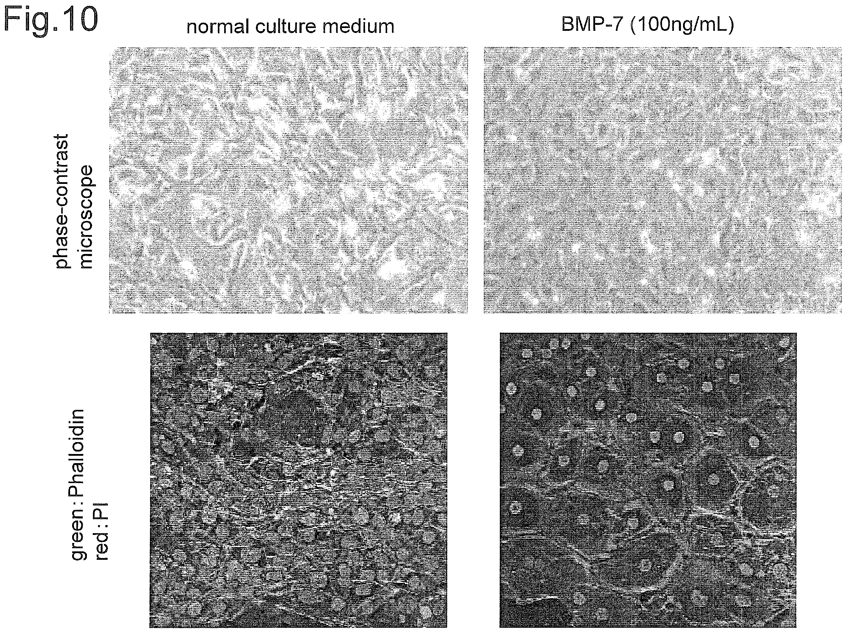

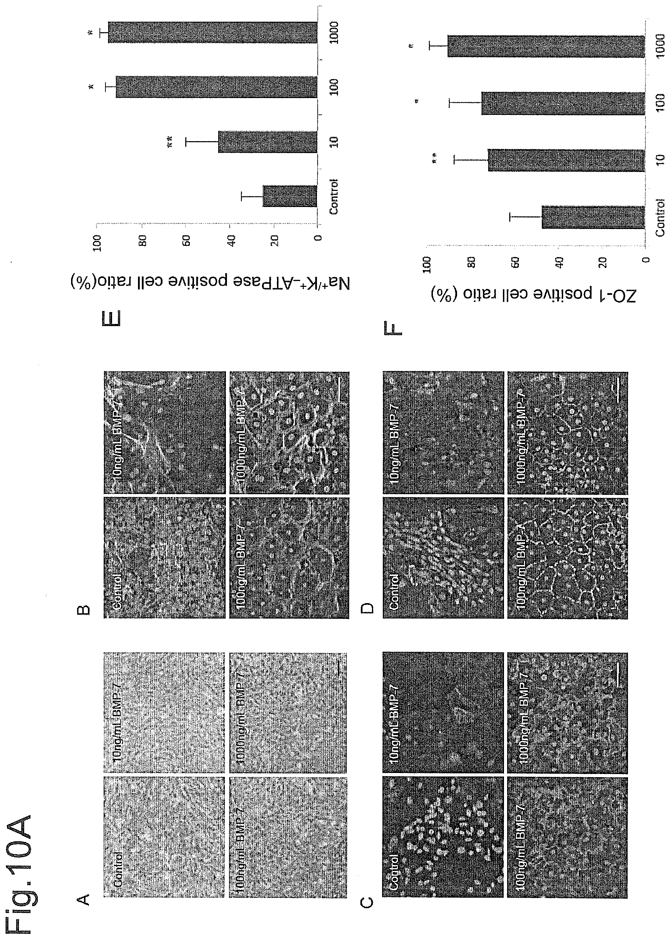

[Figure 10] Figure **10** is a diagram showing that a TGF-β signal was counteracted and transformation of a human corneal endothelium was suppressed in a method other than SB431542. The result on the top row shows was obtained using a phase-contrast microscope. The bottom row shows a result of phalloidin staining (the green staining (netlike appearance around the cells) is phalloidin, and red staining (particulate) is PI). The left panel is a human corneal endothelial cell which was cultured with Opti-MEM I Reduced-Serum Medium, Liquid + 8% FBS + 200mg/ml CaCl₂•2H₂O + 0.08% chondroitin sulfuric acid + 20µg/ml ascorbic acid + 50µg/ml gentamicin + 5ng/ml EGF as a culture medium (which is shown as a normal culture medium in the figure), and the right panel shows results of culturing with 100ng/mL BMP-7 added to the culture medium.
[Figure 10A] Figure **10A** shows the effects of BMP-7 at various concentrationsm as shown in higher magnificationin Figure **10****.** Here, it is indicated that the BMP-7 suppresses the change of the human corneal endothelial cell in a fibroblastic manner and maintains the function thereof. (A) is a photograph obtained using a phase-contrast microscope. The upper left image is a control with no BMP-7 added thereto (shown as Control). The upper right image shows 10ng/mL, the lower right image shows 100 ng/mL, and the lower left image shows 1,000 ng/mL, of BMP-7. The elongated cell form of the fibroblast phenotype was converted into a polygonal cell form in response to the presence of BMP-7, in a concentration-dependent manner. The scale bar shows 100 µm. (B) is a photograph of phalloidin staining. The upper left image is a control with no BMP-7 added thereto. The upper right image shows 10ng/mL, the lower right image shows 100ng/mL, and the lower left image shows 1,000 ng/mL, of BMP-7. The BMP-7 enables a normal hexagonal cell form, and enables cytoskeleton distribution in a cell surface layer of actine. The scale bar shows 100µm. (C) and (D) are each a photograph of Na⁺/K⁺-ATPase and ZO-1 staining. The upper left image is a control with no BMP-7 added thereto. The upper right image shows 10ng/mL, the lower right image shows 100ng/mL, and the lower left image shows 1,000 ng/mL, of BMP-7. The BMP-7 maintained intracellular localization of Na⁺/K⁺-ATPase and ZO-1 in a cell membrane. The scale bar shows 100µm. (E) and (F) are graphs showing the percentage of a Na⁺/K⁺-ATPase positive cell (E) and a ZO-1 positive cell (F) in culture mediums with three concentrations of BMP-7, in addition to control. The control is additive free. The ratio significantly increased in both the Na⁺/K⁺-ATPase positive cell and ZO-1 positive cell when treated with the BMP-7, compared to the control. *P<0.01, **P<0.05.

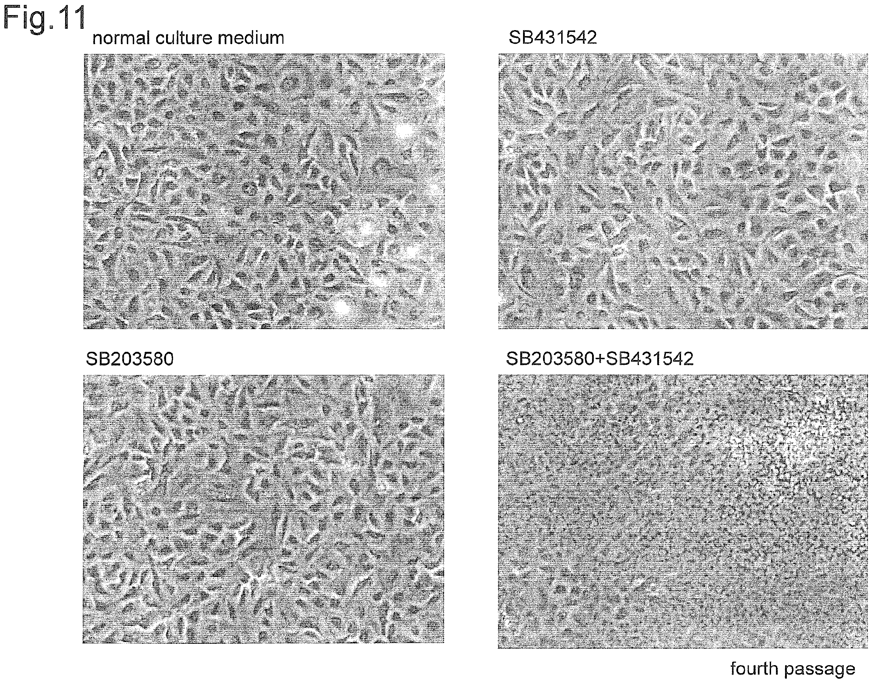

[Figure 11] Figure **11** shows results demonstrating that when an inhibitor of p38 MAPK was added in conjunction with SB431542, human cornea endothelial cells retained their form at high density even after repetitive subculturing due to p38 MAPK inhibition + TGF-β signal inhibition, thereby enabling the culturing. The upper left photograph shows the control (normal culture medium) . The upper right photograph shows a result with SB431542 only. The lower left photograph shows a result with SB203580 only, and the lower right photograph shows a result of both SB431542 and SB203580.

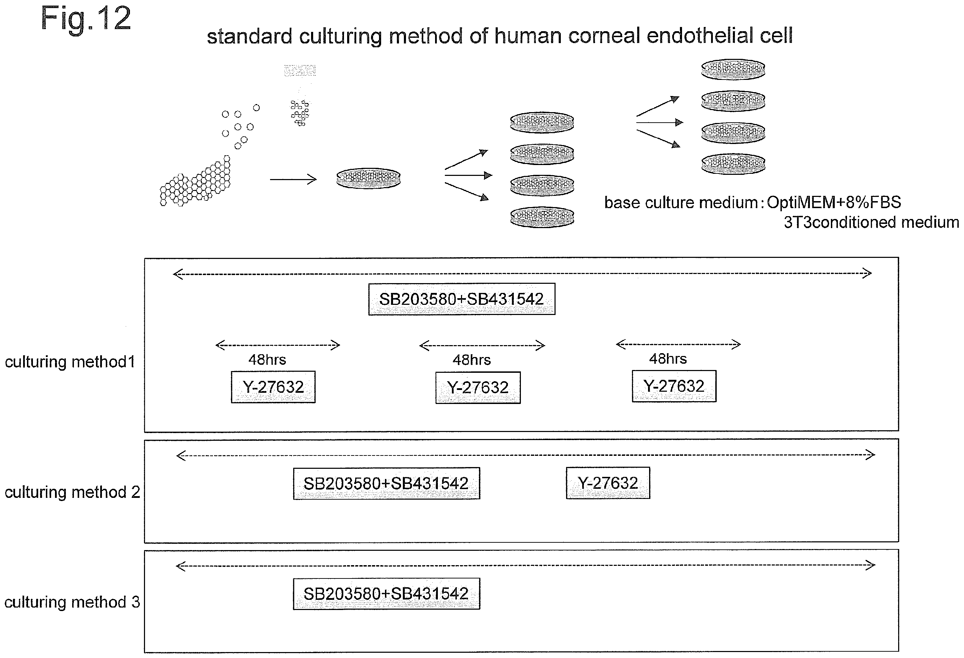

[Figure 12] Figure **12** is an example of a standard culturing method of a human corneal endothelial cell, which is established by the present invention. The upper panel shows a schematic view of subculturing, and shows a schematic view culturing methods 1 to 3 which were conducted in Example 8. In each of the methods, SB203580 and SB431432 were present. The culturing method 1 is a method where Y-27632 was introduced three times (for 48hrs each time) and removed in between each reintroduction. In the culturing method 2, Y-27632 is present the entire duration. In the culturing method 3, Y-27632 is not present at all.

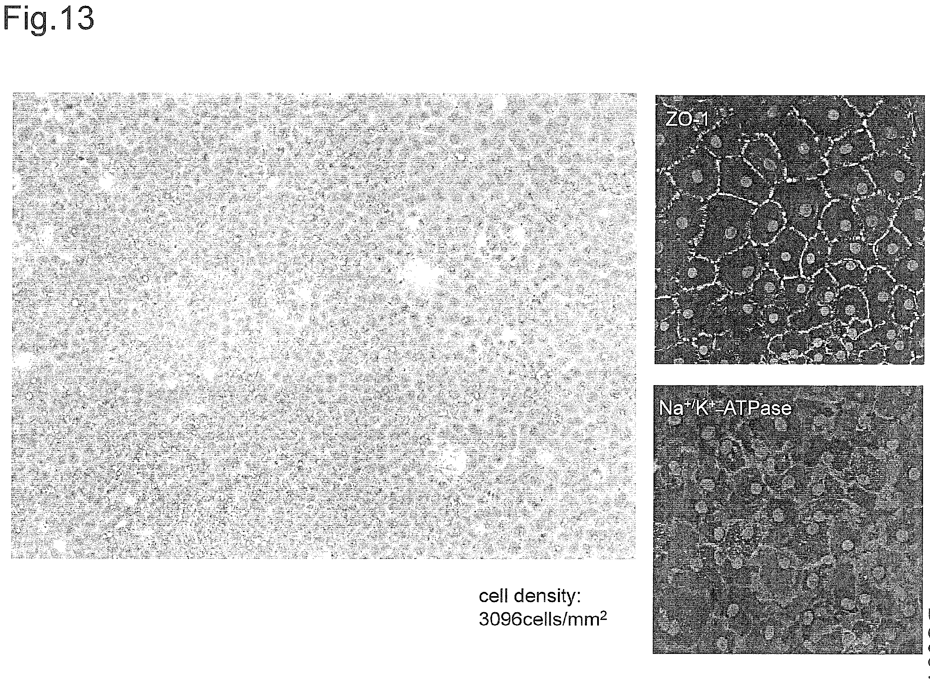

[Figure 13] Figure **13** shows a result of the established final human corneal endothelial cell culture. As shown in the photograph on the left side, it is understood that fibrosis is suppressed and the growth is favorable. As shown in the photograph on the right side, normal functions were retained as apparent from the staining of the ZO-1 shown on the top and the Na⁺/K⁺-ATPase shown on the bottom.

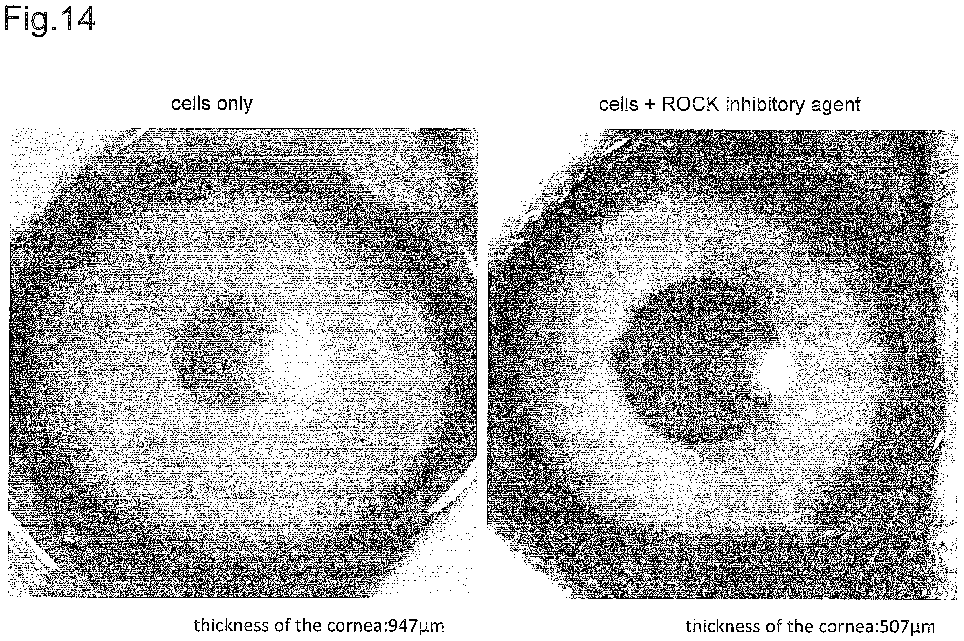

[Figure 14] Figure **14****,** described in Example 9 shows that human corneal endothelial cells, which were cultured in a normal form while maintaining its function, were cultured on a collagen sheet, followed by transplanting to a cynomolgus monkey bullous keratopathy model, thereby obtaining transparent curing of the cornea. The left side shows a result of transplanting only the cells that were cultured by the culturing method according to the present invention, while the right side shows a result of injecting a ROCK inhibitor, Y-27632, in transplanting the cells cultured by the culturing method according to the present invention.

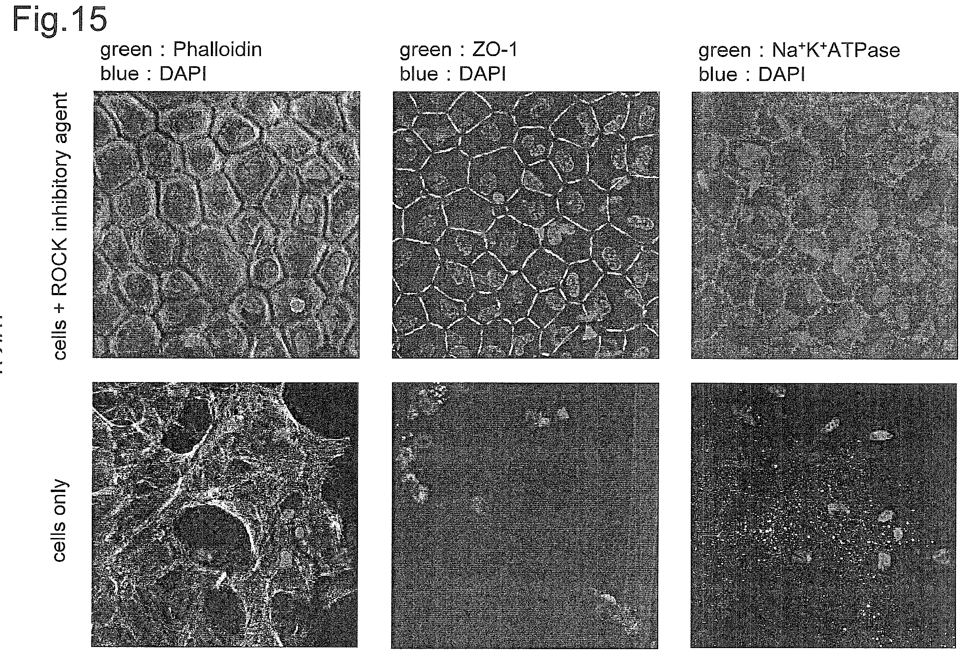

[Figure 15] Figure **15** shows a result of an image captured through a fluorescence microscope in Example 9 in which after euthanasia 2.5 months later, a cornea was extracted and the tissues were fixed, and then immunostained with phalloidin, Na⁺/K⁺-ATPase, and ZO-1 similar to Example 2. The upper row shows a result of the cells + ROCK inhibitor, and the lower row shows a result with the cells only. The left panel shows staining of phalloidin (the original color is green; and in a gray scale, it is in a netlike appearance, as shown by the top image and it appears to be diffused, as shown by the bottom image) and DAPI (the original color is blue; and in a gray scale, the inside of the cells is stained in a particulate manner, as shown by the top image; the cells are also stained in a particulate manner, as shown by the bottom image, but the number is decreased compared to that of the top image, and they appear to be overlapping with phalloidin staining). The center panel shows staining of ZO-1 (the original color is green; in a gray scale, it is in a netlike appearance, as shown by the top image, and it appears to have remained partially on the upper left corner and the lower right corner, as shown by the bottom image) and DAPI (the original color is blue; and in a gray scale, the inside of the cells is stained in a particulate manner, as shown by the top image and the particulate staining disappeared, and is thin even though the overall staining is observed as shown by the bottom image) . The right side shows staining of Na⁺/K⁺-ATPase (the original color is green; in a gray scale, it is in a netlike appearance, as shown by the top image while it appears remained partially at the center, as shown by the bottom image) and DAPI (the original color is blue; in a gray scale, the inside of the cells is stained in a particulate manner on the upper side, the cells are also stained in a particulate manner , as shown on the bottom image, but the number is decreased compared to that of the top image, and they appear overlapping with phalloidin staining).

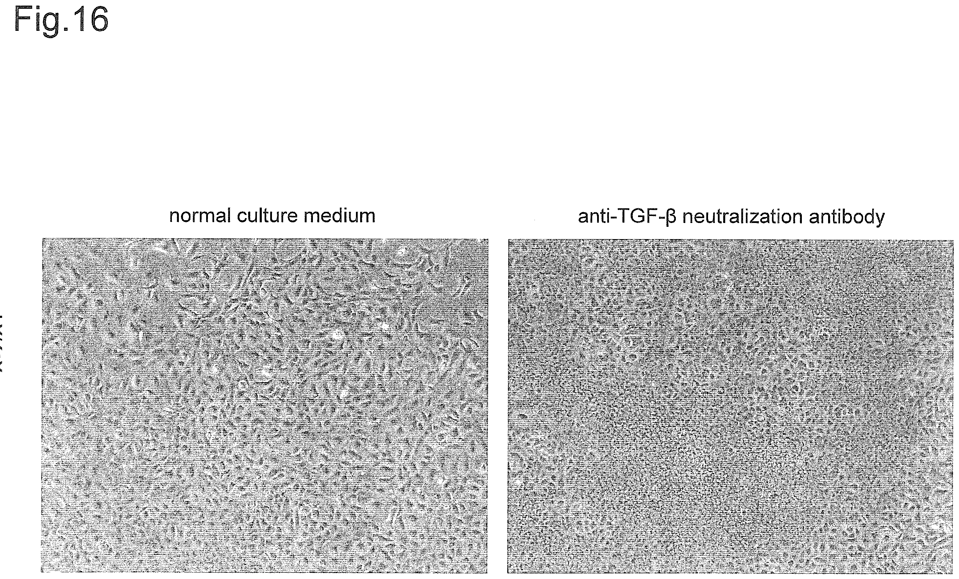

[Figure 16] Figure **16** shows culture normalization in a case of using an anti-TGF-β neutralization antibody, which was performed in Example 10. The left side shows a result with a normal culture medium, and the right side shows a result with an anti-TGF-β neutralization antibody.

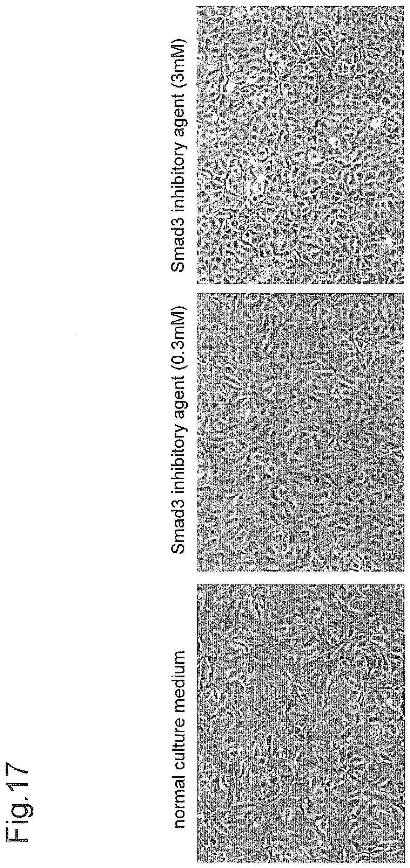

[Figure 17] Figure **17** shows culture normalization in a case of using a Smad3 inhibitor, 6,7-dimethoxy-2-((2E)-3-(1-methyl-2-phenyl-1H-pyrrolo[2 ,3-b]pyridine-3-yl-prop-2-enoyl))-1,2,3,4-tetrahydroiso quinolone (catalog number: 566405), available from Calbiochem, which is performed in Example 11. The left panel shows a result with a normal culture medium, and the center and right panel show results with a Smad3 inhibitor at 0.3 mM and 3 mM.

### [Description of Embodiments]

Hereinafter, the present invention will be described. Throughout the present specification, unless specifically referred to, an expression in a singular form is to be understood to encompass the concept of its plurality form. Therefore, unless specifically referred to, singular form articles (for example, "a", "an", "the" or the like in English, and corresponding articles and adjectives or the like in other languages) are to be understood to encompass the concept of their plurality form. Furthermore, terms used herein, unless specifically referred to, are to be understood to be used in the meaning usually used in the art. Therefore, unless defined otherwise, all technical terms and scientific terms herein have the same meaning as generally recognized by those skilled in the art. In case of contradiction, the present specification (including the definition) governs.

### (Definition)

As used herein, "fibrosis inhibitor" refers to any agent for suppressing fibrosis. The fibrosis inhibitor as used herein includes a cytokine and the like known to have an anti-fibrosis action, such as a transforming growth factor (TGF) -β signal inhibiting agent, amitogenic factor (mitogen) activator protein kinase (MAPK)38 inhibiting agent, interleukin (IL)-12, IL-10, interferon (IFN)-γ, and BMP-7 (OP-1). Information on such cytokines and the like is available from public database, such as GenBank, and journals and publications. Although it is not desired to be restricted by theories, the present invention has been able to achieve significant increase in corneal endothelial cells by suppressing fibrosis, while it was conventionally difficult to achieve the growth of a cell having a normal function. Accordingly, it is understood that the fibrosis inhibitor used in the present invention can be any agent as long as it provides growth of a cell having a normal function.

For example, while a variety of mammalian IFN-γ polypeptides can be used for the treatment of human diseases, human protein is generally used for a human corneal endothelial cell. A human IFN-γ coding sequence can be found in GenBank accession numbers P01579 and CAA00375. A corresponding genome sequence can be found in GenBank accession numbers J00219, M37265, and V00536. For example, see Gray et al. (1982) Nature 295:501 (GenBank X13274) ; and Rinderknecht et al. (1984) J. Biol. Chem. 259:6790.

Alternatively, a calcium channel-blocking agent, such as verapamil, can be used as a fibrosis inhibitor. Such a fibrosis inhibitor can have, not only the ability to decrease the synthesis of collagen type I, but also an anti-fibrosis action due to the stimulation from degradation of collagen type I fibrae. The *in vitro* testing regarding fibroblast demonstrates that extracellular delivery of collagen is dependent on the presence of calcium. A calcium channel blocking agent, verapamil, decreases the concentration of intracellular calcium, and increases collagenase activity. This also inhibits the growth of fibroblast.

As used herein, "transforming growth factor -β (transforming growth factor-β; also referred to as an abbreviated name TGF-β) " is used with the meaning similar to the meaning of those used in the art; and the transforming growth factor -β is a homodimer multifunctional cytokine of a molecular weight of 25kD, which exhibits various types of biological activity. TGF-β has a role in pathogenesis of a variety of sclerosing diseases, rheumatoid arthritis, and proliferative vitreoretinopathy, and is greatly involved in hair loss, suppressing the action of immunocompetent cells, suppressing hyperproduction of protease to prevent lung tissues from being degraded and preventing emphysema, and suppressing the growth of cancer cells, and the like. Three isoforms of TGF-β exist in humans, namely TGF-β1 to β3. TGF-β is produced as an inactive latent type with a molecular weight of about 300kD, which is not able to bind to a receptor. TGF-β is activated on a target cell surface or in the periphery thereof to become an active type capable of binding to a receptor, thus exerting the action thereof.

Although it is not desired to be restricted by theories, the action of TGF-β in a target cell is regarded as being transmitted by a phosphorylation pathway of a set of proteins for performing information transmission, referred to as Smad. First, when active TGF-β is bound to a type II TGF-β receptor present on a surface of a target cell, a receptor complex is formed which consists of two molecules of a type II receptor and two molecules of a type I TGF-β receptor, and the type II receptor phosphorylates the type I receptor. Next, the phosphorylated type I receptor phosphorylates Smad2 or Smad3, and the phosphorylated Smad2 or Smad3 forms a complex with Smad4, and the complex transfers to a nucleus, binds to a target sequence referred to as CAGA box, which is present in a target gene promoter region, and induces transcriptional expression of a target gene together with a coactivator.

The transformation growth factor-β(TGF-β) signaling pathway is capable of regulating many cell activities, such as cell growth and differentiation, growth arrest, apoptosis, and epithelial-to-mesenchymal conversion (EMT), by regulation of a target gene thereof. TGF-β family members, including the TGF-β itself (such as TGF-β1, TGF-β2 andTGF-β3), activin and bone morphogenic protein (BMP), are strong regulating agents for cell growth, differentiation, migration and apoptosis.

The TGF-β is a protein of about 24Kd, which is produced by many cells including B lymphocyte, T lymphocyte and activated macrophage, and by many other cell types. Effects of TGF-β to immune systems include IL-2 receptor induction, inhibition of IL-1 induced thymic cell growth, and blocking of IFN-γ-induced macrophage activation. The TGF-β is thought to be involved in a variety of pathological conditions (Border et al. (1992) J. Clin. Invest. 90:1), and is sufficiently supported to function as either a tumor inhibitory substance or a tumor promoter.

TGF-β mediates the signaling thereof by two serine/threonine kinase cell surface receptors, TGF-βRII and ALK5. TGF-β signaling is initiated by ligand-induced receptor dimerization, which allows TGF-βRII to phosphorylate an ALK5 receptor. The phosphorylation thereof is such that ALK5 kinase activity is activated and the activated ALK5 then phosphorylates a downstream effector Smad protein (vertebrate homologue of MAD or "Mothers against DPP (decapentaplegic)" protein), Smad2 or 3. The p-Smad2/3 complex with Smad4 enters a nucleus to activate the transcription of a target gene.

Smad3 is a member of a R-Smad (receptor-activated Smad) subgroup of Smad, and is a direct mediator of activation of transcription by a TGF-β receptor . TGF-β stimulation causes phosphorylation and activation of Smad2 and Smad3, which forms a complex with Smad4 ("common Smad" or "co-Smad" in vertebrates), which is accumulated together with a nucleus to regulate the transcription of a target gene. R-Smad is localized at a cytoplasm, and forms a complex with a co-Smad through ligand-induced phosphorylation by a TGF-β receptor; and the complex moves to a nucleus, which then regulates gene expression that is associated with chromatin and a cooperative transcription factor. Smad6 and Smad7 are each inhibitory Smad ("I-Smad"), that is, they are transcriptionally induced by TGF-β and function as an inhibitor for TGF-β signaling (Feng et al. (2005) Annu. Rev. Cell. Dev. Biol. 21:659). Smad6/7 inhibits the receptor-mediated activation of R-Smad to exert their inhibitory effect; and they are associated with a type I receptor, which competitively prevents mobilization and phosphorylation of R-Smad. Smad6 and Smad7 are known to replenish E3 ubiquitin ligase, which causes ubiquitination and degradation of Smad6/7 interactive protein.

With regard to the TGF-β signaling pathway, another pathway additionally exists which is transmitted by BMP-7 or the like, which is regarded as exhibiting functions via ALK-1/2/3/6 and then via Smad1/5/8. With regard to the TGF-β signaling pathway, also see J. Massagu'e, Annu. Rev. Biochem. 1998. 67: 753-91; Vilar JMG, Jansen R, Sander C (2006) PLoS Comput Biol 2(1):e3; Leask, A., Abraham, D. J. FASEB J.18,816-827 (2004); Coert Margadant & Arnoud Sonnenberg EMBO reports (2010) 11, 97-105; Joel Rosenbloom et al., Ann Intern Med. 2010; 152: 159-166 and the like.

As used herein, "transforming growth factor (TGF)-β signal inhibiting agent" refers to any factor that inhibits TGF signaling. When TGF-β is counteracted, it agent responsible may be referred to as an antagonist. However, in the case of the present invention, the TGF-β antagonist is encompassed by the TGF-β signal inhibiting agent.

Therefore, the TGF-β signal inhibiting agent used in the present invention typically includes, without limitation, an antagonist of TGF-β, an antagonist of a receptor of TGF-β, and an inhibitor of Smad3.

Exemplary TGF-β signal inhibiting agent used in the present invention include, without limitation, SB431542 (4-[4-(1,3-benzodioxol-5-yl)-5-(2-pyridinyl) -1H-imidazol-2-yl]benzamide), BMP-7, anti-TGF-β antibody, anti-TGF-β receptor antibody, siRNA of TGF-β, siRNA of TGF-β receptor, antisense oligonucleotide of TGF-β, 6,7-dimethoxy-2-((2E)-3-(1-methyl-2-phenyl-1H-pyrrolo[2 ,3-b]pyridine-3-yl-prop-2-enoyl))-1,2,3,4-tetrahydroiso quinolone, A83-01(3-(6-methyl-2-pyridinyl)-N-phenyl-4-(4-quinoliny 1)-1H-pyrazole-1-carbothioamide), Stemolecule™ TLK inhibitor (2-(3-(6-methylpyridine-2-yl)-1H-pyrazole-4-yl)-1,5-nap hthyridine), Stemolecule™ BMP inhibitor LDN-193189(6-(4-(piperidine-1-yl)ethoxy)phenyl)-3-(pyri dine-4-yl)pyrazolo[1,5-a]pyrimidine), SD-208(2-(5-chloro-2-fluorophenyl)-4-[(4-pyridinyl)amin o]pteridine), LY364947(4-[3-(2-pyridinyl)-1H-pyrazole-4-yl]-quinoline ), a pharmaceutically acceptable salt or a solvate thereof, or a solvate of a pharmaceutically acceptable salt thereof, and the like.

Other TGF-β signal inhibiting agents include, without limitation, a monoclonal antibody and a polyclonal antibody to one or more isoforms of TGF-β (US Patent No. 5,571,714; also see International Publication No. WO 97/13844 and International Publication No. WO 00/66631), TGF-β receptor, a soluble form of such a receptor (e.g., soluble TGF-β type III receptor), or an antibody directed to a TGF-β receptor (US Patent No. 5, 693, 607, US Patent No. 6, 001, 969, US Patent No. 6,010,872, US Patent No. 6,086,867, US Patent No. 6,201,108; International Publication No. WO 98/48024; International Publication No. WO 95/10610; International Publication No. WO 93/09228; International Publication No. WO 92/00330), latent and associated peptide (International Publication No. WO 91/08291), large latent TGF-β (International Publication No. WO 94/09812), fetuin (US Patent No. 5,821,227), decorin and biglycan, fibromodulin, lumican, and endoglin and other proteoglycan (International Publication No. WO 91/10727; US Patent No. 5,654,270, US Patent No. 5,705,609, US Patent No. 5,726,149; US Patent No. 5,824,655; International Publication No. WO 91/04748; US Patent No. 5,830,847, US Patent No. 6,015,693; International Publication No. WO 91/10727; International Publication No. WO 93/09800; and International Publication No. WO 94/10187), somatostatin (International Publication No. WO 98/08529), mannose-6-phosphoric acid or mannose-1-phosphoric acid (US Patent No. 5,520,926), prolactin (International Publication No. WO 97/40848), insulin-like growth factor II (International Publication No. WO 98/17304), IP-10 (International Publication No. WO 97/00691), Arg-Gly-Asp-containing peptide (Pfeffer, US Patent No. 5,958,411; International Publication No. WO 93/10808), plants, fungi and bacteria extracts (EP-A-813875; Japanese Laid-Open Publication No. 8-119984; and Matsunaga et al., US Patent No. 5,693,610), antisense oligonucleotide (US Patent No. 5,683, 988; US Patent No. 5,772,995; US Patent No. 5,821,234, US Patent No. 5,869,462; and International Publication No. WO 94/25588), protein associated with TGF-β signaling including Smad and MAD (EP-A-874046; International Publication No. WO 97/31020; International Publication No. WO 97/38729; International Publication No. WO 98/03663; International Publication No. WO 98/07735; International Publication No. WO 98/07849; International Publication No. WO 98/45467; International Publication No. WO 98/53068; International Publication No. WO 98/55512; International Publication No. WO 98/56913; International Publication No. WO 98/53830; International Publication No. WO 99/50296; US Patent No. 5,834,248; US Patent No. 5,807,708; and US Patent No. 5,948,639), Ski and Sno (Vogel, 1999, Science, 286:665; and Stroschein et al.,1999, Science, 286:771 to 774), one or more single-stranded oligonucleotide aptamers or an expression plasmid encoding them, suitable for inhibiting or interfering the binding of TGF-β to a receptor of the same origin, and any mutant, fragment or derivative of a molecule identified above, which retains an ability to inhibit the activity of TGF-β. The TGF-β inhibitor may be a TGF-β antagonist, and may be a human monoclonal antibody or a humanized monoclonal antibody (or F(ab)₂ fragment, Fv fragment, single chain antibody, and other forms or fragments of an antibody retaining the ability to bind to TGF-β, a fragment thereof or the like), which blocks TGF-β binding to the receptor. The TGF-β receptor and a TGF-β binding fragment, and in particular a soluble fragment, of a TGF-β receptor are TGF-β antagonists which are useful in the method according to the present invention. In a certain embodiment, an inhibitor preferable for TGF-β functions is a soluble TGF-β receptor, and in particular, a TGF-β type II receptor (TGFBIIR) or a TGF-β type III receptor (TGFBIIIR or betaglycan) including, for example, TGFBIIRor extracellular domain of TGFBIIIR, preferably a recombinant soluble TGF-β receptor (rsTGFBIIR or rsTGFBIIIR) . The TGF-β receptor and a TGF-β binding fragment of the TGF-β receptor, in particular a soluble fragment, are TGF-β antagonists useful in the method according to the present invention. TGF-β receptors and nucleic acids encoding them are sufficiently known in the art. A nucleic acid sequence encoding TGF-β type 1 receptor is disclosed in GenBank accession number L15436 and US Patent No. 5,538,892 (Donahoe et al.). A nucleic acid sequence of a TGF-β type 2 receptor is publicly available under GenBank accession number AW236001, AI35790, AI279872, AI074706, and AA808255. A nucleic acid sequence of a TGF-β type 3 receptor is also publicly available under GenBank accession number NM003243, AI887852, AI817295, and AI681599.

In addition, still other TGF-β signal inhibiting agents or antagonists and methods for producing them, are sufficiently known in the art, in addition to many of those that are currently under development. Any of effective TGF-β antagonists may be useful in the method according to the present invention, and thus, specific TGF-β signal inhibiting agents or antagonists used are not those with limited characteristics. Examples of such antagonists include a monoclonal and polyclonal antibody to TGF-β of one or more isotypes (US Patent No. 5,571,714 and International Publication No. WO 97/13844), TGF-β receptor, a fragment thereof, a derivative thereof, and an antibody to a TGF-β receptor (US Patent No. 5,693,607, US Patent No. 6,008,011, US Patent No. 6,001,969 and US Patent No. 6,010,872, and International Publication No. WO 92/00330, International Publication No. WO 93/09228, International Publication No. WO 95/10610, and International Publication No. WO 98/48024) ; latency-associated peptide (latency associated peptide; International Publication No. WO 91/08291), large lacent TGF-β (International Publication No. WO 94/09812), fetuin (US Patent No. 5,821,227), decorin, and biglycan, fibromodulin, lumican, endoglin, and other proteoglycan (US Patent No. 5,583,103, US Patent No. 5,654,270, US Patent No. 5,705,609, US Patent No. 5,726,149, US Patent No. 5,824,655, US Patent No. 5,830,847, US Patent No. 6,015,693, and International Publication No. WO 91/04748, International Publication No. WO 91/10727, International Publication No. WO 93/09800 and International Publication No. WO 94/10187) .

Further examples of such an antagonist include a host of other proteins associated with TGF-β signaling, including somatostatin (International Publication No. WO 98/08529), mannose-6-phosphoric acid or mannose-1-phosphoric acid (US Patent No. 5,520,926), prolactin (International Publication No. WO 97/40848), insulin-like growth factor II (International Publication No. WO 98/17304), IP-10 (International Publication No. WO 97/00691), arginine (arg)-glycine (gly)-asparagine acid (asp)-containing peptide (US Patent No. 5,958,411 and International Publication No. WO 93/10808), plants, fungi and bacteria extarcts (European Patent Application Publication No. 813875, Japanese Laid-Open Publication No. 8-119984 and US Patent No. 5,693,610), antisense oligonucleotide (US Patent No. 5,683,988, US Patent No. 5,772,995, US Patent No. 5,821,234 and US Patent No. 5,869,462, and International Publication No. WO 94/25588), and Smad and MAD (European Patent Application No. EP874046, International Publication No. WO 97/31020, International Publication No. WO 97/38729, International Publication No. WO 98/03663, International Publication No. WO 98/07735, International Publication No. WO 98/07849, International Publication No. WO 98/45467, International Publication No. WO 98/53068, International Publication No. WO 98/55512, International Publication No. WO 98/56913, International Publication No. WO 98/53830 and International Publication No. WO 99/50296, and US Patent No. 5,834,248, US Patent No. 5,807,708 and US Patent No. 5,948,639), and Ski and Sno (G.Vogel,Science,286:665(1999) and Stroschein et al.,Science,286:771-74(1999)), and any fragment and derivative of the above-mentioned molecule retaining the ability to inhibit the activity of TGF-β.

The TGF-β antagonists suitable for the use in the present invention also include a functional mutant, a mutant, a derivative, and an analogue of the aforementioned TGF-β antagonist so long as their ability of inhibiting the amount or activity of TGF-β is retained. The "mutant", "derivative", and "analogue" as used herein refers to a molecule having a form or structure similar to that of their parent compound, and retaining an ability to work as a TGF-β antagonist. For example, any of the TGF-β antagonists disclosed in the present specification may be crystallized, and useful analogues may be reasonably designed based on sites that have a role in forming (one or more) active sites. Instead, those skilled in the art can alter a functional group of known antagonists, or can screen such an altered molecule with regard to activity, half-life, bioavailability, or other desirable characteristics, without unnecessary experiments. When the TGF-β antagonist is a polypeptide, a fragment and variant of the polypeptide may be produced to increase the ease of delivery, activity, half-life and the like (e.g., humanized antibodies or functional antibody fragments discussed above). In consideration of the technical level in the art for producing synthetic and recombinant polypeptides, such a variant may be achieved without unnecessary experiments. Those skilled in the art may also design a novel inhibitor based on knowledge on a crystal structure and/or active site of the TGF-β inhibitor as described herein. A polypeptide inhibitor, such as a soluble TGF-β receptor, may be effectively introduced through gene transfer. Accordingly, a certain embodiment for the method according to the present invention includes use of a vector suitable for expression of a TGF-β receptor or a binding partner, preferably a soluble receptor or a soluble binding partner. In a preferable embodiment, administration of a soluble TGF-β antagonist can be achieved by gene transfer which uses a vector comprising a cDNA encoding a soluble antagonist or a cDNA encoding an extracellular domain of a TGF-β type II receptor (rsTGFBIIR) or a TGF-β type III receptor (rsTGFBIIIR) . This vector causes an in situ expression of a soluble TGF-β antagonist in a cell which is transfected using the vector, inhibits the activity of TGF-β, and suppresses TGF-β-mediated fibrogenesis. Any suitable vector can be used. Preferable vectors include a adenovirus vector, a lentivirus vector, an Epstein-Barr virus (EBV) vector, an adeno-associated virus (AAV) vector, and a retrovirus vector, developed for the purpose of gene transfer. Other non-vector methods for gene transfer may also be used, such as lipid/DNA complex, protein /DNA conjugate and naked DNA transfer methods. Further suitable TGF-β antagonists developed for delivery via adenovirus gene transfer include, without limitation, a chimeric cDNA encoding an extracellular domain of a TGF-β type II receptor, fused to an Ig Fc domain (Isaka et al., 1999, Kidney Int., 55:pp.465 to 475), an adenovirus gene transfer vectorof a dominant negative mutant of a TGF-β type II receptor (Zhao et al.,1998, Mech. Dev., 72:pp.89 to 100), and an adenovirus gene transfer vector of decorin, which is a TGF-β binding proteoglycan (Zhao et al., 1999, Am. J. Physiol., 277: pp. L412 to L422). Adenovirus-mediated gene transfer has extremely high efficiency compared to other gene delivery manners.

The TGF-β receptor and a TGF-β binding fragment, a soluble fragment and the like of the TGF-β receptor are TGF-β antagonists useful in the present invention. The TGF-β receptors and nucleic acids encoding them are sufficiently known in the art. The nucleic acid sequence encoding the TGF-β type 1 receptor is disclosed in GenBank, accession number L15436 and US Patent No. 5,538,892 by Donahoe et al. A nucleic acid sequence of the TGF-β type 2 receptor is also publicly available under GenBank accession number AW236001; AI35790; AI279872; AI074706; and AA808255. A nucleic acid sequence of the TGF-β type 3 receptor is also publicly available under GenBank accession number NM003243; AI887852; AI817295; and AI681599. In one exemplary embodiment, the TGF-β antagonist is an antibody which blocks TGF-β binding to a receptor thereof, or to a F(ab)₂ fragment, a Fv fragment, a single-stranded antibody, and a fragment of other "antibody" types retaining the ability to bind to TGF-β. The antibody thereof may be chimerized or humanized. Herein, the chimerized antibody includes a constant region of a human antibody, a variable region of a murine antibody and other non-human antibodies. The humanized antibody includes a constant region and a framework variable region (i.e., variable regions other than hypervariable regions) of a human antibody, and a hypervariable region of a murine antibody and other non-human antibodies. As a matter of course, the antibody thereof may be selected from a phage display system, or may be an antibody derivative of any other types, such as a human antibody selected therefrom or produced from a XenoMouse.

Findings related to Smad are increasing. TGF-β signaling pathway is initiated when this molecule binds to a heterodimer cell surface complex consisting of a serine/threonine kinase receptor of type I (TbRI) and type II (TbRII) and induces this heterodimer cell surface complex. Then, the heterodimer receptor transmits said signal through phosphorylation of a target Smad protein in the downstream. As described above, there are three functional classes for the Smad protein, and they are, for example, Smad(R-Smad) restricted by a receptor such as Smad2 and Smad3, a co-mediator (Co-Smad) which is also referred to as Smad4, and an inhibitory Smad (I-Smad) . Followed by the phosphorylation by the heterodimer receptor complex, this R-Smad forms a complex with this Co-Smad, moves to said nucleus, and working together with other respective proteins, they regulate transcription of the target gene (Derynck, R., et al. (1998) Cell 95: 737-740) ; Massague, J. and Wotton, D. (2000) EMBO J. 19:1745). A nucleotide sequence and an amino acid sequence of human Smad3 are disclosed in, for example, GenBank Accession No. gi : 42476202. A nucleotide sequence and an amino acid sequence of murine Smad3 is disclosed in, for example, GenBank Accession No. gi: 31543221. As described above, TGF-β stimulation provides phosphorylation and activation of Smad2 and Smad3, which form a complex with Smad4 (also referred to as "common Smad" or "co-Smad"), and the complex is accumulated with a nucleus to regulate the transcription of the target gene. Accordingly, the TGF-β signal inhibition may also be achieved by inhibition of Smad2, 3 or co-Smad (Smad4). The R-Smad is localized in a cytoplasm, and forms a complex with a co-Smad through ligand-induced phosphorylation by a TGF-β receptor to move to a nucleus, in which they regulate gene expression associated with a chromatin and a cooperative transcription factor. Thus, TGF-β signal inhibition can also be achieved by inhibiting R-Smad either directly or indirectly. Smad6 and Smad7 are inhibitory Smad (I-Smad), and that is, they are transcriptionally induced by TGF-β to function as an inhibitor of TGF-β signaling (Feng et al., (2005) Annu. Rev. Cell. Dev. Biol. 21: 659) . Smad6/7 prevents receptor-mediated activation of R-Smad, thereby exerting their inhibitory effect. They are associated with a type I receptor, which competitively inhibits mobilization and phosphorylation of R-Smad. Smad6 and Smad7 are known to replenish E3 ubiquitin ligase, which causes ubiquitination and degradation of Smad6/7 interactive protein. Thus, Smad6 and 7 can function as a TGF-β signal inhibiting agent in the present invention.

The inhibitors of Smad3 that may be used in the present invention include, without limitation, antisense nucleotide, siRNA, antibody and the like, and in addition, 6,7-dimethoxy-2-((2E)-3-(1-methyl-2-phenyl-1H-pyrrolo[2 ,3-b]pyridine-3-yl-prop-2-enoyl))-1,2,3,4-tetrahydroiso quinolone, and the like available from Calbiochem, as a low-molecular compound.

As used herein, "culture normalization" of a corneal endothelial cell refers to culturing while maintaining at least one characteristic, such as functions that the corneal endothelial cell originally has (which is also referred as "normal function" herein) or the like. Such functions include, without limitation, ZO-1 and Na⁺/K⁺-ATPase, adaptability to a corneal transplant, (Matsubara M, Tanishima T: Wound-healing of the corneal endotheliumin the monkey: a morphometric study, Jpn J Ophthalmol 1982, 26:264-273;MatsubaraM, Tanishima T: Wound-healing of corneal endothelium in monkey: anautoradiographic study, Jpn J Ophthalmol 1983, 27:444-450; Van Horn DL, Hyndiuk RA: Endothelial wound repair in primate cornea, Exp Eye Res 1975, 21:113-124 and VanHorn DL, Sendele DD, Seideman S, Buco PJ: Regenerative capacity of the cornealendothelium in rabbit and cat, Invest Ophthalmol Vis Sci 1977, 16:597-613) and the like. Specifically, it is understood that the "normal function" may be a function required to achieve corneal transplant or an index indicating sufficiency therefor.

With regard to the adaptability to corneal transplant, normally, a corneal endothelium can be mechanically curetted as a bullous keratopathy model with experimental animals such as rabbits to conduct an implantation test of a cultured cell. However, corneal endothelial cells of rabbits grow *in vivo.* Thus, there is no denying of the possibility of spontaneous healing due to growth of corneal endothelial cells of the host (Matsubara M, et al., Jpn J Ophthalmol 1982, 26:264-273; Matsubara M, et al., Jpn J Ophthalmol 1983, 27:444-450; Van Horn DL, et al., Exp Eye Res1975, 21:113-124 and Van Horn DL, et al., Invest Ophthalmol Vis Sci 1977, 16:597-613) . Thus, in order to evaluate more accurate transplant adaptability, it is preferable to evaluate engraftment to primates. In a case of evaluating transplant adaptability to humans, with a primate such as cynomolgus monkey, adaptability is evaluated after passage of, for example, at least one month, preferably at least two months, more preferably at least three months, further preferably at least six months, still more preferably at least twelve months. It is important to confirm transplant adaptability with primates, such as monkeys, for the application to humans in particular.

As used herein, "culture normalizing agent" refers to an agent for preventing a characteristic, such as a normal function, of a corneal endothelial cell or the like from being lost, which may occur during culturing. In order for a culture normalizing agent to be recognized as exerting its function, it is possible to confirm it by testing at least once to determine whether or not a normal function of a corneal endothelial cell, as described herein, is maintained, or whether or not the function is decreased. For example, a method for judging normalization can be executed by using a functional protein in a corneal endothelial cell, such as ZO-1 and Na⁺/K⁺-ATPase, as an index to see the change in the expression thereof, or by examining as to whether or not it is engrafted to a monkey or the like by transplant to function. A method for judging by transplant can be performed as follows. Specifically, corneal endothelium is cultured on type I collagen to prepare a cultured corneal endothelium sheet. Under general anesthesia, the peripheral portion of a cornea of a cynomolgus monkey is cut by 1.5 mm, and a silicon surgical instrument is inserted into an anterior chamber to mechanically currete a corneal endothelial cell, thus creating a bullous keratopathy model. Then, the peripheral portion of the cornea is cut by 5-6 mm, and the cultured corneal endothelium sheet is inserted into the anterior chamber. By substituting the anterior chamber with air, the sheet is adhered to the surface of the corneal endothelium. The therapeutic effect of the transplant of the cultured corneal endothelium sheet on bullous keratopathy is evaluated by the corneal transparency through a slit-lamp microscope.

As used herein, "cell mitogenic factor (mitogen) activated protein (MAP) kinase inhibitor" refers to any inhibitor for inhibiting a signaling pathway of MAP kinase either directly or indirectly. Thus, a MAP kinase inhibitor is related to a compound targeting, decreasing, or inhibiting a mitogen activated protein for. The MAP kinases are a protein serine/threonine kinase group which are activated in response to various kinds of extracellular stimulation and which mediate signaling from a cell surface to a nucleus. They control some physiological and pathological cellular phenomena, including inflammation, cell death due to apoptosis, carcinogenetic transformation, tumor cell invasion, and metastasis.

The useful MAP kinase inhibitor according to the present invention can inhibit any MAP kinase factors, such as, without limitation, MAPK, ERK, MEK, MEKK, ERK1, ERK2, Raf, MOS, p21ras, GRB2, SOS, JNK, c-jun, SAPK, JNKK, PAK, RAC, andp38. Examples of the MAP kinase inhibitor include, without limitation, PD184352, VX-745, SB202190, anisomycin, PD98059, SB203580, U0126, AG126, apigenin, a HSP25 kinase inhibitor, 5-iodotubercidin, MAP kinase antisense oligonucleotide, control MAP kinase oligonucleotide, a MAP kinase cascade inhibitor, MAP kinase inhibitor set 1, MAP kinase inhibitor set 2, MEK inhibitor set, olomoucine, isoolomoucine, N⁹ isopropyl olomoucine, a p38 MAP kinase inhibitor, PD169316, SB202474, SB202190 hydrochloride, SB202474 dihydrochloride, SB203580 sulfone, Ioto-SB203580, SB220025, SC68376, SKF-86002, Tyrphostin AG 126, U0124, U0125, and ZM33637. See the page of CalBioChem catalog, ixxviii;http://www.tocris.com/; and http://www.vpharm.com/frame09.html.

The MAP kinase is a general name used to describe the family of serine/threonine kinase. The MAP kinase is also referred to as extracellular signal-regulatedprotein kinase or ERK, and it is a terminal enzyme of 3 kinase cascades. The repetition of 3 kinase cascades to a related, but separated signaling pathway demonstrates the concept of a MAPK pathway as a module multifunctional signaling element, which sequentially works in a pathway. In this pathway, each enzyme is characterized to be phosphorylated, thereby activating the following member in the sequence. As such, a standard MAPK module consists of three protein kinases. Specifically, a MAPK kinase (or MEKK) activates another MAPK kinase (or MEK), which sequentially activates a MAPK/ERK enzyme. MAPK/ERK, JNK (c-junamino terminal protein kinase (orSAPK))) andp38 cascade each consist of three enzyme modules including MEKK, MEK and ERK, or MAPK superfamily members. A variety of extracellular signals coalesce with respective cell surface receptors thereof, triggering an initial event, and then this signal is transmitted to the inside the cell, where an appropriate cascade is activated.

The MAPK is a mitogen activated protein kinase (or ERK) superfamily, and has a TXY consensus sequence in a catalytic core. ERK1/2, p38HOG, and JNK/SAPK are terminal enzymes which are related to parallel pathways, but are different from one another.

For example, constitutive activation of MAP kinase is associated with primary tumor derived from a variety of human organs (kidneys, large intestines, and lungs) and a large number of cancer cell lineages (pancreas, large intestines, lungs, ovaries, and kidneys) (Hoshino et al., Oncogene, 18 (3):813-22(Jan.1999)). Furthermore, p38 MAP kinase regulates the production of two cytokines, TNFα and IL-1, which are associated with the onset and progression of inflammation. The p38 MAP kinase inhibitor also plays a role in time to come in the treatment of inflammatory diseases such as rheumatoid arthritis, and in addition, in the treatment of cardiac failure, stroke, neurogenic diseases, and other diseases. As such, the MAP kinase inhibitor is useful for the treatment of various kinds of disease conditions, from cancer to inflammation.

Furthermore, ERK is the only substrate with regard to MEK1, and thus this close selectivity indicates that, together with enhancement of the expression of the essential components thereof in tumor cells and the central role in the MAP kinase pathway, the inhibition of the pathway is an important route for both the chemical sensitization and radiation of tumor cells, and is a target for proliferative diseases that may be used for pharmacological intervention .

Sebolt-Leopoldetal., Nat. Med., 5(7):810-6 (Jul, 1999) describes an *in vitro* cascade assay system for identifying a small molecule inhibitor of a MAP kinase (MAPK) pathway. Glutathione-S-transferase (GST)-MEK1 and GST-MAPK fusion protein were prepared from bacterial cells, and they were used for sequential phosphorylation to MAPK of MEK1, and to MBP (myelin basic protein (myelin basic protein)) in the assay system. PD184352 [2-(2-chloro-4-iodine-phenylamino)-N-cycloprop yl methoxy-3,4-difluoro-benzamide], which directly inhibits MEK1, has also been found.

Examples of the MAP kinase inhibitor include MAP kinase inhibitor :AG126, apigenin (Apigenin), HSP25 kinase inhibitor, 5-iodotubercidin, MAP kinase antisense oligonucleotide, control MAP kinase oligonucleotide, MAP kinase cascade inhibitor, MAP kinase inhibitor set 1, MAP kinase inhibitor set 2, MEK inhibitor set, olomoucine, isoolomoucine, N⁹ isopropyl olomoucine, p38 MAP kinase inhibitor, PD98059 (2'-amino-3'-methoxyflavone), PD98059 solution, PD169316(Calbiochem), SB202474, SB202190(4-[4-(4-fluorophenyl)-5-(4-pyridinyl)-1H-imida zole-2-yl] phenol; BIOMOL Research Labs., Inc.), SB202190 solution, SB202190 hydrochloride, SB202474dihydrochloride, SB203580 (4-(4-fluorophenyl)-2-(4-methylsulfonylphenyl)-5(4-pyri dyl)imidazole<4-[4-(4-fluorophenyl)-2-(4-methylsulfinyl phenyl)-1H-imidazole-5-yl]pyridine>; Journal of Biological Chemistry 272 (18) 12116-12121, 1997), SB203580 solution, SB203580 hydrochloride, SB203580 sulfone, Ioto-SB203580, SB220025, SP600125(1,9-pyrazoloanthrone, anthrapyrazole), SB239063(trans-4-[4-(4-fluorophenyl)-5-(2-methoxy-4-pyr imidinyl)-1H-imidazole-1-yl]cyclohexanol), SC68376, FR167653(Nikken Chemical Co., Ltd.), BIRB796BS (or BIRB-796; 1-(5-tert-butyl-2-p-trile-2H-pyrazole-3-yl)-3 (4-(2-morpholine-4-yl-ethoxy)naph-thaline-1-yl)urea, Blood101,4446-4448,2003), SKF-86002, tyrphostin AG126, U0124, U0125, U0126(1,4-diamino-2,3-dicyano-1,4-bis[2-aminophenylthio ]butadiene), 4-azaindole, 3-(4-fluorophenyl)-2-(pyridine-4-yl)-1H-pyrrolo[3,2-b]p yridine, ZM336372, CalBio506126(2-(4-chlorophenyl)-4-(4-fluorophenyl)-5-py ridine-4-yl-1,2-dihydropyrazole-3-one), RO3201195, R1487, and the like. Page ixxviii of CalBioChem catalog may also be referred. Additional MAP kinase inhibitors that can be used in the present invention include, for example, a neutralization antibody to MAP kinase, a compound for inhibiting activity of MAP kinase, a compound (e.g., antisense nucleic acid, RNAi, ribozyme) for inhibiting transcription of a gene encoding MAP kinase, peptide, and a plant component (e.g., polyphenol, flavonoid, and glycoside) and other compounds. With regard to the concentration used, for SB203580, SB202190, PD169316, FR167653, BIRB796BS and the like, about 50 nmol/l to 100 µmol/l is exemplified, and it normally includes, without limitation, about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5.0 µmol/l, about 1.0 to 5.0 µmol/l, about 1.25 to 5.0 µmol/l, about 1.5 to 5.0 µmol/l, about 1.75 to 5.0 µmol/l, about 2.0 to 5.0 µmol/l, about 2.5 to 5.0 µmol/l, about 3.0 to 5.0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3. 0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l about 0.09 to 35 µmol/l, about 0.09 to 3.2 µmol/l, more preferably, about 0.05 to 1.0 µmol/l, about 0.075 to 1.0 µmol/l about 0.1 to 1.0 µmol/l, about 0.5 to 1.0 µmol/l, and about 0.75 to 1.0 µmol/l.

As used herein, "aging inhibitor" or "antioxidant" for corneal endothelial cells refers to any agent capable of suppressing cellular senescence. Normal human cells lose their ability to divide after repeating a given number or more of divisions, and then become senescent (replicative senescence) . Senescent cells undergo specific morphological and physiological changes, and induce specific genes. Further, normal cells exhibit a phenomenon similar to those described above through various types of treatment (premature senescence). As such, "to suppress senescence" of cells herein refers to having an effect of increasing the degree of density of cells. Thus, more specifically, "aging inhibitor " or "antioxidant" refers to any agent for increasing the degree of density of cells. The degree of senescence of cells can be examined by morphological observation of the cells (when cells become senescent, flattening and hypertropy will occur) and by observing a stained image of β-galactosidase, known as a senescence marker (when senescence progresses, the stained image of β-galactosidase becomes larger). Thus, for the aging inhibitor used in the present invention, any agent can be used so long as it has the above-mentioned action for suppressing senescence. The action for suppressing senescence is such an action that suppresses decreased function of normal cells that is undergoing senescence, including, for example, an action for suppressing arrest of the cell cycle, an action for suppressing shortening the life-span of normal dividing cells, an action for suppressing decrease in the survival rate of normal cells, an action for suppressing morphological change accompanied by senescence in normal cells, and the like. Although it is not desired to be restricted by theories, according to Funayama R and Ishikawa F (Chromosoma (2007) 116:431-440), it is indicated that, although this is not a case of corneal endothelial cells, senescence due to various types of cellular stress in fibroblast and the like is due to activation of p38 MAPK. Moreover, it is reported that a p38 MAPK inhibitor, SB203580, is capable of inhibiting cellular senescence due to cellular stress. In experimental results which were exemplified in the Examples of the invention, it was indicated that SB203580 not only exerted an effect of suppressing fibrosis, but also suppressed decrease in the degree of cell density to enable culturing of corneal endothelial cells of high degree of density. Thus, it is understood that, when used in the present invention, any aging inhibitor can suppress decrease in the degree of density of cells and improve culturing of corneal endothelial cells of high degree of density.

As used herein, judgment for "suppressing senescence" is based on the capability of suppressing decrease in the degree of density of corneal endothelial cells while maintaining the high degree of density. The density of corneal endothelium is known to be decreased in accordance with senescence in a living body (Kunitoshi OHARA, Tadahiko TSURU, Shigeru INODA: Kakumaku Naihi Saibou Keitai No Parameter [Parameter of Corneal Endothelial Cell Form]. Nippan Ganka Gakkai Zasshi 91:1073-1078, 1987), which is also a good index for judging senescence from the clinical point of view. In addition, while decrease in nucleus/cytoplasm ratio is a typical index for cellular senescence, the ratio can also be used for corneal endothelium. In addition, other examples for the aging inhibitor include, without limitation, other p38 MAP kinase inhibitors.

As used herein, "p38 MAP kinase inhibitor" refers to any agent for inhibiting signaling of MAP kinase associated with p38. Thus, a p38 MAP kinase inhibitor is related to a compound targeting a MAPK family member, p38-MAPK, for decreasing or inhibiting.

The p38 is a mammalian MAPK superfamily member, and is activated by stress, ultraviolet radiation, and inflammatory cytokine. The catalytic core thereof has a TGY consensus sequence.

It is gradually recognized that aberrantly regulated kinase is a principal cause of disease for many disease, and in particular proliferative and inflammatory disorders. One of the cancer-causing genes that was identified first in a cancer region was the one for epithelial growth factor receptor kinase (EGFR), and the overexpression thereof is related to lung, breast, brain, prostate, GI and ovarian cancer. For example, constitutive activation of MAP kinase is associated with primary tumor derived from a variety of human organs (kidneys, large intestines, and lungs) and a large number of cancer cell lineages (pancreas, large intestines, lungs, ovaries, and kidneys) (Hoshino et al., Oncogene, 18(3) : 813-22 (Jan. 1999)). Furthermore, p38 MAP kinase regulates the production of two cytokines, TNFα and IL-1, which are associated with the onset and progress of inflammation. The p38 MAP kinase inhibitor also plays a role in time to come in the treatment of inflammatory diseases such as rheumatoid arthritis, and in addition, in the treatment of cardiac failure, stroke, neurogenic diseases, and other diseases. As such, the MAP kinase inhibitor is useful for the treatment of various kinds of disease conditions, from cancer to inflammation.

The p38 MAP kinase inhibitor that may be used in the present invention are not particularly limited so long as it is a compound having activity for inhibitingp38 MAP kinase, in addition to VX-745 (Vertex Pharmaceuticals Inc.); and includes compounds described in patent publications, such as Japanese Laid-Open Publication No. 2002-97189, Japanese National Phase PCT Laid-open Publication No. 2000-503304, Japanese National Phase PCT Laid-open Publication No. 2001-522357, Japanese National Phase PCT Laid-open Publication No. 2003-535023, Japanese National Phase PCT Laid-open Publication No. 2001-506266, Japanese National Phase PCT Laid-open Publication No. 9-508123, International Publication No. WO 01/56553, International Publication No. WO 93/14081, International Publication No. WO 01/35959, International Publication No. WO 03/68229, International Publication No. WO 03/85859, Japanese National Phase PCT Laid-open Publication No. 2002-534468, Japanese National Phase PCT Laid-open Publication No. 2001-526222, Japanese National Phase PCT Laid-open Publication No. 2001-526223, US Patent No. 6344476, International Publication No. WO 03/99811, International Publication No. WO 03/99796, Japanese National Phase PCT Laid-open Publication No. 2004-506042, International Publication No. WO 04/60286, Japanese National Phase PCT Laid-open Publication No. 2002-363179, Japanese National Phase PCT Laid-open Publication No. 2004-107358, US Patent No. 5,670,527, US Patent No. 6,096,753, International Publication No. WO 01/42189, International Publication No. WO 00/31063. Preferably, the compounds are 4-(4-fluorophenyl)-2-(4-hydroxyphenyl)-5-(4-pyridyl)-1H -imidazole(SB-202190), trans-4-[4-(4-fluorophenyl)-5-(2-methoxy-4-pyrimidinyl) -1H-imidazole-1-yl]cyclohexanol(SB-239063), 4-(4-fluorophenyl)-2-(4-methylsulfinylphenyl)-5-(4-pyri dyl)-1H-imidazole(SB-203580), 4-(4-fluorophenyl)-5-(2-methoxypyrimidine-4-yl)-1-(pipe ridine-4-yl)imidazole (SB-242235), 4-(4-fluorophenyl)-2-(4-hydroxy-1-butynyl)-1-(3-phenylp ropyl)-5-(4-pyridyl)imidazole(RWJ-67657), 4-(4-fluorophenyl)-1-(piperidine-4-yl)-5-(4-pyridyl)imi dazole(HEP-689), (S)-2-(2-amino-3-phenylpropylamino)-1-methyl-5-(2-napht hyl)-4-(4-pyridyl)pyrimidine-6-one(AMG-548), 2-chloro-4-(4-fluoro-2-methylanilino)-2'-methylbenzophe none(EO-1606), 3-(4-chlorophenyl)-5-(1-hydroxyacetylpiperidine-4-yl)-4 -(pyrimidine-4-yl)pyrazole(SD-06), 5-(2,6-dichloro phenyl)-2-(2,4-difluorophenylthio)pyrimido[3,4-b]pyrida zine-6-one(VX-745), 4-acetylamino-N-tert-butylbenzamide(CPI-1189), N-[3-tert-butyl-1-(4-methylphenyl)pyrazole-5-yl)-N'-[4-(2-morpholinoethoxy)-1-naphthyl]urea(Dramapimod), 2-benzamide-4-[2-ehtyl-4-(3-methylphenyl)thiazole-5-yl] pyridine(TAK-715), SCIO-469, VX-702, GSK-681323, PS-540446, SC-80036, AVE-9940, RO-320-1195, SB-281832, SCIO-323, KC-706, N,N' -bis[3,5-bis[1-(2-amidinohydrazono)ehtyl]p henyl]decanediamide, N,N'-bis[3,5-bis[1-[2-(aminoiminomethyl)hydrazono]ehtyl ]phenyl]decanediamide(Semapimod).

Furthermore, Tocris Cookson (St Louis, USA) provides a variety of MAP kinase inhibitors exemplified at http://www.tocris.com/. For example, SB202190 (4-[4-(4-fluorophenyl)-5-(4-pyridinyl)-1H-imid azole-2-yl] phenol) is a p38 MAP kinase inhibitor which is highly selective, strong, and cell permeable (SmithKline Beecham, plc) (Jiang et al., J. Biol. Chem., 271:17920 (1996) ; Frantz et al, Biochemistry, 37:138-46 (1998) ; Nemoto et al, J. Biol. Chem., 273:16415 (1998); and Davies et al, Biochem. J., 351:95 (2000)). In addition, anisomycin ((2R,3S,4S)-2-[(4-methoxyphenyl)methyl]-3,4-pyrrolidine diol-3-acetate) is a protein synthetic inhibitor (which blocks translation) . This is a strong activator for stress activated protein kinase (JNK/SAPK) and p38 MAP kinase, and acts as a strong signaling agonist for selectively inducing homologous desensitization induced by an immediate early gene (c-fos, fosB, c-jun, junB, and junD). PD98059 (2-(2-amino-3-methoxyphenyl)-4H-1-benzopyran-4-one) is a speciic inhibitor of mitogen activated protein kinase kinase (MAPKK) (Pfizer = Warner-Lambert Company). SB203580 (4-[5-(4-fluorophenyl)-2-[4-(methylsulfonyl)phenyl]-1H-imidazole-4-yl]pyridine) is a highly selective inhibitor (SmithKlineBeecham, plc) of p38 mitogen activated protein kinase. It is indicated to inhibit interleukin-2-induced T cell growth, cyclooxygenase-1 and -2, and thromboxane synthase. SB203580 hydrochloride (4-[5-(4-fluorophenyl)-2-[4-(methylsulfonyl)phenyl]-1H-imidazole-4-yl]pyridine) compound is a water-soluble salt of an inhibitor of p38 mitogen activated protein kinase, which is highly selective. It is indicated to inhibit interleukin -2-induced T cell growth, cyclooxygenase-1 and -2, and thromboxane synthase. U0126 (1,4-diamino2,3-dicyanol,4-bis[2-aminophenylthio] butadiene) is a string and selective, non-competitive inhibitor of MAP kinase kinase.

As to a preferable p38 MAPK inhibitor, without limitation, SB203580 (4-[4-(4-fluorophenyl)-2-(4-methylsulfinylphenyl)-1H-im idazole-5-yl]pyridine) is exemplified.

As used herein, "cell adhesion promoting agent" or "adhesion promoting agent" of a corneal endothelial cell refers to an agent for providing or improving an adhesive property of a cell, and any agent can be used so long as the agent has such a function . An exemplary adhesion promoting agent for corneal endothelial cells includes, without limitation, Rho kinase inhibitors.

In the present invention, "Rho kinase" means serine/threonine kinase which is activated in accordance with activation of Rho. For example, included are ROKα (ROCK-II: Leung, T. et al., J. Biol. Chem., 270, 29051-29054, 1995), p160ROCK (ROKβ, ROCK-I: Ishizaki, T. et al., TheEMBOJ., 15(8), 1885-1893, 1996) and other proteins having serine/threonine kinase activity.

Rho kinase inhibitors include compounds disclosed in the following documents: US Patent No. 4678783, Japanese Patent No. 3421217, International Publication No. WO 95/28387, International Publication No. WO 99/20620, International Publication No. WO 99/61403, International Publication No. WO 02/076976, International Publication No. WO 02/076977, International Publication No. WO 2002/083175, International Publication No. WO 02/100833, International Publication No. WO 03/059913, International Publication No. WO 03/062227, International Publication No. WO 2004/009555, International Publication No. WO 2004/022541, International Publication No. WO 2004/108724, International Publication No. WO 2005/003101, International Publication No. WO 2005/039564, International Publication No. WO 2005/034866, International Publication No. WO 2005/037197, International Publication No. WO 2005/037198, International Publication No. WO 2005/035501, International Publication No. WO 2005/035503, International Publication No. WO 2005/035506, International Publication No. WO 2005/080394, International Publication No. WO 2005/103050, International Publication No. WO 2006/057270, International Publication No. WO 2007/026664 and the like. The subject compounds each can be manufactured by the methods described in the documents in which the respective compounds are disclosed. The specific examples include 1-(5-isoquinolinesulfonyl) homopiperazine or a salt thereof (e.g., fasudil(1-(5-isoquinolinesulfonyl)homopiperazine)), (+)-trans-4-(1-aminoethyl)-1-(4-pyridylcarbamoyl)cycloh exane((R)-(+)-trans-(4-pyridyl)-4-(1-aminoethyl)-cycloh exanecarboxamide) or a salt thereof (e.g., Y-27632((R)-(+)-trans-(4-pyridyl)-4-(1-aminoethyl)-cycl ohexanecarboxamide2 hydrochloride lhydrate) and the like) and the like. As to these compounds, commercialized product (Wako Pure Chemical Industries, Ltd, Asahi Kasei Pharma Corporation and the like) can also be preferably used.

(+)-trans-4-(1-aminoethyl)-1-(4-pyridylcarbamoyl)cy clohexane, 1-(5-isoquinolinesulfonyl)homopiperazine and a pharmaceutically acceptable salt thereof and the like are particularly excellent for adhesion promotion of corneal endothelial cells, and thus they are preferably used. As to the salt of the compound, a pharmaceutically acceptable acid addition salt is preferable, and such an acid includes muriatic acid, hydrobromic acid, sulfuric acid and other inorganic acid, and methanesulfonic acid, fumaric acid, maleic acid, mandelic acid, citric acid, tartaric acid, salicylic acid and other organic acid. (+)-trans-4-(1-aminoethyl)-1-(4-pyridylcarbamoyl)cycloh exane((R)-(+)-trans-(4-pyridyl)-4-(1-aminoethyl)-cycloh exanecarboxamide)•2 hydrochloride (which may also be monohydrate), and 1-(5-isoquinolinesulfonyl)homopiperazine hydrochloride are more preferable.

In the present invention, "adhesion promotion of corneal endothelial cells" includes, for example, both adhesion promotion of cells of corneal endothelium, and adhesion promotion of a corneal endothelial cell and a culture substrate.

The (cell) adhesion promoting agent that can be used in the present invention exerts an adhesion promotion action to a corneal endothelial cell separated from a corneal tissue derived from a mammal (e.g., humans, mice, rats, hamsters, rabbits, cats, dogs, cows, sheep, monkeys and the like) or a separated and subcultured corneal endothelial cell. The adhesion promoting agent according to the present invention is excellent in an adhesion promotion action of human-derived corneal endothelial cells, which are particularly considered to be difficult to culture and subculture. Thus, it is preferable to define human-derived corneal endothelial cell as the object.

Corneal endothelial cells play a role in maintaining the degree of transparency of cornea. If the density of the endothelial cells is decreased below a certain limit, swelling will occur in the cornea and the degree of transparency will not be maintained in the cornea, resulting in a corneal endothelial damage . The adhesion promoting agent that can be used in the present invention promotes adhesion of a corneal endothelial cell, making it possible to improve the formation of a corneal endothelial cell layer having a favorable cell form and high cell density.

As used herein, "substance (e.g., nucleic acid) for suppressing expression (of TGF-β or the like)" is not particularly limited so long as such a substance is a substance which suppresses transcription of mRNA of a target gene, a substance which degrades a transcribed mRNA (e.g., nucleic acid), or a substance (e.g., nucleic acid) which suppresses translation of protein from mRNA. As to the substances, exemplified are siRNA, antisense oligonucleotide, ribozyme, an expression vector thereof and other nucleic acids. Among them, siRNA and an expression vector thereof are preferable, and siRNA is particularly preferable. "Substance which suppresses expression of a gene" includes, in addition to those described above, protein, peptide, and other small molecules. Note that a target gene herein means any gene that is associated with a TGF-β signaling pathway.

As to a method for inhibiting the expression of a specific endogenous gene, such as TGF-β, that is targeted in the present invention, a method utilizing an antisense technique is well known to those skilled in the art. As to actions for an antisense nucleic acid to inhibit the expression of a target gene, there are a plurality of factors as follows. Specifically, such factors are: inhibition of transcript initiation due to triplex formation; inhibition of transcription due to hybrid formation with a site where an open loop structure is locally formed due to RNA polymerase; inhibition of transcription due to hybrid formation with an RNA whose synthesis is in progress; splicing inhibition due to hybrid formation at a junction of intron and exon; splicing inhibition due to hybrid formation with spliceosome forming site; transfer inhibition from a nucleus to cytoplasm due to hybrid formation with mRNA; splicing inhibition due to hybrid formation with a capping site or a poly (A) addition site; inhibition of translation initiation due to hybrid formation with a translation initiation factor binding site; translational inhibition due to hybrid formation with a ribosome binding site near an initiation codon; elongation inhibition of a peptide chain due to hybrid formation with a polysome binding site or a translation region of mRNA; and gene expression inhibition due to hybrid formation with a interaction site of a nucleic acid and a protein, and the like. As such, an antisense nucleic acid inhibits a variety of processes, such as transcription, splicing or translation, to inhibit the expression of a target gene (Hirashima and Inoue, Shinsei Kagaku Jikken Kouza [New Chemical Experiment Course] 2, Nucleic Acid, IV Idenshi no Fukusei to Hatsugen [Duplication and Expression of Gene], Edited by the Japanese Biochemical Society, Tokyo Kagaku Dozin, 1993, 319-347).

The antisense nucleic acid used in the present invention may inhibit the expression and/or function or a gene (nucleic acid) encoding a member or the like of a signaling pathway of the above-mentioned TGF-β by any of the above-mentioned actions. In one embodiment, it is considered to be effective for the translation inhibition of a gene when a complementary antisense sequence is designed in a non-translation region near 5' terminal of mRNA of a gene encoding the above-mentioned TGF-β or the like. In addition, it is possible to use a sequence complementary to a coding region or a 3' non-translation region. As such, the translation region of a gene encoding the above-mentioned TGF-β or the like as well as a nucleic acid including an antisense sequence of a sequence of a non-translation region are included in the antisense nucleic acid that are used in the present invention. The antisense nucleic acid used is connected to a downstream of an appropriate promoter, and is preferably connected to a sequence including a transcription termination signal on the side closer to 3'. A nucleic acid prepared in such a manner can be transformed into a desired animal (cell) using a publicly known method. While the sequence of the antisense nucleic acid is preferably a sequence complementary to a gene, or a part thereof, encoding TGF-β or the like of an animal (cell) to be transformed, it does not have to be completely complementary so long as the sequence can effectively suppress the expression of genes. The transcribed RNA preferably has 90% or more, and most preferably 95% or more, complementarity to a transcription product of a target gene. In order to effectively inhibit the expression of a target gene using an antisense nucleic acid, the length of the antisense nucleic acid is preferably at least 12 bases or more but less than 25 bases long. However, the antisense nucleic acid according to the present invention is not necessarily limited to this length, and the antisense nucleic acid may be, for example, 11 bases or less, 100 bases or more, or 500 bases or more. While the antisense nucleic acid may be composed of DNA only, it may also include nucleic acids other than DNA, such as locked nucleic acid (LNA). In one embodiment, the antisense nucleic acid used in the present invention may be a LNA-containing antisense nucleic acid including LNA at the 5' terminal, and LNA at the 3' terminal. Furthermore, in an embodiment where an antisense nucleic acid is used in the present invention, an antisense sequence can be designed based on a nucleic acid sequence, such as TGF-β, using a method described in Hirashima and Inoue, Shinsei Kagaku Jikken Kouza [New Chemical Experiment Course] 2, Nucleic Acid, IV Idenshi no Fukusei to Hatsugen [Duplication and Expression of Gene], Edited by the Japanese Biochemical Society, Tokyo Kagaku Dozin, 1993, 319-347, for example.

The inhibition of expression of TGF-β or the like can also be performed by using ribozyme, or DNA encoding ribozyme. The ribozyme refers to a RNA molecule having catalytic activity. There are various types of ribozymes having various types of activities, and researches focusing on a ribozyme as an enzyme for cleaving RNA has made it possible to design a ribozyme for cleaving RNA in a site-specific manner. While ribozymes include those with 400 nucleotides or more in size, such as group I intron type and M1 RNA included in RNase P, there are also such ribozymes having an activity domain of as many as 40 nucleotides, such as those referred to as hammer head type and hairpin type (Makoto Koizumi and Eiko Ohtsuka, Tanpakushitu Kakusan Kouso [Protein Nucleic Acid Enzyme], 1990, 35, 2191).

For example, the self-cleavage domain of the hammer head type ribozyme cleaves the side closer to 3' of C15 in a sequence referred to as G13U14C15, and the base-pair formation of U14 and A9 is considered to be important for the activity thereof; and it is indicated that cleavage can be made by A15 or U15, instead of c15 (Koizumi, M. et al., FEBS Lett, 1988, 228, 228). If a ribozyme is designed in which a substance binding site is complementary to a RNA sequence near a target site, a restriction enzymic RNA cleavage ribozyme can be created which recognizes a sequence such as UC, UU or UA in a target RNA (Koizumi, M. et al., FEBS Lett, 1988, 239, 285., Makoto Koizumi and Eiko Ohtsuka, Tanpakushitu Kakusan Kouso [Protein Nucleic Acid Enzyme], 1990, 35, 2191., Koizumi, M. et al., Nucl. Acids Res., 1989, 17, 7059).

In addition, hairpin type ribozyme are also useful for the purpose of the present invention. Such a ribozyme is found in, for example, a negative strand of a satellite RNA of tobacco ringspot virus (Buzayan, JM., Nature, 1986, 323, 349.). It is indicated that a target-specific RNA cleavage ribozyme can be created from hairpin type ribozyme (Kikuchi, Y. & Sasaki,N., Nucl. Acids Res,1991, 19, 6751., Kikuchi, Yo, Kagaku to Seibutu [Chemistry and Living Organism], 1992, 30, 112.) . As such, a transcription product of a gene encoding TGF-β or the like is specifically cleaved using ribozyme, so that the expression of the gene can be inhibited.

Suppression of expression of an endogenous gene of TGF-β or the like can also be performed by RNA interference (hereinafter, abbreviated as "RNAi") using a double-stranded RNA having a sequence identical or similar to a target gene sequence. When the RNAi double-stranded RNA (dsRNA) is taken directly into a cell, expression of a gene having a sequence homologous to the dsRNA is suppressed, which is a method that is currently attracting attention. In mammalian cells, a short strand dsRNA (siRNA) is used so that RNAi can be induced. In comparison with knockout mice, RNAi has many advantages, such as high stability, easy experimentation, and inexpensive cost. The siRNA will be described in detail in a different part of the present specification.

As used herein, "siRNA" refers to an RNA molecule having a double-stranded RNA moiety consisting of 15 to 40 bases, and the siRNA has a function of cleaving mRNA of a target gene having a sequence complementary to an antisense strand of said siRNA and suppressing the expression of the target gene. More specifically, the siRNA according to the present invention is an RNA including a double-stranded RNA moiety consisting of a sense RNA chain consisting of a sequence homologous to a contiguous RNA sequence in mRNA of TGF-β or the like, and an antisense RNA chain consisting of a sequence complementary to the sense RNA sequence. The manufacturing and designing of the siRNA and a mutant siRNA to be described below are within the scope of the ability of those skilled in the art. The concept of selecting any contiguous RNA region of mRNA, which is a transcription product of a sequence of TGF-β or the like, and creating a double-stranded RNA corresponding to the region is merely a matter that those skilled in the art can perform within the normal creative ability of them. Furthermore, the concept of selecting a siRNA sequence with a more powerful RNAi effect from an mRNA sequence, which is a transcription product of the subject sequence, can be appropriately performed by those skilled in the art using a publicly known method. Furthermore, if one of the strands is identified, it is easy for those skilled in the art to determine a base sequence of the other strand (complementary strand). Those skilled in the art can appropriately create siRNA using a commercially available nucleic acid synthesizing machine. In addition, synthesis entrustment service can be generally used for desired RNA synthesis.

The length of the double-stranded RNA moiety is, as a base, 15 to 40 bases, preferably 15 to 30 bases, more preferably 15 to 25 bases, still more preferably 18 to 23 bases, and most preferably 19 to 21 bases. It is understood that the upper and lower limits thereof are not limited to the specified ones, but the limits can be any combinations of the listed ones. As to a terminal structure of a sense strand or antisense strand of siRNA, there is no particular limitation, and it can be appropriately selected depending on the purpose. For example, the terminal structure may be the one having a flush terminal or the one having protruding terminal (overhang), and the type with protruded 3' terminal is preferable. A siRNA having an overhang consisting of several bases, preferably 1 to 3 bases, and still preferably 2 bases, at the 3' terminal of the sense RNA strand and antisense RNA strand often has a great effect of inhibiting the expression of a target gene, which is preferable. The type of the bases of overhang is not particularly restricted, and the type can be either a base constituting an RNA or a base constituting a DNA. Preferable overhang sequences include dTdT (2bp deoxy T) at the 3' terminal, and the like. For example, preferable siRNAs include, without limitation, those in which dTdT (2bp deoxy T) is added to 3' terminal of the sense and antisense strands of all the siRNA.

Furthermore, it is also possible to use a siRNA in which one to several nucleotides are deleted, substituted, inserted and/or added in either or both of the sense strand and antisense strand of the above-mentioned siRNA. In this regard, the concept of one to several bases is not particularly limited, but it is preferably 1 to 4 bases, still preferably 1 to 3 bases, most preferably 1 to 2 bases. Specific examples of the subject mutation include, without limitation, those in which the number of bases at the 3' overhang moiety is from 0 to 3, those in which the base sequence of the 3' -overhang moiety is changed to another base sequence, those in which the length of the above-mentioned sense RNA strand and antisense RNA strand is different by 1 to 3 bases due to the insertion, addition or deletion of bases, those in which the base in a sense strand and/or antisense strand is substituted with another base, and the like. However, it is necessary for the sense strand and the antisense strand to be able to hybridize in these mutant siRNAs, and it is necessary for these mutant siRNAs to have an ability to inhibit gene expression equivalent to siRNAs that do not have mutation.

Furthermore, the siRNA may be a siRNA (Short Hairpin RNA; shRNA) in which one of the terminals have a molecule of a closed structure, such as a hairpin structure. The shRNA is a sense strand RNA of a specific sequence of a target gene, an antisense strand RNA consisting of a sequence complementary to the sense strand sequence, and a RNA including a linker sequence for connecting the both strands thereof, wherein the sense strand moiety and the antisense strand moiety hybridize to form a double-stranded RNA moiety.

The siRNA desirably does not exhibit a so-called off-target effect when clinically used. The off-target effect refers to an effect for suppressing the expression of another gene with partially homology to the siRNA used, other than the target gene. In order to avoid the off-target effect, it is possible to confirm that a candidate siRNA does not have cross reactivity using DNA microarray or the like in advance. Furthermore, it is possible to avoid the off-target effect by confirming as to whether there is a gene including a moiety having high homology with a sequence of a candidate siRNA, other than a target gene, using publicly known database provided by NCBI (National Center for Biotechnology Information) or the like.

In order to create the siRNA according to the present invention, a publicly known method, such as a method by chemical synthesis and a method using a gene recombination technique, can be appropriately used. With a method by synthesis, a double-stranded RNA can be synthesized based on sequence information, using an ordinary method. In addition, it is also possible to create such a siRNA by constructing an expression vector encoding a sense strand sequence and an antisense strand sequence and introducing the vector into a host cell, and then obtaining a sense strand RNA and an antisense strand RNA, each of which is produced by transcription. Furthermore, it is possible to create a desired double-stranded RNA by expressing a shRNA, which includes a sense strand of a specific sequence of a target gene, an antisense strand consisting of a sequence complementary to the sense strand sequence, and a linker sequence for connecting the both strands, and which forms a hairpin structure.

With regard to the siRNA, all or part of the nucleic acids constituting the siRNA may be a natural nucleic acid or a modified nucleic acid so long as such a nucleic acid has an activity to suppress the expression of a target gene.

The siRNA according to the present invention does not necessarily have to be a pair of double-stranded RNAs to a target sequence, and it may be a mixture of a plurality (the "plurality" is not particularly limited, but preferably refers to a small number of about 2 to 5) of double-stranded RNAs to a region which includes a target sequence. In this regard, those skilled in the art can appropriately create siRNA, as a nucleic acid mixture corresponding to a target sequence, using a commercially available nucleic acid synthesizing machine and DICER enzyme; and as to synthesis of a desired RNA, synthesis entrustment service can be generally used. Note that the siRNA according to the present invention includes a so-called "cocktail siRNA". Furthermore, note that the siRNA according to the present invention is such that not all the nucleotides have to be a ribonucleotide (RNA). Specifically, in the present invention, one or plurality of ribonucleotides constituting a siRNA may be a corresponding deoxyribonucleotide. The term "corresponding" refers to being the same base type (adenine, guanine, cytosine, thymine (uracil)) although the structure of the sugar portion is different. For example, a deoxyribonucleotide corresponding to a ribonucleotide having adenine refers to a deoxyribonucleotide having adenine.

Furthermore, a DNA (vector) which may express the above-mentioned RNA according to the present invention is also included in a preferred embodiment of a nucleic acid which may suppress expression of TGF-β or the like. For example, the DNA (vector) which may express the above-mentioned double-stranded RNA according to the present invention is such a DNA having a structure in which DNA encoding one of the strands of the double-stranded RNA and a DNA encoding the other of the strands of the double-stranded RNA are connected to a promoter so that each of the DNAs is capable of being expressed. The above-mentioned DNA according to the present invention can be appropriately created by those skilled in the art using a general genetic engineering technique. More specifically, the expression vector according to the present invention can be created by appropriately inserting the DNA encoding RNA according to the present invention, into a variety of publicly known expression vectors.

In the present invention, a modified nucleic acid may be used for the nucleic acid for suppressing the expression of a target gene. The modified nucleic acid means such a nucleic acid in which modification is provided at a nucleoside (base moiety, sugar moiety) and/or an inter-nucleoside binding site, and has a structure different from that of a natural nucleic acid. "Modified nucleoside", which constitutes a modified nucleic acid, includes, for example, abasic nucleoside; arabinonucleoside, 2'-deoxyuridine, a-deoxyribonucleoside, β-L-deoxyribonucleoside, nucleoside having other sugar modification; peptide nucleic acid (PNA), phosphate group-binding peptide nucleic acid (PHONA), locked nucleic acid (LNA), morpholino nucleic acid and the like. The above-mentioned nucleoside having sugar modification includes 2'-O-methylribose, 2'-deoxy-2'-fluororibose, 3'-O-methylribose and other substituted pentose; 1',2'-deoxyribose; arabinose; substituted arabinose sugar; and nucleoside having sugar modification of alpha-anomer and hexose. These nucleosides may be a modified base in which the base moiety is modified. Such modified bases include, for example, 5-hydroxycytosine, 5-fluorouracil, 4-thiouracil and other pyrimidine; 6-methyladenine, 6-thioguanosine and other purine ; and other heterocyclic bases.

"Modified inter-nucleoside binding", which constitutes a modified nucleic acid, includes non-natural inter-nucleoside binding, such as alkyl linker, glyceryl linker, amino linker, poly(ethylene glycol) binding, inter-methyl phosphonate nucleoside binding; methylphosphonothioate, phosphotriester, phosphothiotriester, phosphorothioate, phosphorodithioate, triester prodrug, sulfone, sulfonamide, sulfamate, Holm acetal, N-methylhydroxylamine, carbonate, carbamate, morpholino, boranophosphonate, phosphoramidate and the like.

The nucleic acid sequence included in the double-stranded siRNA according to the present invention includes a siRNA directed to a member of TGF-β or other TGF-β signaling members, and the like.

It is also possible to introduce the nucleic acid or agent according to the present invention into liposome or other phospholipid endoplasmic reticulums and administer the endoplasmic reticulum. An endoplasmic reticulum in which a siRNA or shRNA is retained can be introduced into a predetermined cell using a lipofection method. Then, the obtained cell is systemically-administered, for example intravenously, intra-arterially or the like. The endoplasmic reticulum can also be locally administered to a required site in an eye or the like. While the siRNA exhibits an extremely excellent specific post-transcription suppressing effect *in vitro,* it is quickly degraded *in vivo* due to nuclease activity in blood serum. Thus, the duration is limited, and because of this, there has been a need for development for a better and more effective delivery system. As to one example, Ochiya, T et al., Nature Med., 5:707-710, 1999, Curr. Gene Ther., 1: 31-52, 2001 reports as follows:
a biocompatible material, atelocollagen, is mixed with a nucleic acid to form a complex, which has an action for protecting a nucleic acid from a degrading enzyme in a living organism and which is a carrier that is extremely suitable as a carrier for siRNA. While such a form can be used, the method for introducing a nucleic acid or medicament according to the present invention is not limited to this method. As such, due to quick degradation by the action of the nucleic acid degrading enzyme in blood serum in a living organism, it becomes possible to achieve long-time continuation of the effect. For example, Takeshita F. PNAS. (2003) 102 (34) 12177-82, Minakuchi Y Nucleic Acids Research (2004) 32 (13) e109 reports as follows: atelocollagen derived from bovine skin forms a complex with a nucleic acid, which has an action for protecting a nucleic acid from degrading enzyme in a living organism and which is extremely suitable as a carrier of siRNA. Such a technique can be used.

As used herein, "culture medium" refers to any culture medium capable of maintaining or growing a corneal endothelial cell, and in an appropriate case as needed, the culture medium can take any form such as a liquid culture medium (culture solution), a suspension culture medium, a solid culture medium and the like. Ingredients of such a culture medium used for corneal endothelial cells include, for example, DMEM (GIBCO BRL), OptiMEM (Life Technologies), blood serum (e.g., fetal bovine serum (FBS)), proliferative factor/growth factor (e.g., b-FGF) , an antibiotic substance (such as penicillin, streptomycin, and gentamicin), and the like.

### (General Techniques)

The molecular biological technique, biochemical technique, and microbiological technique as used herein are well known and commonly used in the art, and they are described in, for example, Sambrook J. et al. (1989) . Molecular Cloning: A Laboratory Manual, Cold Spring Harbor and 3rd Ed. (2001) ; Ausubel, F. M. (1987) . Current Protocols inMolecularBiology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M. (1992). Short Protocols in Molecular Biology: A Compendium of Methods from Current Protocols in Molecular Biology, Greene Pub. Associates; Ausubel, F. M. (1995) . Short Protocols in Molecular Biology: A Compendium of Methods from Current Protocols in Molecular Biology, Greene Pub. Associates; Innis, M. A. et al. (1995) . PCR Strategies, Academic Press; Ausubel, F. M. (1999) . Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Gait, M. J. (1985). Oligonucleotide Synthesis: A Practical Approach,IRLPress; Gait, M. J. (1990). Oligonucleotide Synthesis: A Practical Approach, IRL Press; Eckstein, F. (1991). Oligonucleotides and Analogues : A Practical Approach, IRL Press; Adams, R. L. et al. (1992). The Biochemistry of the Nucleic Acids, Chapman & Hall; Shabarova, Z. et al . (1994) . Advanced Organic Chemistry of Nucleic Acids, Weinheim; Blackburn, G. M. et al. (1996). Nucleic Acids in Chemistry and Biology, Oxford University Press; Hermanson, G. T. (1996) . Bioconjugate Techniques, Academic Press, Jikken Igaku Bessatsu [Experimental Medicine, Separate Volume], "Idenshi Dounyu & Hatsugen Kaiseki Jikken [Gene Introduction & Expression Analysis Experimental Technique" Yodosha Co., Ltd., 1997, and the like. With regard to corneal endothelial cells, the report from Nancy Joyce et al., {Joyce, 2004 #161} {Joyce, 2003 #7} is well known, while researches are currently conducted for effective culturing methods by conducting transformation in a fibroblastic manner through long-term culturing and subculturing as described above.

### (Description of Preferred Embodiment)

Hereinafter, preferred embodiments will be described, but it should be understood that the embodiments are exemplification of the present invention and the scope of the present invention is not limited to such preferred embodiments. It should also be understood that those skilled in the art can easily perform alteration, change and the like within the scope of the present invention with reference to the following preferable Examples.

### (Culture Normalizing Agent)

In one aspect, the present disclosure provides a culture normalizing agent of a corneal endothelial cell, including a fibrosis inhibitor. Prior to the provision of the present invention, it was difficult to grow a corneal endothelial cell while maintaining a form suitable for transplant. In particular, transplant would be difficult if subculturing is repeated over and over again . The loss of functional protein indicates that it may be difficult to conduct transplant. Conventionally, morphological change was known to occur in a normal culturing method. In the present invention, since this was morphologically fibroblastic like, it was considered to be fibrotic change, which was found to be involved with activation of a TGF-β signaling. In this regard, the activation of the TGF-β signal can be judged, as exemplified in the Examples, by examining the amount, level and the like of fibronectin and collagen type 1, type 4 and type 8 fibronectin, integrin α5, and integrin β1 and other extracellular matrices or integrin. It is not intended to be limiting, but the protein expression level of fibronectin is strongly up-regulated in the phenotype of fibroblast compared to the normal phenotype. Conventionally, it has been found that fibrosis of a corneal endothelial cell is involved in an extremely rare disease, such as congenital syphilis, in a living body; however, there has been no development of a treatment method for suppressing fibrosis. Thus, it was not possible to anticipate as to whether or not a normal function could be maintained by suppressing fibrosis with drugs under such disease and culturing conditions . When the inventors used an agent known to suppress fibrosis, with a TGF-β signal inhibiting agent as a representative example in other cells, culturing with suppressed morphological change became possible and normal function of the cells were unexpectedly maintained during subculturing (that is, culture normalization became possible). As a result, the inventors discovered a method to achieve significant growth of corneal endothelial cells. It was conventionally impossible to culture a large amount of corneal endothelial cells while maintaining the normal function. Thus, the effect achieved by the present invention should be indeed considered significant.

In the present invention, it is possible to include a fibrosis inhibitor alone, and it is also possible to include several types in conjunction with each other as needed.

The concentration of the fibrosis inhibitor used in the present invention is, without limitation, normally about 0.1 to 100 µmol/l, preferably about 0.1 to 30 µmol/l, and more preferably about 1 µmol/l; when several types thereof are used, the concentration may be changed appropriately, and other concentration ranges include, for example, normally about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5. 0 µmol/l, about 1.0 to 5. 0 µmol/l, about 1.25 to 5. 0 µmol/l, about 1.5 to 5.0 µmol/l, about 1.75 to 5.0 µmol/l, about 2.0 to 5.0 µmol/l, about 2.5 to 5.0 µmol/l, about 3.0 to 5. 0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, more preferably, about 0.05 to 1.0 µmol/l, about 0.075 to 1.0 µmol/l, about 0.1 to 1.0 µmol/l, about 0.5 to 1.0 µmol/l, and about 0.75 to 1.0 µmol/l.

In the present invention, when used for a culture medium or the like, a culture normalizing agent alone can be included, and several types thereof can be included in conjunction with each other as needed; and a single effective ingredient can be included in the culture normalizing agent itself, and several types thereof can also be included in conjunction with each other as needed.

The concentration of the culture normalizing agent according to the present invention, used in a culture medium or the like, is without limitation, normally about 0.1 to 100 µmol/l, preferably about 0.1 to 30 µmol/l, more preferably about 1 µmol/l, when several types thereof are used, the concentration may be changed appropriately, and other concentration ranges include, for example, normally about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5. 0 µmol/l, about 1.0 to 5. 0 µmol/l, about 1.25 to 5. 0 µmol/l, about 1.5 to 5.0 µmol/l, about 1.75 to 5.0 µmol/l, about 2.0 to 5.0 µmol/l, about 2.5 to 5.0 µmol/l, about 3.0 to 5. 0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, andmorepreferably, about 0.05 to 1.0 µmol/l, about 0.075 to 1.0 µmol/l, about 0.1 to 1.0 µmol/l, about 0.5 to 1.0 µmol/l, and about 0.75 to 1.0 µmol/l.

The TGF-β signaling pathways are largely classified into a Smad 2/3 system via ALK 4, 5 or 7, and a Smad1/5/8 system via ALK 1, 2, 3 or 6; and both of them are well known to be associated with fibrosis (J. Massagu'e, Annu. Rev. Biochem. 1998. 67:753-91; Vilar JMG, Jansen R, Sander C (2006) PLoS Comput Biol 2 (1) : e3; Leask, A., Abraham, D. J. FASEB J. 18, 816-827 (2004); Coert Margadant & Arnoud Sonnenberg EMBO reports (2010) 11, 97-105; Joel Rosenbloomet al., Ann Intern Med. 2010; 152 : 159-166.) . BMP-7 is known to suppress a TGF-β signaling and is known to be able to suppress fibrosis (in addition to the above-mentioned documents, Ralf Weiskirchen, et al., Frontiers in Bioscience 14, 4992-5012, June 1, 2009; Elisabeth M Zeisberg et al., Nature Medicine 13, 952-961 (2007); Michael Zeisberg et al., Nature Medicine 9, 964-968 (2003)) . However, while Non Patent Literature 2 and 4 describe involvement of TGF-β with regard to a state associated with membranous tissues actually consisting of an extracellular matrix, such as collagen, by an extremely rare disease, syphilitic keratitis parenchymatosa, or an artificially-created severe disorder, it is difficult to achieve maintenance of normalization from this. In addition, Non Patent Literature 5 indicates that fibrosis during a severe lesion at a cornea is due to IL-1β, and due to activation of p38 MAPK in the course. Non Patent Literature 6 indicates, with rabbits, that fibrosis, observed when severe inflammation occurred in the living organism due to excess external injury by freezing, involves activation of p38 MAPK, and part of fibrosis can be suppressed by an inhibitor, with rabbits. These documents indicate that activation of p38 MAPK is involved in a situation when an extremely strong inflammation occurs in a living organism and membranous tissues consisting of an extracellular matrix are involved. The documents do not mention that fibrosis occurs in a normal culturing state, and they do not mention that TGF-β signal inhibiting agent and p38 MAPK inhibitor are effective for maintaining normalization. The subject documents do not provide any suggestion with regard to maintaining of a normal state. As such, it was previously considered to be difficult to culture corneal endothelial cells while maintaining the normal function, and Non Patent Literature 7 and the comparative examples in the present specification, and the like demonstrate that the culture media reported in Non Patent Literature 7 to 11 and the like were after all not able to maintain the normalization ability. Still more, it was not considered to be possible to normalize the culturing of corneal endothelial cells by fibrosis suppression or suppression of a TGF-β signaling pathway.

In one embodiment, the fibrosis inhibitor used in the present invention includes a transforming growth factor (TGF) β signal inhibiting agent. Thus, the present disclosure provides a culture normalizing agent for corneal endothelial cells, including a TGF-β signal inhibiting agent. The TGF-β signal inhibiting agent used in the present invention may be any agent as long as the agent can inhibit the signal pathway of TGF-β. In addition, as is well known, the TGF-β signaling pathway to be inhibited may be a factor associated with any signaling pathways, as long as such a factor ultimately exerts an effect similar (opposite in a case of an inhibitor, an antagonist or the like) to the signaling pathway of TGF-β, like BMP-7, in addition to factors that are directly associated with which the TGF-β and TGF-β receptor.

In the present invention, it is possible to include a TGF-β signal inhibiting agent alone, and it is also possible to include several types thereof in combination with each other as needed.

The concentration of the TGF-β signal inhibiting agent used in the present invention is, without limitation, normally about 0.1 to 100 µmol/l, preferably about 0.1 to 30 µmol/l, and more preferably about 1 µmol/l; when several types thereof are used, the concentration may be changed appropriately, and other concentration ranges include, for example, normally, about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5.0 µmol/l, about 1.0 to 5.0 µmol/l, about 1.25 to 5.0 µmol/l, about 1.5 to 5.0 µmol/l, about 1.75 to 5.0 µmol/l, about 2.0 to 5.0 µmol/l, about 2.5 to 5.0 µmol/l, about 3.0 to 5.0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, and more preferably, about 0.05 to 1.0 µmol/l, about 0.075 to 1.0 µmol/l, about 0.1 to 1.0 µmol/l, about 0.5 to 1.0 µmol/l, and about 0.75 to 1.0µmol/l.

In one embodiment, culture normalization includes a cellular function being normal, which is selected from the group consisting of those that express ZO-1 and Na⁺/K⁺-ATPase, that are morphologically polygonal and that are not multi-layered.

In one embodiment, culture normalization is for manufacturing a cell for transplantation which adapts to corneal transplantation. In a preferred embodiment, the above-mentioned cell for transplantation is a cell of a primate. In one preferred embodiment, the above-mentioned cell for transplantation is a cell of a human.

In one embodiment, the TGF-β signal inhibiting agent includes at least one of an antagonist of TGF-β, an antagonist of a receptor of TGF-β or an inhibitor of Smad3, other ingredients exemplified in the present specification, a pharmaceutically acceptable salt or a solvate thereof, or a solvate of a pharmaceutically acceptable salt thereof. As for the antagonist of TGF-β, the antagonist of a receptor of TGF-β, and the inhibitor of Smad3, any one of them described in other parts of the present specification can be used.

In one embodiment, the TGF-β signal inhibiting agent which can be used in the present invention includes at least one of SB431542 (4-[4-(1,3-benzodioxol-5-yl)-5-(2-pyridinyl)-1 H-imidazol-2-yl]benzamide), BMP-7, anti-TGF-β antibody, anti-TGF-β receptor antibody, siRNA of TGF-β, siRNA of a TGF-β receptor, antisense oligonucleotide of TGF-β, 6,7-dimethoxy-2-((2E)-3-(1-methyl-2-phenyl-1H-pyrrolo[2 ,3-b]pyridine-3-yl-prop-2-enoyl))-1,2,3,4-tetrahydroiso quinolone,
A83-01 (3-(6-methyl-2-pyridinyl)-N-phenyl-4-(4-quinolin yl)-1H-pyrazole-1-carbothioamide), Stemolecule™ TLK inhibitor (2-(3-(6-methylpyridine-2-yl)-1H-pyrazole-4-yl)-1,5-nap hthyridine), Stemolecule™ BMP inhibitor LDN-193189(6-(4-(piperidine-1-yl)ethoxy)phenyl)-3-(pyri dine-4-yl)pyrazolo[1,5-a]pyrimidine), SD-208(2-(5-chloro-2-fluorophenyl)-4-[(4-pyridinyl)amin o]pteridine), LY364947(4-[3-(2-pyridinyl)-1H-pyrazole-4-yl]-quinoline ), other ingredients exemplified in the present specification, a pharmaceutically acceptable salt or a solvate thereof, or a solvate of a pharmaceutically acceptable salt thereof. Although it is not desired to be restricted by theories, since normalization is observed in both of SB431542, which exerts an effect via Smad2/3(associated with ALK4, 5 and 7), and BMP-7, which exerts an effect via Smad1/5/8(associated with ALK1, 2, 3 and 6), it is understood that the TGF-β signal inhibiting agent of either of the pathways can achieve the effect of the present invention.

In a preferred embodiment, the TGF-β signal inhibiting agent used in the present invention includes SB431542 (4-[4-(1,3-benzodioxol-5-yl)-5-(2-pyridinyl)-1 H-imidazol-2-yl]benzamide). This is because fibrosis was suppressed, and moreover, it was indicated that the protein in charge of normal functions was retained, and transplant to primates was bearable. In a preferred embodiment, SB431542 is included to be present at a concentration of about 0.1 µM to about 10 µM in use, preferably included to be present at a concentration of about 1 µM to about 10 µM in use, and still preferably included to be present at a concentration of about 1 µM in use.

In another preferred embodiment, the TGF-β signal inhibiting agent used in the present invention includes BMP-7. This is because fibrosis was suppressed, and moreover, it was indicated that the protein in charge of normal functions was retained, and transplant to primates was bearable. In a preferred embodiment, BMP-7 is included to be present at a concentration of about 10 ng/ml to about 1,000 ng/ml in use, and more preferably, included to be present at a concentration of about 100 ng/ml to about 1,000 ng/ml in use. BMP-7 may be included to be present at a concentration of about 100 ng/ml in use, or may be included to be present at a concentration of about 1,000 ng/ml.

In a preferred embodiment, the fibrosis inhibitor used in the present invention further includes a MAP kinase inhibitor. For the MAP kinase inhibitor targeted in the present invention, any agent may be used so long as the agent is capable of inhibiting the signal pathway of the MAP kinase. Furthermore, the MAP kinase signal to be inhibited is associated with phosphorylation of the MAP kinase; and while signals are transmitted to the upstream or downstream thereof, or there is a pathway to which other pathways join together as a minor stream, the signal may be any signal.

In the present invention, it is possible to include one type of MAP kinase inhibitor alone, or it is also possible to include several types thereof in combination with each other as needed.

The concentration of the MAP kinase agent used in the present invention includes, without limitation, normally about 0.1 to 100 µmol/l, preferably about 0.1 to 30 µmol/l, and more preferably about 1 µmol/l; when several types thereof are used, the concentration may be changed appropriately, and other concentration ranges include, for example, normally, about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5. 0 µmol/l, about 1.0 to 5. 0 µmol/l, about 1.25 to 5.0 µmol/l, about 1.5 to 5.0 µmol/l, about 1.75 to 5.0 µmol/l, about 2.0 to 5.0 µmol/l, about 2.5 to 5.0 µmol/l, about 3.0 to 5. 0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, andmorepreferably, about 0.05 to 1.0 µmol/l, about 0.075 to 1.0 µmol/l, about 0.1 to 1.0 µmol/l, about 0.5 to 1.0 µmol/l, and about 0.75 to 1.0 µmol/l.

In a preferred embodiment, the MAP kinase inhibitor used in the present invention includes other ingredients exemplified in the present invention, in addition to SB203580 (4-[4-(4-fluorophenyl)-2-(4-methylsulfinylphen yl)-1H-imidazole-5-yl]pyridine).

In another embodiment, the culture normalizing agent according to the present invention further includes an aging inhibitor. Herein, it is understood that any agent known to suppress cellular senescence may be used as the aging inhibitor that can be used.

In the present invention, it is possible to include one type of aging inhibitor alone, and it is also possible to include several types thereof in combination as needed.

The concentration of the aging inhibitor used in the present invention includes, without limitation, normally about 0.1 to 100 µmol/l, preferably about 0.1 to 30 µmol/l, and more preferably about 1 µmol/l; when several types thereof are used, the concentration may be changed appropriately, and other concentration ranges include, for example, normally, about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5. 0 µmol/l, about 1.0 to 5. 0 µmol/l, about 1.25 to 5. 0 µmol/l, about 1.5 to 5.0 µmol/l, about 1.75 to 5.0 µmol/l, about 2.0 to 5.0 µmol/l, about 2.5 to 5.0 µmol/l, about 3.0 to 5. 0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, andmorepreferably, about 0.05 to 1.0 µmol/l, about 0.075 to 1.0 µmol/l, about 0.1 to 1.0 µmol/l, about 0.5 to 1.0 µmol/l, and about 0.75 to 1.0 µmol/l.

In one embodiment, the aging inhibitor used in the present invention includes a p38 MAP kinase inhibitor.

In the present invention, it is possible to include one type of p38 MAP kinase inhibitor alone, and it is also possible to include several types thereof in combination with each other as needed.

The concentration of the p38 MAP kinase agent used in the present invention includes, without limitation, normally about 0.1 to 100 µmol/l, preferably about 0.1 to 30 µmol/l, and more preferably about 1µmol/l; when several types thereof are used, the concentration may be changed appropriately, and other concentration ranges include, for example, normally, about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l, about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5. 0 µmol/l, about 1.0 to 5. 0 µmol/l, about 1.25 to 5. 0 µmol/l, about 1.5 to 5.0 µmol/l, about 1.75 to 5.0 µmol/l, about 2.0 to 5.0 µmol/l, about 2.5 to 5.0 µmol/l, about 3.0 to 5. 0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, and more preferably, about 0.05 to 1.0 µmol/l, about 0.075 to 1.0 µmol/l, about 0.1 to 1.0 µmol/l, about 0.5 to 1.0 µmol/l, and about 0.75 to 1.0 µmol/l.

In one preferred embodiment, the aging inhibitor used in the present invention includes SB203580 (4-[4-(4-fluorophenyl)-2-(4-methylsulfinylphen yl)-1H-imidazole-5-yl]pyridine) .

In a still preferred embodiment, the present invention provides a culture normalizing agent including SB431542 (4-[4-(1,3-benzodioxol-5-yl)-5-(2-pyridinyl)-1 H-imidazol-2-yl]benzamide), and SB203580 (4-[4-(4-fluorophenyl)-2-(4-methylsulfinylphen yl)-1H-imidazole-5-yl]pyridine) . Due to the combination of the two agents, the normalization is maintained while the growth rate is increased and culturing with a sufficient cell density is further improved.

In another embodiment, the culture normalizing agent according to the present disclosure further includes a cell adhesion promoting agent. For the cell adhesion promoting agent used in the present invention, any agent may be used so long as the agent is capable of promoting cell adhesion.

In one preferred embodiment, the cell adhesion promoting agent used in the present invention includes 1-(5-isoquinolinesulfonyl)homopiperazine or a salt thereof (e.g., fasudil(1-(5-isoquinolinesulfonyl)homopiperazine)), (+)-trans-4-(1-aminoethyl)-1-(4-pyridylcarbamoyl)cycloh exanecarboxamide or a salt thereof (e.g., Y-27632((R)-(+)-trans-(4-pyridyl)-4-(1-aminoethyl)-cycl ohexanecarboxamide2 hydrochloride 1 hydrate) and the like) and other Rho kinase inhibitors.

The adhesion promoting agent that can be used in the present invention can be added to a culture normalizing agent or a culture medium, such as a culture solution, when corneal endothelial cells are cultured *in vitro.* A Rho kinase inhibitor is added to the culture normalizing agent or a culture medium to continue culturing, so that the Rho kinase inhibitor and the corneal endothelial cells contact with each other *ex vivo* to promote the adhesion of the corneal endothelial cells.

The culture medium that can be used in the present invention can include a culture medium used for culturing an endothelial cell (e.g., DMEM(GIBCO BRL)), blood serum (e.g., fetal bovine serum (FBS)), growth factor (e.g., (b-)FGF), an antibiotic substance (such as penicillin and streptomycin) and the like.

A Rho kinase inhibitor is included in the culture medium ingredient of the present invention to enhance the adhesion of a corneal endothelial cell, so that the cell is prevented from being dropped, making it possible to form a corneal endothelial cell layer having a favorable cell form and high cell density. Thus, the Rho kinase inhibitor is preferably used in a method for manufacturing the corneal endothelial formulation according to the present invention, as described herein. Furthermore, the culture solution according to the present invention is used also to maintain the corneal endothelial cell.

The culture normalizing agent according to the present disclosure may further contain a Rho kinase inhibitor. The Rho kinase inhibitor included in the present invention is as described above. As used herein, "cornea preservation solution" is a liquid solution for preserving a cornea piece extracted from a donor during a period until it is transplanted to a recipient, or for preserving a corneal endothelial cell prior to growth or after growth.

The culture normalizing agent according to the present disclosure may also be used as a cornea preservation solution. Such a cornea preservation solution, to which the culture normalizing agent according to the present invention may be added, includes a preservation solution which is normally used for corneal transplant (sclerocorneal piece preservation solution (Optisol GS: registered trademark), an eyeball preservation solution for corneal transplant (EPII: registered trademark), saline, phosphate buffered saline (PBS) and the like.

Herein, it is possible to include one type of Rho kinase inhibitor alone, and it is also possible to include several types thereof in combination with each other as needed.

The concentration of the Rho kinase inhibitor in the present invention includes, without limitation, normally 1 to 100 µmol/l, preferably 5 to 20 µmol/l, and more preferably 10 µmol/l; when several types thereof are used, the concentration may be changed appropriately, and other concentration ranges include, for example, normally, about 0.001 to 100 µmol/l, preferably, about 0.01 to 75 µmol/l, about 0.05 to 50 µmol/l, about 1 to 10 µmol/l, about 0.01 to 10 µmol/l, about 0.05 to 10 µmol/l, about 0.075 to 10 µmol/l, about 0.1 to 10 µmol/l, about 0.5 to 10 µmol/l, about 0.75 to 10 µmol/l, about 1.0 to 10 µmol/l, about 1.25 to 10 µmol/l, about 1.5 to 10 µmol/l, about 1.75 to 10 µmol/l, about 2.0 to 10 µmol/l, about 2.5 to 10 µmol/l, about 3.0 to 10 µmol/l, about 4.0 to 10 µmol/l, about 5.0 to 10 µmol/l, about 6.0 to 10 µmol/l, about 7.0 to 10 µmol/l, about 8.0 to 10 µmol/l about 9.0 to 10 µmol/l, about 0.01 to 50 µmol/l, about 0.05 to 5.0 µmol/l, about 0.075 to 5.0 µmol/l, about 0.1 to 5.0 µmol/l, about 0.5 to 5.0 µmol/l, about 0.75 to 5. 0 µmol/l, about 1.0 to 5. 0 µmol/l, about 1.25 to 5. 0 µmol/l, about 1.5 to 5.0 µmol/l, about 1.75 to 5.0 µmol/l, about 2.0 to 5.0 µmol/l, about 2.5 to 5.0 µmol/l, about 3.0 to 5. 0 µmol/l, about 4.0 to 5.0 µmol/l, about 0.01 to 3.0 µmol/l, about 0.05 to 3.0 µmol/l, about 0.075 to 3.0 µmol/l, about 0.1 to 3.0 µmol/l, about 0.5 to 3.0 µmol/l, about 0.75 to 3.0 µmol/l, about 1.0 to 3.0 µmol/l, about 1.25 to 3.0 µmol/l, about 1.5 to 3.0 µmol/l, about 1.75 to 3.0 µmol/l, about 2.0 to 3.0 µmol/l, about 0.01 to 1.0 µmol/l, about 0.05 to 1.0 µmol/l, about 0.075 to 1.0 µmol/l, about 0.1 to 1.0 µmol/l, about 0.5 to 1.0 µmol/l, about 0.75 to 1.0 µmol/l, about 0.09 to 35 µmol/l, about 0.09 to 3.2 µmol/l, andmorepreferably, about 0.05 to 1.0 µmol/l, about 0.075 to 1.0 µmol/l, about 0.1 to 1.0 µmol/l, about 0.5 to 1.0 µmol/l, and about 0.75 to 1.0 µmol/l.

The present invention prevents transformation of a cornea from occurring, enables normalized culturing, or enhances the adhesion of a corneal endothelial cell to prevent the cell from being detached, making it possible to form a corneal endothelial cell layer having a favorable cell form and high cell density. Thus, the present disclosure provides a preservation solution for cornea used for organ transplantation or the like. In addition, the culture normalizing agent used in the present invention is also used as a preservation solution for cryopreserving a corneal endothelial cell or an ingredient thereof. For cryopreservation, it is also possible to further add glycerol, dimethylsulfoxide, propylene glycol, acetamide and the like to the preservation solution according to the present invention.

In one embodiment, when the culture normalizing agent according to the present disclosure is used, a plurality of agents may be used separately as the culture normalizing agent. In such an embodiment, for example, the fibrosis inhibitor can be allowed to be present at all times during the culturing of said corneal endothelial cell, while the adhesion promoting agent can be allowed to be present for a certain period of time, and then the adhesion promoting agent can be once deleted, and the cell adhesion promoting agent can be allowed to be present for a certain period of time once again.

In another embodiment, when the culture normalizing agent according to the present disclosure is used, both of a fibrosis inhibitor and said cell adhesion promoting agent can be allowed to be present at all times during the culturing of said corneal endothelial cell.

In one embodiment, the corneal endothelial cell cultured with the culture normalizing agent according to the present disclosure is those derived from a primate. In a preferred embodiment, the corneal endothelial cell cultured with the culture normalizing agent according to the present disclosure is derived from humans.

In a preferred embodiment, the culturing as the objective of the present invention is cell culturing for the prevention or treatment of corneal endothelial damage.

### (Culture Medium for Normally Culturing Corneal Endothelial Cells)

The present disclosure provides a culture medium for normally culturing a corneal endothelial cell, including a culturing ingredient for corneal endothelium and a culture normalizing agent. It is understood that any form described herein can be used for the culture normalizing agent used in the culture medium. In addition, any ingredient can be used as the culturing ingredient that can be used in the present invention so long as the culturing ingredient can be used for the culturing of corneal endothelium. Such an ingredient may be a culture medium ingredient that has been conventionally sold and used. Alternatively, such an ingredient may be an ingredient separately developed for corneal endothelium. Examples of such a culture medium ingredient include, without limitation, OptiMEM, DMEM, M199, and MEM (which are available from INVITROGEN or the like).

### (Method for Normally Culturing Corneal Endothelial Cells)

In another aspect, the present invention provides a method for normally culturing a corneal endothelial cell, comprising the step of culturing a corneal endothelial cell using a culture medium according to the present disclosure.
It is understood that any form described herein can be used for the culture normalizing agent used in the method according to the present invention. In addition, any ingredient can be used as the culturing ingredient that can be used in the method according to the present invention, so long as the ingredient can be used for the culturing of corneal endothelium; and those described in section (Culture Medium for Normally Culturing Corneal Endothelial Cells) can be exemplified.

One exemplary culturing method is shown in Figure **12****.** For example, in one exemplary culturing method, a plurality of agents can be separately used as the culture normalizing agent according to the present invention. For example, the fibrosis inhibitor can be allowed to be present at all times for a certain period of time (e.g., 24 to 72 hours, or 48 hours, or the like) during the culturing of said corneal endothelial cell, while the adhesion promoting agent can be allowed to be present and then the adhesion promoting agent can be deleted, and the cell adhesion promoting agent can be present at all times for a certain period of time (e.g., 24 to 72 hours, or 48 hours, or the like; the period of time may vary each time, or may be the same) . Alternatively, there is a pattern where no adhesion promoting agent is used in these culturing methods. Specifically, this exemplary culturing method is shown in the lower part of Figure **12****.** For example, in this exemplary culturing method, the fibrosis inhibitor can be allowed to be present at all times during the culturing of said corneal endothelial cell, as the culture normalizing agent according to the present invention.

In another embodiment, in the method according to the present invention, the culture normalizing agent to be used includes both a fibrosis inhibitor and said cell adhesion promoting agent, and they can be allowed to be present at all times during the culturing of said corneal endothelial cell.

In one embodiment, the corneal endothelial cell cultured with the culture normalizing agent according to the present disclosure is derived from a primate. In a preferred embodiment, the corneal endothelial cell cultured with the culture normalizing agent according to the present disclosure is derived from humans.

In a preferred embodiment, the culturing as the objective of the method according to the present invention is cell culturing for prevention or treatment of corneal endothelial damage, which can be used, in particular, to produce a cell, tissue or the like for transplantation.

### (Corneal Endothelial Cell and Corneal Endothelial Formulation)

The present disclosure provides a corneal endothelial cell cultured by a method according to the present invention. Such a cell can be considered as having a characteristic that conventional cells do not have in that the cell according to the present invention does not experience fibrosis and does not lose normal function even if normal culturing is performed and subculturing is also performed. In addition, the most important characteristic is that the cell has a characteristic of normal corneal endothelium as a function. Accordingly, the corneal endothelial cell of the present disclosure can be provided as a formulation, which means that the present disclosure provides a corneal endothelial formulation.

Thus, the present invention provides a method for manufacturing a corneal endothelial formulation, comprising the step of culturing a corneal endothelial cell using a culture solution including a culture normalizing agent according to the present disclosure.

In one aspect, the corneal endothelial formulation according to the present disclosure contains a substrate, and a corneal endothelial cell layer cultured *in vitro* on the substrate.

The substrate used in the present disclosure is not particularly limited so long as the substrate can support a cultured corneal endothelial cell layer and maintain its form *in vivo* for a certain period of time, preferably for at least 3 days, after transplantation. In addition, the substrate may have a role as a scaffold when a corneal endothelial cell is cultured *in vitro,* or the substrate may have only a role for supporting a corneal endothelial cell layer after culturing. Preferably, the substrate is such a substrate that is used for the culturing of a corneal endothelial cell and that has a role as a scaffold that can be subjected to transplantation as-is, after the completion of culturing.

Usable substrates include, for example, collagen, gelatin, cellulose and other natural product-derived polymer materials, polystyrene, polyester, polycarbonate, poly(N-isopropyl acrylamide) and other synthetic polymer materials, polylactic acid, polyglycolic acid and other biodegradable polymer materials, hydroxyapatite, amnion and the like.

The shape of the substrate is not particularly limited so long as it has a shape that supports the corneal endothelial cell layer and is suitable for transplantation, but the shape is preferably a sheet. When the formulation according to the present disclosure is a sheet, it can be cut and used in a size conforming to an application site at the time of transplantation. In addition, it is also possible to roll up the sheet and insert the sheet through a wound opening. As a preferred specific example, exemplified is a circular shape covering about 80% of the area of damaged corneal endothelium. It is also preferable to make cuts in the periphery portion of the circle so that the circle will be in close contact with the applied site.

The substrate is preferably collagen. As for the collagen, the collagen sheet described in Japanese Laid-Open Publication No. 2004-24852 can be preferably used. The subject collagen sheet can be prepared from, for example, amnion in accordance with the method described in Japanese Laid-Open Publication No. 2004-24852.

Hereinafter, preparation of a corneal endothelial cell layer will be described as an example of a corneal endothelial formulation.

The corneal endothelial cell layer of the disclosure preferably comprises at least one of the following characteristics. More preferably, the corneal endothelial cell layer comprises two or more of the following characteristics, and still more preferably comprise all of the following characteristics.
(1) The cell layer has a single layer structure. This is one of the characteristics that a corneal endothelial cell layer of a living organism comprises.
(2) The cell density of the cell layer is about 1,000 to about 4,000 cells/mm². In particular, when an adult is a recipient (transplant recipient), the cell density is preferably in the range of about 2,000 to about 3,000 cells/mm².
(3) The shape of the cells constituting the cell layer in planar view is substantially hexagon. This is one of the characteristics that a cell constituting a corneal endothelial cell layer in a living organism comprises. The formulation according to the present disclosure is similar to a corneal endothelial cell layer of living organisms, is capable of exerting a function similar to that of an innate corneal endothelial cell layer, and is capable of exerting growth capacity in living organisms.
(4) Cells are arranged with regularity in the cell layer. In a corneal endothelial cell layer of a living organism, cells constituting the layer are arranged with regularity, which is considered as maintaining normal functions and high degree of transparency of the corneal endothelial cell, and as appropriately adjusting the moisture in the cornea. Thus, by comprising such a morphological characteristic, the formulation according to the present disclosure is expected to exert a function similar to that of the corneal endothelial cell layer in living organisms.

The manufacturing method according to the present invention comprises the step of culturing a corneal endothelial cell using a culture normalizing agent or a culture medium according to the present disclosure.

### <1> Collection and Culturing In Vitro of Corneal Endothelial Cells

Corneal endothelial cells are collected either from the cornea of the recipient themselves or an appropriate donor using an ordinary method. In consideration of the transplant conditions it is sufficient to prepare homologously-derived corneal endothelial cells. For example, the Descemet's membrane and endothelial cell layer of corneal tissues are exfoliated from the parenchyma of the cornea, and then they are transferred to a culture plate and treated with dispase or the like. As a result, the corneal endothelial cells are detached from the Descemet's membrane. Corneal endothelial cells remaining in the Descemet's membrane can be detached by pipetting or the like. After the Descemet's membrane is removed, the corneal endothelial cell are cultured in a culture solution according to the present disclosure. As to the culture medium or culture solution, it is possible to use, for example, a commercially available DMEM (Dulbecco's Modified Eagle's Medium) (e.g., INVITROGEN, catalog number: 12320 or the like) with FBS (fetal bovine serum) (e.g., BIOWEST, catalog number: S1820-500), b-FGF (basic fibroblast growth factor) (e.g., INVITROGEN, catalog number: 13256-029), and penicillin, streptomycin or other antibiotic substances added thereto as appropriate, and an ingredient of the culture normalizing agent according to the present disclosure further added thereto. As to a culture vessel (culture plate), it is preferable to use those with type I collagen, type IV collagen, fibronectin, laminin or an extracellular matrix of bovine corneal endothelial cells coated on the surface thereof . Alternatively, it is also possible to use an ordinary culture vessel treated with FNC coating mix (registered trademark) (50ml (AES-0407), ATHENA, catalog number: 0407) or other commercially available coating agent. This is because, by co-using the subject coating and the culture solution according to the present disclosure, the adhesion of the corneal endothelial cells to the surface of the culture vessel is promoted, and favorable growth is performed.

Temperature conditions in culturing corneal endothelial cells are not particularly limited so long as corneal endothelial cells grow, but they are, for example, in the range of about 25°C to about 45°C, and in consideration of growth efficiency, preferably about 30°C to about 40 °C, still preferably about 37°C. As to a method of culturing, the culturing is performed in a normal incubator for culturing cells, under a humidified environment, and under the environment of about 5 to 10% CO₂ concentration.

### <2> Subculturing

After corneal endothelial cells subjected to culturing are grown, subculturing can be performed. Preferably, subculturing is performed at the time of being sub-confluent or confluent. The subculturing can be performed as follows. First, by treating with trypsin-EDTA or the like, cells are exfoliated from the surface of a culture vessel, and then the cells are collected. A culture normalizing agent or culture medium according to the present invention is added to the collected cells to form cell suspended liquid. It is preferable to perform centrifugation when the cells are collected or after the collection. Centrifugation allows for preparation of a cell-suspending liquid with high cell density. Preferable cell density is in a range of about 1 to 2×10⁶ cell/mL. Note that the conditions for the centrifugation herein include, for example, 500 rpm (30g) to 1,000 rpm (70g), and 1 to 10 minutes.

The cell suspended liquid is disseminated to a culture vessel similar to the above-mentioned initial culturing, followed by being subjected to culturing. The dilution ratio at the time of subculturing is about 1:2 to 1:4, and preferably about 1:3 although it varies in accordance with the condition of the cells. The subculturing can be performed under culturing conditions similar to the above-mentioned initial culturing. The culturing period varies in accordance with the condition of the cells to be used, and it is, for example, 7 to 30 days. The above-mentioned subculturing can be performed multiple times as required. In the culture normalizing agent or culture medium according to the present disclosure, if a cell adhesion promoting agent is used, the cell adhesion in the initial stage of the culturing can be enhanced, making it possible to shorten the culturing period.

### <3> Preparation of Corneal Endothelial Cell Layers

Liquid cell suspension is disseminated on a substrate such as a collagen sheet, and is subjected to culturing. At this stage, the number of cells to be disseminated is adjusted so as to form a cell layer with desired cell density in corneal endothelial formulation that is manufactured in the end. Specifically, the cells are disseminated so that a cell layer will be formed, cell density of which will be about 1,000 to about 4,000 cells/mm². The culturing can be performed under conditions similar to those of the above-mentioned initial culturing or the like. The culturing period is, for example, 3 to 30 days although it varies based on the condition of the cells to be used.

By performing the culturing as described above, a corneal endothelial formulation is obtained in which the corneal endothelial cell layer cultured *in vitro* is formed on the substrate.

In order to maintain the corneal endothelial cell, the corneal endothelial formulation may include the culture normalizing agent according to the present disclosure or a culture medium which includes the culture normalizing agent. In addition, the corneal endothelial formulation may include the culture normalizing agent according to the present invention or a culture medium which includes the culture normalizing agent until the corneal endothelial formulation is subjected to transplantation. It is also provided a combination of the corneal endothelial formulation and the culture normalizing agent according to the present disclosure or a culture medium which includes the culture normalizing agent.

The corneal endothelial formulation obtained by manufacturing method the according to the present invention can be used as a graft in the treatment of diseases which require transplantation of corneal endothelium, such as bullous keratopathy, corneal edema, corneal leukoma, in particular, corneal dystrophy, or bullous keratopathy caused by corneal endothelial damage due to external injury or intraocular operation. The cause of bullous keratopathy or corneal endothelial damage includes Fuchs' corneal endothelial dystrophy, pseudoexfoliation syndrome, corneal endotheliitis and the like, in addition to operations.

The subject of the administration of the corneal endothelial formulation according to the present disclosure includes mammals (e.g., humans, mice, rats, hamsters, rabbits, cats, dogs, cows, sheep, monkeys and the like), and preferably primates (e.g., humans).

### (Treatment or Prevention of Corneal Endothelial Disease, Damage or Condition)

The present disclosure provides a medicament for treating or preventing a corneal endothelial disease, damage or condition, including a corneal endothelial cell produced by a method for normally culturing a corneal endothelial cell, the method comprising the step of culturing a corneal endothelial cell using a culture normalizing agent or a culture medium according to the present disclosure. It is understood that the culture medium or culture normalizing agent can take any form, as described in the present specification. For example, it is possible to refer to the matters described in sections (Culture Normalizing Agent), (Culture Medium for Normally Culturing Corneal Endothelial Cells), and (Method for Normally Culturing Corneal Endothelial Cells) . In addition, it is understood that the corneal endothelial cell used as a medicament can take any form used in the present specification, and for example, it is possible to refer to the matters described in section (Corneal Endothelial Cell and Corneal Endothelial Formulation).

In one embodiment, the medicament according to the present disclosure is for the purpose of treating or preventing corneal endothelium of a primate. Preferably, the subject of the treatment or prevention is human corneal endothelium.

In one embodiment, the corneal endothelial cell used in the medicament according to the present disclosure is derived from a primate. Preferably, the corneal endothelial cell used in the medicament is derived from humans.

In one embodiment, the corneal endothelial disease, damage or condition as the target of the medicament according to the present disclosure
is bullous keratopathy, corneal endotheliitis, corneal edema, corneal leukoma and the like.

In one embodiment, the medicament according to the present disclosure is provided in a form of a sheet or a suspended substance.

In one embodiment, the medicament according to the present disclosure further comprises a cell adhesion promoting agent. The cell adhesion promoting agent exerts an adhesion promoting action to a corneal endothelial cell separated from corneal tissues or separated and subcultured corneal endothelial cell therefrom. The cell adhesion promoting agent can be provided together with, or separated from, the corneal endothelial cell provided as a medicament. In a specific embodiment, the cell adhesion promoting agent used in the medicament includes a Rho kinase inhibitor . As to the Rho kinase inhibitor, included are compounds disclosed in the following documents : US Patent No. 4, 678, 783, Japanese Patent No. 3,421,217, International Publication No. WO 95/28387, International Publication No. WO 99/20620, International Publication No. WO 99/61403, International Publication No. WO 02/076976, International Publication No. WO 02/076977, International Publication No. WO 2002/083175, International Publication No. WO 02/100833, International Publication No. WO 03/059913, International Publication No. WO 03/062227, International Publication No. WO 2004/009555, International Publication No. WO 2004/022541, International Publication No. WO 2004/108724, International Publication No. WO 2005/003101, International Publication No. WO 2005/039564, International Publication No. WO 2005/034866, International Publication No. WO 2005/037197, International Publication No. WO 2005/037198, International Publication No. WO 2005/035501, International Publication No. WO 2005/035503, International Publication No. WO 2005/035506, International Publication No. WO 2005/080394, International Publication No. WO 2005/103050, International Publication No. WO 2006/057270, International Publication No. WO 2007/026664, and the like. The subject compounds can be manufactured by the methods described in the documents in which the respective compounds are disclosed. For example, included are 1-(5-isoquinolinesulfonyl)homopiperazine or a salt thereof (e.g., fasudil(1-(5-isoquinolinesulfonyl)homopiperazine)), (+)-trans-4-(1-aminoethyl)-1-(4-pyridylcarbamoyl)cycloh exanecarboxamide or a salt thereof (e.g., Y-27632((R)-(+)-trans-(4-pyridyl)-4-(1-aminoethyl)-cycl ohexanecarboxamide 2 hydrochloride 1 hydrate) and the like) and the like.

The subject of the administration (transplantation) of the medicament or method according to the present disclosure includes mammals (e.g., humans, mice, rats, hamsters, rabbits, cats, dogs, cows, horses, sheep, monkeys and the like), and the subject is preferably primates, and particularly preferably humans. Corneal endothelium treatment with primates had not achieved sufficient results before, and from that point of view, the present disclosure provides an innovative treatment method and medicament.

It is provided a method for treating or preventing a corneal endothelial disease, damage or condition, comprising the step of using a corneal endothelial cell produced by a method for normally culturing a corneal endothelial cell, comprising the step of culturing a corneal endothelial cell using a culture normalizing agent or a culture medium according to the present disclosure.

It is provided a medicament for treating or preventing a corneal endothelial disease, damage or condition of a human, comprising a cell adhesion promoting agent. The adhesion promoting action of the cell adhesion promoting agent is used for a corneal endothelial cell separated from corneal tissues or a corneal endothelial cell separated and subcultured therefrom. In a specific embodiment of this aspect, the cell adhesion promoting agent used in the medicament according to the present disclosure includes a Rho kinase inhibitor. The Rho kinase inhibitor includes compounds disclosed in the following documents: US Patent No. 4,678,783, Japanese Patent No. 3,421,217, International Publication No. WO 95/28387, International Publication No. WO 99/20620, International Publication No. WO 99/61403, International Publication No. WO 02/076976, International Publication No. WO 02/076977, International Publication No. WO 2002/083175, International Publication No. WO 02/100833, International Publication No. WO 03/059913, International Publication No. WO 03/062227, International Publication No. WO 2004/009555, International Publication No. WO 2004/022541, International Publication No. WO 2004/108724, International Publication No. WO 2005/003101, International Publication No. WO 2005/039564, International Publication No. WO 2005/034866, International Publication No. WO 2005/037197, International Publication No. WO 2005/037198, International Publication No. WO 2005/035501, International Publication No. WO 2005/035503, International Publication No. WO 2005/035506, International Publication No. WO 2005/080394, International Publication No. WO 2005/103050, International Publication No. WO 2006/057270, International Publication No. WO 2007/026664 amino acid. The subject compounds can be manufactured by the methods described in the documents in which the respective compounds are disclosed, For example, included are 1-(5-isoquinolinesulfonyl)homopiperazine or a salt thereof (e.g., fasudil(1-(5-isoquinolinesulfonyl)homopiperazine)), (+)-trans-4-(1-aminoethyl)-1-(4-pyridylcarbamoyl)cycloh exanecarboxamide or a salt thereof (e.g., Y-27632((R)-(+)-trans-(4-pyridyl)-4-(1-aminoethyl)-cycl ohexanecarboxamide 2 hydrochloride 1hydrate)and the like) and the like. In particular, the present disclosure has achieved a favorable treatment performance for the first time using a cell adhesion promoting agent in cases with primate models and human cells.

The medicament comprising the cell adhesion promoting agent according to the present disclosure is used together with a corneal endothelial cell produced by a method for normally culturing a corneal endothelial cell, the method comprising the step of culturing a corneal endothelial cell using a culture normalizing agent or a culture medium according to the present disclosure. In this regard, the medicament including the cell adhesion promoting agent according to the present disclosure may be administered or transplanted together with the corneal endothelial cell, or may be administered or transplanted separately.

In one specific embodiment, the corneal endothelial disease, damage or condition targeted by the medicament including the cell adhesion promoting agent according to the present disclosure includes bullous keratopathy, corneal endotheliitis, corneal edema, corneal leukoma and the like.

It is also disclosed a method for treating or preventing a corneal endothelial disease, damage or condition of a human, the method comprising the step of administering a cell adhesion promoting agent to a subject who is in need of treatment or prevention.

With regard to the medicament including a cell adhesion promoting agent as well, the target for the administration (transplantation) of the medicament or method according to the present disclosure includes mammals (e.g., humans, mice, rats, hamsters, rabbits, cats, dogs, cows, horses, sheep, monkeys and the like), and the subject is preferably primates, and particularly preferably humans. Corneal endothelium treatment with primates had not achieved sufficient results before, and from that point of view, the present disclosure provides an innovative treatment method and medicament.

The medicament for treating or preventing a corneal endothelial disease, damage or condition including a corneal endothelial cell produced using the method according to the present invention, which includes cell adhesion promoting agent or Rho kinase inhibitor is used at the concentration, without limitation, for example, normally about 0.00001 to 1 w/v%, preferably, about 0.00001 to 0.1 w/v%, more preferably about 0.0001 to 0.05 w/v%, about 0.001 to 0.05 w/v%, about 0.002 to 0.05 w/v%, about 0.003 to 0.05 w/v%, about 0.004 to 0.05 w/v%, about 0.005 to 0.05 w/v%, about 0.006 to 0.05 w/v%, about 0.007 to 0.05 w/v%, about 0.008 to 0.05 w/v%, about 0.009 to 0.05 w/v%, about 0.01 to 0.05 w/v%, about 0.02 to 0.05 w/v%, about 0.03 to 0.05 w/v%, about 0.04 to 0.05 w/v%, about 0.003 to 0.04 w/v%, about 0.004 to 0.04 w/v%, about 0.005 to 0.04 w/v%, about 0.006 to 0.04 w/v%, about 0.007 to 0.04 w/v%, about 0.008 to 0.04 w/v%, about 0.009 to 0.04 w/v%, about 0.01 to 0.04 w/v%, about 0.02 to 0.04 w/v%, about 0.03 to 0.04 w/v%, about 0.003 to 0.03 w/v%, about 0.004 to 0.03 w/v%, about 0005 to 0.03 w/v%, about 0.006 to 0.03 w/v%, about 0.007 to 0.03 w/v%, about 0.008 to 0.03 w/v%, about 0.009 to 0.03 w/v%, about 001 to 0.03 w/v%, about 0.02 to 0.03 w/v%, about 0.003 to 0.02 w/v%, about 0.004 to 0.02 w/v%, about 0.005 to 0.02 w/v%, about 0.006 to 0.02 w/v%, about 0.007 to 0.02 w/v%, about 0.008 to 0.02 w/v%, about 0.009 to 0.02 w/v%, about 0.01 to 0.02 w/v%, about 0.003 to 0.01 w/v%, about 0.004 to 0.01 w/v%, about 0.005 to 0.01 w/v%, about 0.006 to 0.01 w/v%, about 0.007 to 0.01 w/v%, about 0.008 to 0.01 w/v%, and about 0.009 to 0.01 w/v%. The dosage amount and the frequency of administration vary in accordance with symptoms, ages, weights or administration forms. In case of the use as an eye lotion, for example, for normal adults, the formulation, containing an effective ingredient of about 0.0001 to 0.1 w/v%, and preferably about 0.003 to 0.03 w/v%, can be administered 1 to 10 times per day, preferably 1 to 6 times per day, and more preferably 1 to 3 times per day, and at the amount in the range of about 0.01 to 0.1 mL per time. When the medicament according to the present disclosure is introduced into an anterior chamber, the medicament at a concentration one-tenth to one-thousandth of the above-mentioned concentration can be used. Those skilled in the art can appropriately select the type and concentration of the cell adhesion promoting agent in accordance with disease states.

The present invention has been described in the above by showing preferred embodiments thereof for the sake of easy understanding. Hereinafter, the present invention is described based on examples, but the above-mentioned descriptions and the following examples are provided merely for the purpose of exemplifications and not provided for the purpose of limiting the invention. Accordingly, the scope of the present invention is not limited to the embodiments or examples which are specifically described in the present specification, but is limited by the claims alone.

### [Examples]

Hereinafter, examples of normally culturing a cell of a corneal endothelial cell according to the present invention will be described. The experimental animal was used according to the International Guiding Principles for Biomedical Research Involving Animals, as well as law relating to protection and management of animals, and standards relating to feeding and housing and the like of experimental animals . This experiment was performed according to Guidelines of the Association for Research in Vision and Ophthalmology on the Use of Animals in Ophthalmic and Vision Research. Isolation of tissues was approved by the animal experiment ethical committee of Shiga Laboratory, Nissei Bilis Co., Ltd. (Ohtsu city, Shiga, Japan) and the animal experiment committee of Eve Bioscience, Co., Ltd. (Hashimoto city, Wakayama, Japan). Further, in applicable, standards stipulated by Ministry of Health, Labour and Welfare, Ministry of Education, Culture, Sports, Science and Technology, or the like were observed for the handling of biological samples or the like; and if applicable, the handling was performed based on Helsinki Declaration or ethical codes prepared based on the Declaration. For the donation of eyes used for the research, agreements were obtained from close relatives of all the deceased donors. The present research was approved by the institutional review board of SightLife™ (Seattle, WA) eyebank.

### (Experimental Method: Corneal Tissues of Monkeys and Research Grade, Human Corneal Tissues)

Eight corneas from four cynomolgus monkeys (ages of 3 to 5 years old; assumed to be equivalent to 5 to 20 years old in humans), respectively fed by Shiga Laboratory, Nissei Bilis Co., Ltd. and Eve Bioscience, Co., Ltd., were used to culture monkey corneal endothelial cells (MCEC). Twelve corneas from human donors were obtained from SightLife™ eyebank, and all the corneas were preserved in a preservation culture medium (Optisol; Chiron Vision Corporation, Irvine, CA) at the temperature of 4°C during the period of less than 14 days prior to the primary culture.

### (Statistics Analysis)

The statistically-significant difference (P value) in the average value in comparison of two samples was determined by using t-test of students. The statistically-significant difference in the comparison of a plurality of sample sets was analyzed by Dunnett's multiple comparison test. The values shown in the graph represent an average ±SE.

### (Comparative Example 1)

In the present example, states of corneal endothelial cells of cynomolgus monkeys and humans, which were cultured using a conventional method, will be described. The details will be described hereinafter.

### (Material and Method)

*Cynomolgus monkey corneal endothelial cells (MCEC; source of supply and culturing method): the MCECs were cultured by the improved-type protocol described previously [Koizumi N, et al. (2007) Invest Ophthalmol Vis Sci 48: 4519-4526], [Li W, et al. (2007) Invest Ophthalmol Vis Sci 48: 614-620] . Briefly speaking, eyeballs of cynomolgus monkeys, which were humanely killed for another purpose, were purchased (Nissei Bilis Co., Ltd., Ohtsu, Japan and Keari Co., Ltd., Wakayama, Japan) (the method is described above). The Descemet's membrane including corneal endothelial cells were exfoliated, and the corneal endothelial cells were mechanically exfoliated together with basement membranes, followed by treatment using dispase or collagenase (ROCHE catalog number : 10 103 586 001) and then primary culture. Typically, treatment was performed using 1 mg/mL collagenase A (Roche Applied Science, Penzberg, Germany) at 37°C for 2 hours. For the culture medium, DMEM (INVITROGEN catalog number 12320) was used with 10% FBS (BIOWEST, catalog number: S1820-500) and 2 ng/ml basic FGF (INVITROGEN, catalog number: 13256-029) added thereto. For culturing, 6 well plates and the like were used, which were coated with FNC Coating MIX (registered trademark) (Athena Environmental Sciences., Baltimore, MD) . Next, the MCECs were cultured in 5% CO₂ at 37°C in a humidified atmosphere, and the culture media were replaced every three days. When the MCECs reached confluency in 10 to 14 days, they were rinsed with Ca²⁺ and Mg²⁺-free Dulbecco's Phosphate Buffered Saline (PBS), tripsinized at 37°C for 5 minutes with 0.05% trypsin-EDTA (Life Technologies), and subcultured at the ratio of 1:2 to 4. A selective inhibitor, of the transforming growth factor -β (TGF-β), SB431542 (Merck Millipore, Billerica, MA) was examined with regard to its anti-fibroblastic action.

* Human corneal endothelial cells (HCEC; source of supply and culturing method) : the HCECs were cultured by an improved version of the protocol used for the MCECs. Briefly, Descemet's membranes, including corneal endothelial cells, were exfoliated from research corneas purchased from the Seattle bank, the corneal endothelial cells were mechanically exfoliated together with basement membranes, and the corneal endothelial cells were removed from the basement membranes using collagenase (ROCHE catalog number: 10 103 586 001) (typically, treated at 37°C for 2 hours with 1 mg/mL collagenase A (Roche Applied Science)) . After the collection, primary culture was performed. As for the culture medium for the human samples, Opti-MEM I Reduced-Serum Medium, Liquid (INVITROGEN catalog number:31985-070) + 8% fetal bovine serum (FBS) (BIOWEST, catalog number: S1820-500) + 200mg/ml CaCl_{2·}2H₂O (SIGMA catalog number: C7902-500G) + 0.08% chondroitin sulfuric acid (SIGMA catalog number: C9819-5G) + 20 µg/ml ascorbic acid (SIGMA catalog number: A4544-25G) +50 µg/ml gentamicin (INVITROGEN catalog number: 15710-064) + 5 ng/ml EGF (INVITROGEN catalog number: PHG0311), conditioned for 3T3 feeder cells, was used. Specifically, after digestion at 37°C, the HCECs obtained from individual corneas were re-suspended in a culture medium, followed by plating on one well of a 12 well plate coated with FNC Coating Mix (registered trademark) . The culture medium was prepared in accordance with a publicly known protocol with a partial modification added thereto. Briefly, a base culture medium was prepared, containing OptiMEM-I (Life Technologies), 8% FBS, 5 ng/mL epithelial growth factor (EGF) (Sigma-Aldrich Co., St. Louis, MO), 20 µg/mL ascorbic acid (Sigma-Aldrich), 200 mg/L calcium chloride (Sigma-Aldrich), 0.08% chondroitin sulfuric acid (Wako Pure Chemical Industries, Ltd, Osaka city) and 50 µg/mL gentamicin. Next, after the culturing of inactivated 3T3 fibroblast, the conditioned culture medium was collected. The inactivation of the 3T3 fibroblast was performed as previously described. Briefly, confluent 3T3 fibroblast, together with 4 µg/mL mitomycin C(MMC) (Kyowa Hakko Kirin Co., Ltd, Tokyo), was incubated under 5% CO₂ at 37°C for 2 hours, followed by trypsinization and plating on a plastic plate in the density of 2 × 10⁴ cells/cm². The HCECs were cultured in 5% CO₂, at 37°C in a humidified atmosphere, and the culture media were replaced every three days. When the HCECs reached confluency in 10 to 14 days, they were rinsed in Ca²⁺ and Mg²⁺-free PBS, tripsinized at 37°C for 5 minutes with 0.05% trypsin-EDTA, and subcultured at the ratio of 1:2. SB431542 (Merck Millipore), neutralization antibody directed to TGF-β (R&DSystems, Inc., Minneapolis, MN), Smad3 inhibitor (Merck Millipore) and osteogenic protein (BMP) BMP-7 (R&D Systems) were examined with regard to anti-fibroblastic actions.

* cell were observed using methods such as staining (Histology)using a phase-contrast microscope. In addition, after the cells were fixed, immunostaining was performed using ZO-1, Na⁺/K⁺-ATPase as a function-associated marker, followed by observation through a fluorescence microscope. For tissue staining examination, cultured MCECs or HCECs were put into Lab-Tek™ Chamber Slides™ (NUNC A/S, Roskilde, Denmark), fixed with 4% formaldehyde for 10 minutes at a room temperature (RT), and incubated for 30 minutes with

1% bovine serum albumin (BSA) . Specifically, cultured MCECs or HCECs on the Lab-Tek™ Chamber Slides™ (NUNC A/S, Roskilde, Denmark) were fixed at a room temperature for 10 minutes in 4% formaldehyde, followed by incubation for 30 minutes together with 1% bovine serum albumin (BSA). In order to examine the phenotype of CEC, immunohistochemical analysis was directed to a protein associated with tight junction, ZO-1 (Zymed Laboratories, Inc., South San Francisco, CA), a protein associated with the pumping function, Na⁺/K⁺-ATPase (Upstate Biotec, Inc., Lake Placid, NY), fibronectin (BD, Franklin Lakes, NJ), and actine. As markers associated with the function of CEC, ZO-1 and Na⁺/K⁺-ATPase were used; fibronectin and type 1 collagen were used to evaluate the change in a fibroblastic manner; and staining of actine was used to evaluate the phenotype of the cells. Staining of ZO-1, Na⁺/K⁺-ATPase, type 1 collagen and fibronectin was each performed using 1:200 dilution of ZO-1 polyclonal antibody, Na⁺/K⁺-ATPase monoclonal antibody, and fibronectin monoclonal antibody. As to secondary antibody, 1:2000 dilution of Alexa Fluor (registered trademark) 488 label or Alexa Fluor (registered trademark) 594 label goat antimouse IgG (Life Technologies) was used. Staining of actine was performed using 1:400 dilution of Alexa Fluor (registered trademark) 488 label phalloidin (Life Technologies). Next, the nucleus of each cell was stained with DAPI (Vector Laboratories, Inc., Burlingame, CA) or PI (Sigma-Aldrich). Next, the slides were observed through a fluorescence microscope (TCS SP2 AOBS; Leica Microsystems, Welzlar, Germany).

### (Result)

Figure **1** shows a result of culturing in cynomolgus monkeys and humans using a conventional cell culturing method. As clear from the culturing result, it is understood that transformation occurred in the corneal endothelium of the monkeys and humans with the normal culturing method, that is, fibrosis occurred in the respective cells, resulting in a different phenotype from that of the normal cells, namely the cells are of a polygonal shape and a single layer, which is not suitable for transplantation.

### (Comparative Example 2)

In the present example, an experiment was conducted to show that normal functions would be lost in culturing based on prior art. In the present example, it was demonstrated as to whether or not monkey corneal endothelium would lose the expression of function-associated protein using immunostaining and a Western blot technique as well as a real-time PCR method. The details will be provided hereinafter.

### (Material and Method)

Among the materials used hereinafter, the same materials as those used in the Comparative Example 1 were obtained and were subjected to culturing and the like in a similar manner to Comparative Example 1.
*Cynomolgus monkey corneal endothelial cells: the same as Comparative Example 1.
*Human corneal endothelial cells: the same as Comparative Example 1.
* Antibodies to Na⁺/K⁺-ATPase: those available fromMILLIPORE (MILLIPORE catalog number: 05-369) were used.
* Antibodies to ZO-1: mice, those available from INVITROGEN (INVITROGEN catalog number: 339100), and rabbit, those available from ZYMED LABORATORIES (ZYMED LABORATORIES catalog number: 61-7300), were used.
*Antibodies to fibronectin: available from BD BIOSCIENCES (catalog number: 610077)
*Antibodies to collagen type 1: (available from ABEAM) (catalog number: ab292)
*Antibodies to GAPDH: those available from ABEAM (catalog number: ab36840) were used.
*Secondary antibody (HER binding anti-rabbit IgG secondary antibody) available from Cell Signaling Technology (catalog number: 7074)
*Secondary antibody (anti-rabbit IgG secondary antibody) available from Cell Signaling Technology (catalog number: 7076)
*Cell fraction extract or preparation method: cells which had reached confluency were washed three times with PBS (Dulbecco's PBS, Nissui, catalog number: 5913), followed by dissolution with RIPA buffer (1×PBS, 1% Nonidet P-40 (Nacalai Tesque, catalog number: 23640-94), 0.5% sodium deoxycholate (NacalaiTesque, catalog number: 10712-12), and 0.1% SDS (sodium lauryl sulfate, Nacalai Tesque, catalog number:31607-65)). Phosphatase inhibitor cocktail 2 (Sigma-Aldrich) and protease inhibitor cocktail (Nacalai Tesque, Inc., Kyoto city) were added to the aforementioned RIPAbuffer. The obtained cell lysate was centrifuged (15,000 rpm, 10 minutes) where the supernatant thereof was collected, and the protein was quantitated using a BCA PROTEIN ASSAY KIT (PIERCE (catalog number: 23227)). Dissolution was performed using 100µl dissolution buffer (Laemmli sample buffer) including 5 mM 2-mercaptoethanol (Nacalai Tesque (catalog number: 21418-42)).
*Immunostaining: after washing cells which had reached confluency with PBS (Nissui, catalog number: 5913), the cells were fixed for 10 minutes with iced ethanol (Nacalai Tesque, catalog number: 14713-95) and acetic acid (WAKO catalog number:017-00256) (95:5) .

Blocking was performed via incubation for 1 hour with 0.1% (vol/vol) polyethylene sorbitan monolaurate (Nacalai Tesque, catalog number: 28353-85) (TBS-T) and Tris buffered saline (10mMTris-HCl, pH7.4;100mMNaCl) with 10% fetalbovine serum. Rabbit anti-human ZO-1 antibody (1:200), and murine anti-human Na⁺/K⁺-ATPase antibody (1:200) were used as primary antibodies to allow for reaction for 1 hour at a room temperature . The same was used with regard to antibodies to fibronectin, and antibodies to collagen type 1. As to the dilution rate, 1:200 or 1:1000 was used as appropriate.

Next, reaction was allowed for 1 hour at a room temperature with ALEXA FLUOR 488 (INVITROGEN (catalog number: A21206)) and ALEXA FLUOR 594 (INVITROGEN (catalog number: A21203)), diluted with TBS-T by 1,000 fold. After washing with PBS, the sample was encapsulated with VECTASHIELD WITH DAPI (VECTOR LABORATORIES (catalog number: 94010)) onto a slide, followed by observation through confocal microscope (Leica) . *Western blot technique: protein extracted by RIPA buffer was electrophoresed with 7.5% polyacrylamide. The separated protein was transferred to a PVDF film (PALL LIFE SCIENCE (catalog number: EH-2222)). The blotted film was incubated for an hour with Tris buffered saline (10m MTris-HCl, pH 7.4; 100mM NaCl) (TBS-T) including 0.1% (vol/vol) polyethylene sorbitan monolaurate (Nacalai Tesque, catalog number: 28353-85) supplemented with 5% fat-free dried milk (5% NON FAT DRY MILK, CELL SIGNALING, catalog number: 9999) for blocking purposes. Thereafter, the ZO-1 antibody and Na⁺/K⁺-ATPase antibody were diluted by 1, 000 fold with TBS-T, supplemented with5% non-fat dried milk. The films were immersed into a membrane for an hour at room temperature to allow the reaction to take place. After washing 3 times with TBS-T, incubation was performed with a murine-IgG antibody HRP complex (CELL SIGNALING (catalog number: 7074P2)). After washing, bands were detected using an ECL-ADVAVCE Western Blotting Detection Kit (GE healthcare Japan (catalog number: RPN2135V)) . Antibodies to fibronectin, and antibodies to collagen type 1 were also used in a similar manner. Next, incubation was performed with the following primary antibodies: Na⁺/K⁺-ATPase (Merck Millipore), ZO-1, GAPDH (Abcam, Cambridge, UK), fibronectin and Smad2 (Cell Signaling Technology), phosphorylatedSmad2 (Cell Signaling Technology), ERK1/2 (BD), phosphorylation ERK1/2 (BD), p38MAPK (BD), phosphorylated p38MAPK (BD), JNK (BD) or phosphorylated JNK (BD) (1:1000 dilution), and HRP label anti-rabbit or anti-rabbit IgG secondary antibody (Cell Signaling Technology) (1:5000 dilution). The membrane was exposed using an ECL Advance Western Blotting Detection Kit (GE Healthcare, Piscataway, NJ) and then examined using a LAS4000S imaging system (FUJIFILM Corporation, Tokyo). *Real-time PCR (semiquantitative reverse transcriptase polymerase chain reaction (RT-PCR)): in addition, a PCR method was performed to Na⁺/K⁺-ATPase, ZO-1, and GAPDH using the following method. The primers were purchased from an oligonucleotide synthesizing company, INVITROGEN, and desalted primers were used. Corneal endothelial cells which naturally changed into a fibrous form, and normal corneal endothelial cells were used as specimens, and the mRNA amount of Na⁺/K⁺-ATPase and ZO-1 was examined using a semiquantitative PCR method. For the extraction of the total RNA from the cells, RNEasy (QIAGEN, catalog number: 74106) was used. The extracted RNA was subjected to reverse transcription reaction (42°C, 60 minutes) using ReverTra Ace (TOYOBO (catalog number: TRT-101)), and Na⁺/K⁺-ATPase and ZO-1 were amplified using TAKARA Taq HotStart Version (TAKARA BIO INC., catalog number: RR001A) with GAPDH as an internal standard. The same amount of cDNA was amplified using a PCR machine (GeneAmp 9700; Applied Biosystems) and the below primer pair. For PCR reaction, the following primers were used. The following primers were also used in a similar manner for the PCR reaction to antibodies to fibronectin, collagen type 1 and 4, integrin α5, and integrin β1.
*Na⁺/K⁺-ATPase-F-CTTCCTCCGCATTTATGCTCATTTTCTCACCC (SEQ ID NO: 1)
*Na⁺/K⁺-ATPase-R: GGATGATCATAAACTTAGCCTTGATGAACTC (SEQ ID NO: 2)
*ZO-1-F: GGACGAGGCATCATCCCTAA (SEQ ID NO: 3)
*ZO-1-R: CCAGCTTCTCGAAGAACCAC (SEQ ID NO: 4)
*GAPDH-F: GAGTCAACGGATTTGGTCGT (SEQ ID NO: 5)
*GAPDH-R: TTGATTTTGGAGGGATCTCG (SEQ ID NO: 6)
*Collagen 1-F: TCGGCGAGAGCATGACCGATGGAT (SEQ ID NO: 7)
*Collagen 1-R: GACGCTGTAGGT GAAGCGGCTGTT (SEQ ID NO: 8)
*Collagen 4-F: AGCAAGGTGTTACAGGATTGGT (SEQ ID NO: 9)
*Collagen 4-R: AGAAGGACACTGTGGGTCATCT (SEQ ID NO: 10)
*Collagen 8-F: ATGTGATGGCTGTGCTGCTGCTGCCT (SEQ ID NO: 11)
*Collagen 8-R: CTCTTGGGCCAGGCTCTCCA (SEQ ID NO: 12)
*Fibronectin-F: AGATGAGTGGGAACGAATGTCT (SEQ ID NO: 13)
*Fibronectin-R: GAGGGTCACACTTGAATTCTCC (SEQ ID NO: 14)
*integrin α5-F: TCCTCAGCAAGAATCTCAACAA (SEQ ID NO: 15)
*integrin α5-R: GTTGAGTCCCGTAACTCTGGTC (SEQ ID NO: 16)
*integrin β1-F: GCTGAAGACTATCCCATTGACC (SEQ ID NO: 17)
*integrin β1-R: ATTTCCAGATATGCGCTGTTTT (SEQ ID NO: 18)

Amplified DNA fragments were electrophoresed with 1.5% agarose gel (Nacalai Tesque, catalog number: 01149-76), and detected by staining with ethidium bromide (Nacalai Tesque, catalog number: 14603-51). *Quantitative PCR was performed using the following TaqMan (registered trademark) (Invitrogen) primers. collagen type 1: Hs00164004_m1; fibronectin : Hs01549976_m1; GAPDH: Hs00266705_g1. The PCR was performed using a StepOne™ (Applied Biosystems) real-time PCR system. The GAPDH was used as an internal standard.

### (Result)

As shown in Figure **2****,** when cell culturing was conducted based on prior art, the normal functions were lost in the monkey corneal endothelial cells which were morphologically changed to the fibroblast phenotype. In comparison with monkey corneal endothelial cells which were cultured into a normal form, it was shown that the monkey corneal endothelium would lose the expression of a function-associated marker by the morphological change to the fibroblast phenotype(fibroblastic), through the immunostaining, Western blot technique and real-time PCR method.

More particularly, two different phenotypes of primate CEC in cell culture were shown. Most interestingly, the primate CEC in culture showed two different phenotypes when determined by cell forms and phenotypes of characteristic contact inhibition type. About 60% of the cells maintained a characteristic, polygonal cell shape and a contact inhibition-type phenotype, and these cells were referred to as normal phenotype. On the other hand, 40% of the cells showed a fibroblastic shape having multi layers, and these cells were referred to as fibroblast-like phenotype (Figure **1**). Next, these two phenotypes were examined with regard to endothelial characteristics; the staining pattern of the Na⁺/K⁺-ATPase and ZO-1 in plasma membrane was well preserved in normal phenotype, while the fibroblast-like phenotype completely lost the characteristic staining profile of Na⁺/K⁺-ATPase and ZO-1 in plasma membrane (left side in Figure **2**). The expression of two functional proteins, which were observed in both of the protein level (upper right, Figure **2**) and mRNA level (lower right, Figure **2**), was significantly higher in the normal phenotype than the fibroblast-like phenotype.

### (Behavior of Extracellular Matrix)

The fibroblast primate CEC was further examined with respect to the state of the extracellular matrix and the like. Figure **2A** shows the results.

Figure **2A** shows that fibroblast primate CEC produces an abnormal extracellular matrix, and specifically, it shows that normal functions are lost when cultured based on prior art. (A) shows expression in a fibroblast phenotype and normal cell phenotype in fibronectin and collagen type 1. The top row shows fibronectin, and the bottom row shows collagen type 1. The left side shows a normal cell phenotype, and the right side shows a fibroblast phenotype. The fibroblast phenotype showed an excess extracellular matrix such as fibronectin and collagen type 1. On the other hand, the normal cell phenotype completely lost staining capacity. (B) shows Western blot of the expression of fibronectin protein in the cells of fibroblast phenotype and normal phenotype. The GAPDH was used as control. The protein expression level of fibronectin was upregulated more in the fibroblast phenotype than the normal phenotype. (C) shows results of semiquantitative PCR in fibroblast phenotype (right) and normal cell phenotype (left) of collagen type 1, type 4 and type 8, fibronectin, integrin α5, and integrin β1 (listed in order from the top). The GAPDH was used as a control. From the semiquantitative PCR analysis, while collagen type 1 transcripts (α1 (I) mRNA) were expressed abundantly in the fibroblast phenotype, the expression of the α1(I)mRNA was decreased in the normal phenotype. Basement membrane collagen α1 (IV) mRNA and α1 (VIII) mRNA, were expressed in both of the normal phenotype and fibroblast phenotype, but the degree of the expression in the normal phenotype was less than that of the fibroblast phenotype. The mRNA of fibronectin and integrin α5 was observed in the fibroblast phenotype, but the mRNA of these two types was not expressed in the normal phenotype . The mRNA of β1 integrin was expressed at similar levels in both of the phenotypes.

From the comparison of true fibrous extracellular matrix (ECM) proteins, those of the fibroblast phenotype showed a fibrous ECM staining pattern of fibronectin, while the normal phenotype completely lost the staining capacity of fibronectin (A in Figure **2A**)**.** The protein level of fibronectin was upregulated more in the fibroblast phenotype than in the normal phenotype (B in Figure **2A**). The collagen type 1 produced by the fibroblast phenotype shows expression at overlapping sites, which was seen both in the ECM and cytoplasm. Interestingly, the site of the cytoplasm in collagen type 1 is in the golgi complex, and therefore the intracellular localization thereof is essential for secretion. These findings are similar to existing data (Ko MK, Kay EP. Subcellular localization of procollagen I and prolyl 4-hydroxylase in corneal endothelial cells. Experimental cell research. 2001;264:363-71) . On the other hand, collagen type 1 staining in the normal phenotype was not clearly observed (Figure **2A**). Major ECM protein expression was measured using RT-PCR analysis. It was found that collagen type 1 transcript (α1 (I) mRNA) was expressed abundantly in the fibroblast phenotype . On the other hand, the expression of the α1 (I) mRNA was negligible in the normal phenotype (Figure **2C**). In contrast to the transcript of the collagen type I, the mRNA of basement membrane collagen phenotypes, α1 (IV) mRNA and αI (VIII), was expressed in both of the normal phenotype and fibroblast phenotype, but the degree of the expression was less in the normal phenotype than in the fibroblast phenotype. The expression of the fibronectin and integrin α5 was observed in the fibroblast phenotype. On contrary, these two transcripts were not expressed in the normal phenotype (C in Figure **2A**). On the other hand, the mRNA of the β1 integrin was expressed at similar levels in both of the normal phenotype and fibroblast phenotype (C in Figure **2A**).

### (Comparative Example 3: Loss of Normal Functions in Another Method of Conventional Methods)

In the present Comparative Example, it was confirmed that fibrosis of corneal endothelial cells occurred with a conventional culturing method (Figure **3**). The conditioned culture medium derived from 3T3 feeder cells suppresses fibroblastic change. However, it is shown that the use of only the conditioned culture medium derived from the 3T3 feeder cells results in transformation when subculturing is performed (right side in Figure **3**).

### (Material and Method)

Among the materials used, the materials that were the same as those in Comparative Example 1 and 2 were obtained, cultured and the like in a similar manner.
*Control: the culture medium used for the culturing of a control, human corneal endothelial cell, was the following: Opti-MEM I Reduced-Serum Medium, Liquid (INVITROGEN catalog number: 31985-070) +8% FBS (BIOWEST, catalog number: S1820-500) + 200 mg/ml CaCl₂•2H₂O (SIGMA catalog number: C7902-500G) + 0.08% chondroitin sulfuric acid (SIGMA catalog number: C9819-5G) + 20 µg/ml ascorbic acid (SIGMA catalog number: A4544-25G) + 50 µg/ml gentamicin (INVITROGEN catalog number: 15710-064) + 5 ng/ml EGF (INVITROGEN catalog number: PHG0311) .
*Conditioned culture medium for 3T3 feeder cell: NIH3T3 cells were disseminated to a 150mm dish (FALCON, catalog number: 3025), coated with 0.1% gelatin (SIGMA, catalog number: G1890-500G), using 10% FBS (BIOWEST, catalog number: S1820-500) / DMEM (INVITROGEN, catalog number: 12320), followed by culturing to subconfluency. Next, incubation was performed for 2 hours in a mitomycin C solution with a final concentration of 0.04mg/mL (Kyowa Hakko Kirin Co., Ltd, catalog number: 874231) at 37°C, in a 5% CO₂ incubator. Culturing was performed overnight by substitution with a 10% FBS (BIOWEST, catalog number: S1820-500) / DMEM culture medium. Opti-MEMI Reduced-Serum Medium, Liquid (INVITROGEN, catalog number: 31985-070) + 8% FBS (BIOWEST, catalog number: S1820-500) + 200 mg/ml CaCl₂•2H₂O (SIGMA, catalog number: C7902-500G) + 0.08% chondroitinsulfuric acid (SIGMA, catalog number: C9819-5G) + 20 µg/ml ascorbic acid (SIGMA, catalog number: A4544-25G) + 50 µg/ml gentamicin (INVITROGEN, catalog number: 15710-064) + 5 ng/ml EGF (INVITROGEN, catalog number: PHG0311) were added to thus created NIH3T3 cells, followed by culturing for overnight to create a conditioned culture medium for culturing human corneal endothelium.
*Culturing method: culturing was performed using respective culture media in a method similar to Comparative Example 1.
*Cell observation method such as staining: the form of cells was observed through a phase-contrast microscope.

### (Result)

As shown in Figure **3****,** it was indicated that fibrosis occurred in culturing with a conditioned culture medium using 3T3 feeder cells, as another conventional method, resulted in a condition that was not suitable for transplantation.

The results in Comparative Examples 1 and 3 allow one to confirm that when subculturing is performed, it becomes impossible for growth to occur while maintaining a normal condition with the conventional culture media for corneal endothelial cells, as shown in Non Patent Literature 7.

### (Example 1)

In the present Example, by focusing on the fact that transformation was in form of a fibroblastic manner, activation of a pathway that was activated in fibrosis induction known with a general cell species was examined using a Western blot method.

### (Material and Method)

Among the materials used hereinafter, the same materials as those used in the Comparative Examples 1 to 3 were obtained and subjected to culturing and the like in a similar manner to Comparative Examples 1 to 3.
*Cynomolgus monkey corneal endothelial cell: Eyeballs of cynomolgus monkeys, which were humanely killed for another purpose, were purchased (Nissei Bilis Co., Ltd., Ohtsu, Japan and Keari Co., Ltd., Wakayama, Japan), corneal endothelial cells were mechanically exfoliated together with basement membranes, followed by exfoliating from the basement membranes using dispase or collagenase for collection, and then primary culturing. For the culture medium, DMEM (INVITROGEN, catalog number: 12320) was used with 10% FBS (BIOWEST, catalog number:S1820-500) and 2 ng/ml basic FGF (INVITROGEN, catalog number: 13256-029) added thereto. At this stage, the monkey corneal endothelial cells may be cultured into a normal form as shown in Figure **1****,** but they often change their form in a fibroblastic manner by a similar culturing method, long-term culturing, and subculturing. Accordingly, cells cultured to a normal form and cells morphologically changed in a fibroblastic manner were collected and used for a Western blot method.
*Antibodies to pSmad2: those obtained from CELL SIGNALING (catalog number: 3108P) were used.
*Antibodies to pSmad: those obtained from CELL SIGNALING (catalog number: 5339P) were used.
*Antibodies to pp38: those obtained from BD TRANSDUCTIONL LABORATORIES (same as p38a/SAPK2a. catalog number: 612168) were used.
*Antibodies to p38: those obtained from BD TRANSDUCTIONL LABORATORIES (catalog number: 612280) were used.
*Antibodies to pERK1/2: those obtained from BD TRANSDUCTIONL LABORATORIES (catalog number: 612358) were used.
*Antibodies to ERK1/2: those obtained from BD TRANSDUCTIONL LABORATORIES (catalog number: 610030) were used.
*Antibodies to pJNK: those obtained from BD TRANSDUCTIONL LABORATORIES (catalog number: 610627) were used.
*Antibodies to JNK: those obtained from BD TRANSDUCTIONL LABORATORIES (catalog number: 612540) were used.

### (Experiment Method)

*Cellular fraction extracting and preparing methods: cells which had reached confluency were washed three times with PBS, followed by dissolution with RIPAbuffer (1 × PBS (Nissui, catalog number: 5913), 1% Nonidet
   P-40 (Nacalai Tesque, catalog number: 23640-94), 0.5% sodium deoxycholate (Nacalai Tesque, catalog number: 10712-12), and 0.1% SDS (Nacalai Tesque, catalog number:31607-65)). Thus obtained cell lysate was centrifuged (15, 000 rpm, 10 minutes), the supernatant thereof was collected, and the protein was quantitated using a BCA PROTEIN ASSAY KIT (PIERCE, catalog number: 23227) . Dissolution was performed using 100 µl dissolution buffer (Laemmli sample buffer) including 5mM 2-mercaptoethanol (Nacalai Tesque, catalog number: 21418-42).
*Western blot technique: the protein which was extracted using the RIPA buffer and obtained was electrophoresed with 7.5% polyacrylamide. The separated protein was transcribed to a PVDF film (PALL LIFE SCIENCE, catalog number: EH-2222) . The films were incubated for an hour in Tris buffered saline (10 mM Tris-HCl, pH7.4;100 mM NaCl) supplemented with 0.1% (vol/vol) polyethylene sorbitan monolaurate (Nacalai Tesque, catalog number: 28353-85) (TBS-T) and 5% fat-free dried milk (CELL SIGNALING, catalog number: 9999) for blocking. Thereafter, Smad2 antibodies, pSmad2 antibodies, p38 antibodies, pp38 antibodies, ERK antibodies, pERK antibodies, JNK antibodies, and pJNK antibodies, which were diluted by 1, 000 fold with TBS-T supplemented with 5% NON FAT DRY MILK (CELL SIGNALING, catalog number: 9999), and the films were immersed into the antibody solution for an hour to at room temperature to allow the reaction to take place . After washing 3 times with TBS-T, incubation was performed with a murine-IgG antibody HRP complex (CELL SIGNALING, catalog number: 7074P2) and a rabbit-IgG antibody HRP complex (GE Healthcare, catalog number: NA934). After washing, bands were detected using ECL-ADVAVCE (GE healthcare Japan, catalog number: RPN2135V) .

### (Result)

Figure **4** shows the activity of the primary pathway, which is associated with the cause of transformation of fibrosis, using monkey corneal endothelium, examined using Western blotting. In fibroblast, phosphorylation of Smad2 (activation of TGF-β pathway), activation of p38 MAPK, and activation of JNK pathway were determined. On the other hand, phosphorylation of ERK1/2 was suppressed. According to a report, Smad2, p38, ERK1/2 and JNK were all involved in the EMT pathway [Chen KH, et al.(1999) Invest Ophthalmol Vis Sci 40: 2513-2519], [Kim TY, et al. (2001) Invest Ophthalmol Vis Sci 42: 3142-3149], [Naumann GO, et al. (2000) Ophthalmology 107: 1111-1124], [Parsons CJ, et al. (2007) J Gastroenterol Hepatol 22 Suppl 1: S79-84], [Ma FY, et al.(2009) Front Biosci (Schol Ed) 1: 171-187]. Thus, the inventors examined as to whether or not the Smad2 and MAPK were involved in endothelial-mesenchymal transition similar to EMT observed in epithelial cells. The phosphorylation of Smad2 was found to be greatly promoted in the fibroblast-like phenotype compared to those of the normal phenotype (Figure **4**)**.** The phosphorylation of p38 and ERK1/2 was greatly enhanced in the fibroblast-like phenotype, while the activation of JNK was negligible. However, the phosphorylation of ERK is influenced by cell growth in addition to change in the fibrosis of cells, it has been confirmed that different results may be observed in accordance with conditions of growth of the cells. These findings indicate that TGF-β signaling can take an important role in the fibroblast transition of the CEC.

### (Example 2: inhibition example of transformation of corneal endothelium)

In the present example, an example will be described where TGF-β signals were inhibited by a phosphorylation inhibitor of a receptor so that the transformation of monkey corneal endothelium was able to be suppressed.

### (Material and Method)

Among the materials used, the materials that were the same as those in Comparative Examples 1 to 3 were obtained, cultured and the like in a similar manner as in Comparative Example 1 to 3.
*Cynomolgus monkey corneal endothelial cell: Eyeballs of cynomolgus monkeys, which were humanely killed for another purpose, were purchased (Nissei Bilis Co., Ltd., Ohtsu, Japan and Keari Co., Ltd., Wakayama, Japan), corneal endothelial cells were mechanically exfoliated together with basement membranes, followed by exfoliating from the basement membranes using collagenase for collection, and then primary culturing. At this stage, the same corneas were divided into two, and a DMEM (INVITROGEN, catalog number: 12320)) with a base culture medium for culturing monkey corneal endothelium (10% FBS (BIOWEST, catalog number: S1820-500) and 2 ng/ml basic FGF (INVITROGEN, catalog number: 13256-029) added thereto, and abase culture medium with 1 µmοl/1 SB431542 (TOCRIS, catalog number: 1614) added thereto.

### (Result)

As shown in Figure **5****,** while the phase difference images show that primate CEC, cultured in the presence of SB431542, showed a true shape of a polygonal cell and a single layer of a contact inhibition type, it was demonstrated that the CEC of the control showed a fibroblast phenotype. While the one cultured by the base culture medium of the control was recognized to be transformed into fibroblast phenotype and to be multi-layered, cells cultured in the presence of the phosphorylation inhibitor resulting in inhibition of TGF-β signaling the phenotype was similar to the living organism in that a layer of cells of polygonal shape was observed, demonstrating the suppression of transformation of the monkey corneal endothelium.

### (Example 3: Demonstration of corneal endothelium maintaining normal functions)

In the present Example, by the present invention, as the demonstration of normalization of culturing, it was demonstrated that function-associated protein of corneal endothelium would be maintained. The details will be provided hereinafter.

### (Material and Method)

Among the materials used hereinafter, the same materials as those used in the above Comparative Examples. Method of culturing and the like was conducted in a similar manner as the Comparative Examples. In particular, materials similar to those in Example 2 were used.
*SB431542: this was obtained from TOCRIS (catalog number: 1614) .
*Antibodies to Na⁺/K⁺-ATPase: those obtained from MILLIPORE (catalog number: 05-369) were used.
*Antibodies to ZO-1: mice, those obtained from INVITROGEN (catalog number: 339100) were used; and rabbits, those obtained from ZYMED LABORATORIES (catalog number: 61-7300) were used.
*Antibodies to GAPDH: those obtained from ABCAM (catalog number: ab36840) were used.
*Immunostaining and the like: cells cultured in a similar manner to Example 2 were fixed and immunostaining was performed on Na⁺/K⁺-ATPase and ZO-1, followed by taking an image using a fluorescence microscope.
*Western blot method: similar to Example 1, a Western blot method was performed on the Na⁺/K⁺-ATPase, ZO-1, and GAPDH.
*Realtime PCR method: in addition, PCR was performed for Na⁺/K⁺-ATPase, ZO-1, and GAPDH in the following method. The primers were purchased from an oligonucleotide synthesizing company, INVITROGEN, and desalted primer were used. Corneal endothelial cells which naturally changed into a fibrous form, and normal corneal endothelial cells were used as specimens, and the amount of mRNA for Na⁺/K⁺-ATPase and ZO-1 was examined using a semiquantitative PCR method. For the extraction of the total RNA from the cells, RNEasy (QIAGEN, catalog number: 74106) was used. The extracted RNA was subjected to reverse transcription reaction (42°C, 60 minutes) using ReverTra Ace (TOYOBO (catalog number: TRT-101)), and Na⁺/K⁺-ATPase and ZO-1 were amplified using TAKARA Taq HotStart Version (TAKARABIO INC., catalog number: RR001A) with GAPDH as an internal standard. For PCR reaction, the below described primers were used.
*Na⁺/K⁺-ATPase-F-CTTCCTCCGCATTTATGCTCATTTTCTCACCC (SEQ ID NO: 1)
*Na⁺/K⁺-ATPase-R-GGATGATCATAAACTTAGCCTTGATGAACTC (SEQ ID NO: 2)
*ZO-1-F-GGACGAGGCATCATCCCTAA (SEQ ID NO: 3)
*ZO-1-R-CCAGCTTCTCGAAGAACCAC (SEQ ID NO: 4)
*GAPDH-F-GAGTCAACGGATTTGGTCGT (SEQ ID NO: 5)
*GAPDH-R-TTGATTTTGGAGGGATCTCG (SEQ ID NO: 6)

Amplified DNA fragments were electrophoresed in a 1.5% agarose gel, and were detected by ethidium bromide staining.

### (Result)

As shown in Figure **6****,** in the monkey corneal endothelial cells transformed into fibroblast phenotype by culturing, the expression of the function-associated markers such as Na⁺/K⁺-ATPase and ZO-1 was detected by immunostaining, Western blotting, and PCR. In the meantime, it was indicated that inhibition of TGF-β signals by the phosphorylation inhibitor of the receptor allowed the function-associated protein of the monkey corneal endothelium to be maintained. Specifically, the CEC treated with SB431542 showed characteristic plasma membrane staining of Na⁺/K⁺-ATPase and ZO-1, while the CEC of the control lost its staining. It was thus suggested that the cells treated with SB431542 maintain endothelial functions (left side in Figure **6****).** In addition, expression of Na⁺/K⁺-ATPase and ZO-1 was strongly enhanced in the SB431542-treated, fibroblast-like phenotype in both of the protein (upper right in Figure **6**) and mRNA level (lower right in Figure **6**). These data further confirmed that TGF-β can be a direct mediator of endothelial-mesenchymal transition observed in the primate CEC culturing.

### (Example 4: Normalization losing ability of TGF-β)

In the present Example, in order to confirm that TGF-β signals are involved in the transformation of monkey corneal endothelium, TGF-β was added to induce transformation to show that function-associated protein would be lost (immunostaining). In addition, by Western blotting, TGF-β was added to induce transformation to show that function-associated protein would be lost. The details will be provided hereinafter.

### (Material and Method)

Among the materials used hereinafter, the same materials as those used in the above Comparative Examples and Examples were obtained and subjected to culturing and the like in a similar manner to the Comparative Examples and Examples.
*TGF-β: those available from R&D SYSTEMS (catalog number: 240-B) were used.
*Antibodies to Na⁺/K⁺-ATPase : those available from MILLIPORE (catalog number: 05-369) were used.
*Antibodies to ZO-1: mice, those available from INVITROGEN (catalog number: 339100) were used; and rabbits, those available from ZYMED LABORATORIES (catalog number: 61-7300) were used.
*Antibodies to GAPDH: those available from ABCAM (catalog number: ab36840) were used.
*Antibodies to pSmad2: those available from CELL SIGNALING (catalog number: 3108P) were used.
*Antibodies to pSmad: those available from CELL SIGNALING (catalog number: 5339P) were used.
*Culturing method: monkey corneal endothelial cells were cultured to their sub-confluency, and TGF-β1 was added thereto during culturing to reach the final concentration of 0, 1, 10 ng/mL, and the culturing was continued at 37C until the cells began to change in form.
*Staining method: the method was the same as the above Examples.
*Cellular fraction extracting method: the method was the same as the above Examples.
*Western blot technique: the method was the same as the above Examples.

### (Result)

As shown in Figure **7****,** immunostaining was performed to confirm that TGF-β signals were involved in the transformation of monkey corneal endothelium, and it was indicated that addition of TGF-β and induction of transformation resulted in the loss of function-associated protein. Specifically, as shown in Figure **7****,** it was indicated that when the normal phenotype was exposed to exogenous TGF-β, the normal phenotype transformed into a fibroblast-like cell. Staining patterns of Na⁺/K⁺-ATPase and ZO-1 in plasma membranes of the normal phenotype were completely lost by being exposed to TGF-β (middle and right columns in Figure **7****)**.

In addition, as shown in Figure **8****,** it was also indicated in Western blotting that addition of TGF-β and induction of transformation resulted in the loss of function-associated protein. In addition, it can be confirmed that phosphorylation of Smad2 was induced by the addition of TGF-β and the addition of TGF-β caused the activation of the downstream signaling. Based on this result, it is understood that the activation of TGF-β and the downstream signal thereof is the direct cause of the loss of normal functions, and the normalization can be maintained by inhibiting the pathway thereof. Specifically, while the growth factor significantly decreased the expression of these two proteins at the protein level in the concentration dependent form (left column in Figure **8**), the phosphorylation of Smad2 was greatly increased in the concentration dependent form (right column in Figure **8**). These data indicate that even the normal phenotype of primate CEC have a tendency to obtain a fibroblast-like phenotype in response to TGF-β stimulation.

### (Example 5: Demonstration with human cells)

In the present Example, it was also confirmed that in human corneal endothelium, inhibition of TGF-β signals by a phosphorylation inhibitor of a receptor suppressed the transformation, thereby culturing normal endothelium. The details will be provided hereinafter.

### (Material and Method)

Among the materials used hereinafter, the same materials as those used in the above Comparative Examples and Examples were obtained and subjected to culturing and the like in a similar manner to the Comparative Examples and Examples.

Corneal endothelial cells were mechanically exfoliated together with basement membranes from research corneas purchased from the Seattle bank, followed by exfoliating from the basement membranes using collagenase for collection, and then primary culturing. For the culture medium, Opti-MEM I Reduced-Serum Medium, Liquid (INVITROGEN, catalog number:31985-070) + 8% FBS (BIOWEST, catalog number: S1820-500) + 200 mg/ml CaCl₂•2H₂O (SIGMA, catalog number: C7902-500G) + 0.08% chondroitinsulfuric acid (SIGMA, catalog number: C9819-5G) + 20 µg/ml ascorbic acid (SIGMA, catalog number:A4544-25G) +50µg/ml gentamicin (INVITROGEN, catalog number: 15710-064) + 5 ng/ml EGF (INVITROGEN, catalog number: PHG0311), which were conditioned for 3T3 feeder cells, were used as a base culture medium. The collected human corneal endothelial cells were divided into two, and one group of them was cultured with the base culture medium as a control, and the other group of them was cultured with a base culture medium with SB431542 (TOCRIS, catalog number: 1614) added thereto such that the final concentration would be 1 µmol/L. *Enzyme-linked immunosorbent assay (ELISA) : type 1 collagen in a culture supernatant of HCEC was assayed by using a ELISA kits for Collagen Type I Alpha 2 (COL1a2) (Uscn Life Science Inc. , Wuhan, China) in accordance with the instruction manual of the manufacturer. Culture supernatants derived from HCEC, cultured together with SB431542 or without SB431542, were used for respective groups (n=5).

### (Result)

As shown in Figure **9****,** when cells were cultured in the SB431542-free base culture medium, the cells became transformed in a fibroblastic manner to be multi-layered. On the other hand, a layer of cells with a small difference in size of a polygonal shape were cultured in the medium culture with SB431542 added thereto. Based on this, not only in monkey corneal endothelium, but also in human corneal endothelium, it was confirmed that the inhibition of the TGF-β signals by the phosphorylation inhibitor of the receptor suppressed transformation, and normal endothelium was cultured. Specifically, based on the interesting finding observed in primate CEC, it was further examined as to whether or not HCEC was subjected to similar and undesirable, unavoidable change of cells to endothelial-mesenchymal transition. Most interestingly, cultured HCEC lost a characteristic, single-layer structure of contact inhibition type and a polygonal phenotype, and obtained a fibroblastic manner cell form like primate CEC (Figure **9**)**.**

### (Further analysis on SB431542)

Next, the inventors tested if SB431542 could maintain the functions of endothelial cells. Herein, it was demonstrated that SB431542 would maintain the functions of HCEC and suppress the fibroblastic manner change of HCEC, through various experiments. The results will be shown in Figure **9A****.**

As shown in A and B of Figure **9A****,** the blockage of TGF receptor signaling by SB431542 allows for the intracellular localization of Na⁺/K⁺-ATPase and ZO-1 in a cell membrane, which makes it possible to maintain the protein expression thereof. The scale bar shows 100 µm. As shown in C of Figure **9A****,** it was indicated that SB431542 significantly down-regulated the secretion of collagen type 1 to the cell supernatant, by ELISA assay. **P<0.05. As shown in D and E of Figure **9A****,** it was indicated that SB431542 significantly decreased the expression of collagen type 1 and fibronectin at mRNA level.

As described above, based on the findings shown in A and B of Figure **9A****,** it was demonstrated that the blockage of TGF receptor signaling by SB431542 allows for the intracellular localization of Na⁺/K⁺-ATPase and ZO-1 in a cell membrane (plasma membrane), which makes it possible to maintain the protein expression thereof. More importantly, it was clarified that SB431542 significantly down-regulated the secretion of collagen type 1 to the culture supernatant, by ELISA assay (C in Figure **9A**). In addition, SB431542 significantly decreased the expression of collagen type I and fibronectin at the mRNA level (D and E in Figure **9A**).

### (Example 6: Example of culture normalization of corneal endothelium using another method)

In the present Example, it was demonstrated that TGF-β signals was able to be counteracted and the transformation of human corneal endothelium was able to be suppressed using BMP-7 as a method other than those using SB431542, which was used in the above-mentioned Examples. The details will be provided hereinafter.

### (Material and Method)

Among the materials used hereinafter, the same materials as those used in the above Comparative Examples and Examples were obtained and subjected to culturing and the like in a similar manner to the Comparative Examples and Examples.
*BMP-7: those available from R&D SYSTEMS (catalog number: 354-BP) were used.
*phalloidin: Alexa Fluor (registered trademark) 488 (INVITROGEN, catalog number: A12379) was used.
*Culturing method: Human corneal endothelial cells were cultured in a conditioned culture medium using a method similar to that in Figure **3****.** Next, while subculturing was performed with trypsin, a comparison was made between the cells cultured in a cultured medium obtained by adding BMP-7 (100ng/ml) to a similar conditioned culture medium, and the cells cultured in a similar conditioned culture medium as a control.
*In a form observation by staining of cytoskeleton with phalloidin and a phase-contrast microscope, transformation occurred in a fibroblastic manner to be multi-layered in the control while the form of a layer of polygonal cells was maintained in the culture medium with BMP-7 added thereto .

### (Result)

As shown in Figure **10****,** TGF-β signals was able to be counteracted and the transformation of human corneal endothelium was able to be suppressed by using BMP-7 as a method other than those using SB431542. BMP-7 is known to be common as a factor associated with TGF-β signals while the other detailed transfer pathways are different from SB431542 . Specifically, TGF-β signaling pathways are broadly classified into a Smad2/3 system via ALK4, 5 or 7, and a Smad1/5/8 system via ALK1, 2, 3 or 6, either of which is well known to be associated with fibrosis. Accordingly, it is understood that by substantially suppressing the overall TGF-β signal, the transformation of corneal endothelium can be suppressed. Although it is not desired to be restricted by theories, since normalization was observed in both of SB431542, which exerted the effect through Smad2/3(associated with ALK4, 5 and 7), and BMP-7, which exerted the effect through Smad1/5/8 (associated with ALK1, 2, 3 and 6), it is understood that either TGF-β signal inhibiting agent of these pathways can achieve the effect according to the present invention. The TGF-β signaling pathways are broadly classified into a Smad2/3 system through ALK4/5/7, and a Smad1/5/8 system through ALK1/2/3/6, either of which is well known to be associated with fibrosis (J. Massagu'e, Annu. Rev. Biochem. 1998. 67: 753-91; Vilar JMG, Jansen R, Sander C (2006) PLoS Comput Biol 2(1) :e3; Leask, A., Abraham, D.J. FASEB J. 18, 816-827 (2004) ; CoertMargadant & Arnoud Sonnenberg EMBO reports (2010) 11, 97-105; Joel Rosenbloom et al., Ann Intern Med. 2010; 152: 159-166) . Thus, either of the two types of representative TGF-β signal inhibiting agents could achieve the culture normalization, and based on these results, it is understood that regardless of Smad pathways, any TGF-β signal inhibiting agent can function as a culture normalizing agent.

### (Confirmation of concentration dependency of BMP-7)

Next, by using three different concentrations, BMP7 was able to suppress the change in a HCEC to a fibroblast phenotype and to maintain the function thereof. BMP-7 promotes MET, and specifically inhibits TGF-β-mediated epithelial-mesenchymal transition. Thus, this molecule is used to antagonize an EMT process [ZeisbergM, et al. (2003) Nat Med 9: 964-968], [Simic P, et al. (2007) EMBO Rep 8: 327-331] , [BuijsJT, et al. (2007) Am J Pathol 171: 1047-1057], [Zeisberg M, et al. (2007) J BiolChem 282: 23337-23347]. Hence, the inventors examined as to whether or not BMP-7 was able to antagonize the unavoidable change of HCEC. Fibroblast-like HCEC was treated with BMP-7 in the concentration ranging from 10 to 1,000 ng/ml.

Results will be shown in Figure **10A****.** As shown in A of Figure **10A****,** the elongated cell form of the phenotype of the fibroblast was converted into a polygonal cell form in response in the presence of BMP-7, in a concentration-dependent manner. The scale bar shows 100 µm. As shown in B of Figure **10A****,** similar to those observed in normal CEC [BarryPA, et al. (1995) Invest Ophthalmol Vis Sci 36: 1115-1124], BMP-7 allowed for a normal hexagonal cell form, and allowed for cytoskeleton distribution on the cell surface of actine. The scale bar shows 100 µm. As shown in C and D of Figure **10A****,** BMP-7 maintained the intracellular localization of Na⁺/K⁺-ATPase and ZO-1 in a cell membrane (plasma membrane) . The scale bar shows 100 µm. As shown in E and F of Figure **10A****,** BMP-7 was able to maintain the CEC in a contact inhibition type phenotype of a polygonal shape, associated with positive expression of a function-associated marker, at a concentration of 1,000 ng/ml. Note that the control was not added. With regard to both of the Na⁺/K⁺-ATPase positive cell and ZO-1 positive cell, the ratio was significantly increased compared to the control when treated with BMP-7. *P<0.01, **P<0.05.

It was demonstrated that the use of BMP-7 suppresses the change in a fibroblastic manner and maintains the endothelial cell function.

The inventors examined as to whether or not bone morphogenic protein 7 (BMP-7) could inhibit preceding change of HCEC. Fibroblast-like HCEC was treated with BMP-7 in a concentration range of 10 ng/ml to 1,000 ng/ml. Importantly, the elongated cell form of the fibroblast-like phenotype was converted into a polygonal cell form in response in the presence of BMP-7, in a concentration-dependent manner (A in Figure **10A**). A hexagonal cell form has become possible, and maintenance of cytoskeleton distribution to the cell surface of actine has become possible by BMP-7 (B in Figure **10A**). This is a state similar to that observed with a normal CEC (Barry PA, Petroll WM, Andrews PM, Cavanagh HD, Jester JV. The spatial organization of corneal endothelial cytoskeletal proteins and their relationship to the apical junctional complex. Investigative ophthalmology & visual science. 1995; 36:1115-24). Intracellular localization of Na⁺/K⁺-ATPase positive cells (C in Figure **10A**) and ZO-1 positive cells (D in Figure **10A**) to a cell membrane was also maintained. Thus, it was indicated that BMP-7 was capable of maintaining the CEC in a polygonal form and maintaining the phenotype due to contact inhibition accompanied by positive expression of the function-associated marker, at a concentration of 1, 000 ng/ml (E and F of Figure **10A**)**.** This tendency was seen at 10 ng/ml, and the tendency increased at 100 ng/ml, and the tendency was more significant at 1, 000 ng/ml.

### (Example 7: Confirmation of additional effects)

In the present Example, it is indicated that in TGF-β signaling, SB203580, which is also an inhibitor of p38 MAPK, is to be used with SB431542, thereby enhancing culture normalization. The details will be provided hereinafter.

### (Material and Method)

Among the materials used hereinafter, the same materials as those used in the above Comparative Examples and Examples were obtained and subjected to culturing and the like in a similar manner to the Comparative Examples and Examples.

Corneal endothelial cells were mechanically exfoliated together with basement membranes from research corneas purchased from the Seattle bank, followed by exfoliating from the basement membranes using collagenase for collection, and then primary culturing. For the culture medium with regard to the humans, Opti-MEM I Reduced-Serum Medium, Liquid (INVITROGEN catalog number: 31985-070) + 8% FBS (BIOWEST, catalog number: S1820-500) + 200 mg/ml CaCl₂•2H₂O (SIGMA catalog number: C7902-500G) + 0.08% chondroitin sulfuric acid (SIGMA catalog number: C9819-5G) + 20 µg/ml ascorbic acid (SIGMA catalog number: A4544-25G) + 50 µg/ml gentamicin (INVITROGEN catalog number: 15710-064) + 5 ng/ml EGF (INVITROGEN catalog number : PHG0311), which were conditioned for 3T3 feeder cells, were used as a base culture medium. In addition, culturing was performed using a base culture medium with SB431542 (1µmol/l, TOCRIS, catalog number: 1614) added thereto, with SB203580 (1µmol/l, CALBIOCHEM, catalog number: 559389) added thereto, and with SB431542 (1µmol/l) and SB203580 (1 µmol/l) added thereto.

Collected human corneal endothelial cells were divided into two, one group of them was cultured with the base culture medium as a control, and the other group of them was cultured with a base culture medium with SB431542 (TOCRIS) added thereto such that the final concentration would be 1 µmol/l.

### (Result)

As shown in Figure **11****,** in TGF-β signaling, SB203580, which is also an inhibitor of p38 MAPK, was used in conjunction with SB431542, and it was demonstrated that culture normalization was enhanced; and furthermore, by adding SB203580, which is an inhibitor against p38 MAPK that is known to be activated due to aging, the corneal endothelium density was increased (the density is known to be decreased due to aging). Based on this fact, it is understood that the effect is enhanced by addition of SB203580 which further suppressed aging (maintaining an undifferentiated state) . As such, it has been found that by TGF-β signal inhibition in addition to p38 MAPK inhibition, even if human corneal endothelial cells repeat subculturing, the culturing of human corneal endothelial cells (HCEC) which retain their form with high density becomes possible, and the normalization of culturing is further enhanced (indicating that HCEC retaining their form with high density can be produced even subculturing is repeated).

Non Patent Literature 7 describes that conventional culture media for corneal endothelial cells are not able to allow cells to grow while maintaining normal state in subculturing. In addition, Non Patent Literatures 8 to 11 describe a culture medium including FBS, EGF andNGF, a culture medium with b-FGF used therefor, a culture medium with collagenase used therefor, and a culture medium with a conditioned culture medium used therefore, respectively. However, as indicated in Non Patent Literature 7 and Comparative Examples, none of the culture media are able to grow corneal endothelial cells while maintaining normal cell functions . If the effect of the culture medium or culture normalizing agent according to the present invention is evaluated in reference to this document, it is understood that the culture medium or culture normalizing agent has significantly more normalization maintaining ability compared to the culture media of prior art.

### (Example 8: Establishment of preferable culturing method)

Based on the results of the above-mentioned Examples and the like, establishment of a preferable culturing method for a human corneal endothelial cell was attempted. The details will be provided hereinafter.

### (Material and Method)

Among the materials used hereinafter, the same materials as those used in the above Comparative Examples and Examples were obtained and subjected to culturing and the like in a similar manner to the Comparative Examples and Examples.

Corneal endothelial cells were mechanically exfoliated together with basement membranes from research corneas purchased from the Seattle bank, followed by exfoliating from the basement membranes using collagenase for collection, and then primary culturing. For the culture medium with regard to the humans, Opti-MEM I Reduced-Serum Medium, Liquid (INVITROGEN catalog number: 31985-070) + 8% FBS (BIOWEST, catalog number: S1820-500) + 200 mg/ml CaCl₂•2H₂O (SIGMA catalog number: C7902-500G) + 0.08% chondroitin sulfuric acid (SIGMA catalog number: C9819-5G) + 20 µg/ml ascorbic acid (SIGMA catalog number: A4544-25G) + 50 µg/ml gentamicin (INVITROGEN catalog number: 15710-064) + 5 ng/ml EGF (INVITROGEN catalog number : PHG0311), which were conditioned for 3T3 feeder cells, were used for culturing as a base culture medium, with SB431542 (1 µmol/l) and SB203580 (1 µmol/l) added thereto.

The protocol is exemplified in Figure **12****.** The details are as follows.

### (Culturing method 1)

At the primary culturing and subculturing, a Rho kinase inhibitor having an adhesion promoting action, Y-27632 (WAKO or TOCRIS), was added for 48 hours with a final concentration of 10 µmol/l.

### (Culturing method 2)

A Rho kinase inhibitor, Y-27632 (WAKO, catalog number: 253-00513), with a final concentration of 10 µmol/l was added at all times during culturing.

### (Culturing method 3)

Without adding Y-27632, culturing was performed with SB431542 (1 µmol/l) and SB203580 (1 µmol/l) added as a base culture medium.

### (Result)

Figure **13** shows an example of human corneal endothelial cell culturing that has been established ultimately. As shown in the subject Figure and Figure **12****,** it was confirmed that any of culturing methods 1 to 3 grew human corneal endothelial cells, with high density, as cells indicating a normal state of a layer of a polygonal shape while maintaining the normal functions thereof, and an example of a standard culturing method was able to be established.

### (Example 9: Example of transplanting cultured human corneal endothelium)

A result will be described, in which human corneal endothelial cells cultured using the culturing method 3, among the culturing methods established in Example 8, were transplanted to a corneal endothelial failure model (bullous keratopathy model) using a primate, cynomolgus monkey. It is indicated that human corneal endothelial cells, which were cultured together with a ROCK inhibitor having an adhesion promoting action, were transplanted and the healing of the transparency in the cornea was attained. This indicates that the human corneal endothelial cells cultured in the present invention also exert a normal function in a living body and they can be applied for regenerative medicine.

### (Material and Method)

Among the materials used hereinafter, the same materials as those used in the above Comparative Examples and Examples were obtained and subjected to culturing and the like in a similar manner to the Comparative Examples and Examples.
*Cynomolgus monkey: after conducting ethical review at Research Center for Animal Life Science, Shiga University of Medical Science, the following examinations were performed from the viewpoint of animal protection as much as possible.

Under general anesthesia, the peripheral portion of a cornea of a cynomolgus monkey is cut by 1.5 mm, and a silicon surgical instrument is inserted into an anterior chamber to mechanically currete a corneal endothelial cell, thus creating a bullous keratopathy model. Next, of human corneal endothelial cells cultured by the method according to the present invention *ex vivo,* 2.0×10⁵ were suspended in a basal culture medium, and a ROCK inhibitor having an adhesion promoting effect, Y-27632, was added thereto such that the final concentration would be 100 µmol/l, followed by introduction into the anterior chamber. As a control, 2.0×10⁵ human corneal endothelial cells, suspended in a basal culture medium, were introduced without a ROCK inhibitor. After introduction, the model was maintained with its head down for three hours so that the eyeball would be downwards, thus promoting cellular adhesion to the corneal endothelium surface. Therapeutic effects of bullous keratopathy by transplanting a cultured corneal endothelium sheet was evaluated by corneal transparency evaluation through a slit-lamp microscope, and corneal thickness measurement using a ultrasonic tachymeter (Figure **14**). As shown in Figure **14****,** in a primate model corneal endothelial failure model, the cells cultured by the method according to the present invention demonstrated a favorable therapeutic result by the cells alone, and the therapeutic result was further improved after adding the ROCK inhibitor.

The model was euthanized 2.5 months later, and the corneas were extracted and the tissues were fixed. Then, similar to Example 2, immunostaining was performed directed to phalloidin, Na⁺/K⁺-ATPase and ZO-1, followed by taking images using a fluorescence microscope (Figure **15**). Results are shown in Figure 15. As also shown in Figure **15****,** in the primate model corneal endothelial failure model, the cells cultured by the method according to the present invention demonstrated a favorable therapeutic result by the cells alone, and the therapeutic result was further improved after adding the ROCK inhibitor. In particular, for the first time with human subjects, a treatment method which maintains a normal function of a corneal endothelium is provided by the present invention, which had not been achieved before.

### (Example 10: Example with anti-TGF-β neutralization antibody)

In the present Example, it was confirmed as to whether or not culture normalization would be similarly achieved with an anti-TGF-β neutralization antibody. Except for exchanging agents, the experiment was conducted in accordance with the above-mentioned Comparative Examples and Examples.

### (Material and Method)

Among the materials used hereinafter, the same materials as those used in the above Comparative Examples and Examples were obtained and subjected to culturing and the like in a similar manner to the Comparative Examples and Examples.
*Anti-TGF-β neutralization antibody: those available from R&D SYSTEMS (catalog number: MAB240) were used.
*Culturing method: human corneal endothelial cells were cultured using a method similar to the method that demonstrated the result in Figure **3** (see Comparative Example 3 and the like) . Next, subculturing was performed with trypsin. Comparison was made between cells cultured in Opti-MEM I Reduced-Serum Medium, Liquid (INVITROGEN catalog number: 31985-070) + 8% fetal bovine serum (FBS) (BIOWEST, catalog number: S1820-500) + 200 mg/ml CaCl₂•2H₂O (SIGMA catalog number: C7902-500G) + 0.08% chondroitin sulfuric acid (SIGMA catalog number: C9819-5G) + 20 µg ascorbic acid (SIGMA catalog number: A4544-25G) + 50 µg/ml gentamicin (INVITROGEN catalog number: 15710-064) + 5 ng/ml EGF (INVITROGEN catalog number: PHG0311) as a normal culture medium, and cells cultured in the above-mentioned culture medium with TGF-β neutralization antibodies (500 ng/ml) added thereto.
*In a form observation through a phase-contrast microscope, as shown in Figure **16****,** transformation occurred and the form of a polygonal cell was lost, and many cells with difference in size were recognized in the normal culture medium, while a layer of polygonal cells with high density were maintained in the TGF-β neutralization antibody-added culture medium. In conformity to primate CEC, when CEC was cultured together with a specific inhibitor (SB431542) to a TGF-β receptor, this inhibitor was able to block the shape of the cell from changing into a fibroblast-like phenotype. Similar to the inhibitory action of SB431542 to fibroblast-like phenotypes, the neutralization antibody (Figure **16B**) to TGF-β also blocked the cells from obtaining a fibroblast-like phenotype.

As such, the anti-TGF-β neutralization antibody was used, instead of SB431542 or BMP-7, to conduct similar experimentation, thereby confirming culture normalization.

In the present Example as well, by neutralizing the TGF-β itself, it was indicated that, even if TGF-β signaling was inhibited, fibrosis was suppressed, and the activity of ZO-1 and Na⁺/K⁺-ATPase was retained. It was thus demonstrated that even if subculturing is performed, the cells can be grown while maintaining the "normalization" activity.

### (Example 11: Example of culture normalization of corneal endothelium using another method)

In the present Example, it was demonstrated that TGF-β signals would be inhibited by using a Smad3 inhibitor, as a method other than SB431542 used in the above-mentioned Example, to suppress the transformation of a human corneal endothelium. The details will be provided hereinafter.

### (Material and Method)

Among the materials used hereinafter, the same materials as those used in the above Comparative Examples and Examples were obtained and subjected to culturing and the like in a similar manner to the Comparative Examples and Examples.
*Smad3 inhibitor: 6,7-dimethoxy-2-((2E)-3-(1-methyl-2-phenyl-1H-pyrrolo[2 ,3-b]pyridine-3-yl-prop-2-enoyl))-1,2,3,4-tetrahydroiso quinolone (catalog number: 566405) available from Calbiochem was used. The Smad3 inhibitor is also available from Merck Millipore.
*Culturing method: human corneal endothelial cells were cultured using a method similar to the method that demonstrated the result in Figure **3** (see Comparative Example 3 and the like) . Next, subculturing was performed with trypsin. Comparison was made between cells cultured in Opti-MEM I Reduced-Serum Medium, Liquid (INVITROGEN catalog number: 31985-070) + 8% fetal bovine serum (FBS) (BIOWEST, catalog number: S1820-500) + 200 mg/ml CaCl₂•2H₂O (SIGMA catalog number: C7902-500G) + 0.08% chondroitin sulfuric acid (SIGMA catalog number: C9819-5G) + 20 µg ascorbic acid (SIGMA catalog number: A4544-25G) + 50 µg/ml gentamicin (INVITROGEN catalog number: 15710-064) + 5 ng/ml EGF (INVITROGEN catalog number: PHG0311) as a normal culture medium, and cells cultured in the above-mentioned culture medium with Smad3 inhibitor (0.3mM and 3mM) added thereto.

Figure **17** shows the result, in a form observation through a phase-contrast microscope, the cells became transformed and lost the form of a polygonal cell and many cells were recognized to have difference in size in the normal culture medium, while a layer of polygonal cells with high density were maintained both with 0.3mM and 3mM in the Smad3 inhibitor-added culture medium. Specifically, in conformity to primate CEC, when CEC was cultured together with a specific inhibitor (SB431542) to TGF-βreceptor, this inhibitor was able to block the shape of the cell from changing into a fibroblast-like phenotype. Similar to the inhibitory action of SB431542 to fibroblast-like phenotypes, Smad3 inhibitor (Figure **17**) also blocked the cells from obtaining a fibroblast-like phenotype.

### (Consideration)

Corneal endothelial dysfunction associated with a visual impairment is a major indication of corneal transplant operations [Darlington JK, et al. (2006) Ophthalmology 113: 2171-2175], [Price MO, et al. (2010) Clin Experiment Ophthalmol 38: 128-140]. While corneal transplantation is broadly performed for corneal endothelial dysfunction, researchers are currently searching for an alternative method for recovering a healthy corneal endothelium. As corneal endothelium is cultured from a young donor to be stocked as "master cell", transplantation of a cell having high functional ability becomes possible. In addition, HLA adoptive transplantation for reducing the risk of rejection [Khaireddin R, et al. (2003) Graefes Arch Clin Exp Ophthalmol 241: 1020-1028], [Coster DJ, et al. (2005) Am J Ophthalmol 140: 1112-1122] becomes possible. Tissue bionics is a new approach for developing a treatment for patients who have lost their eyesight [Engelmann K, et al. (2004) Exp Eye Res 78: 573-578] . To date, there are two methods existing which utilize a bionic approach: 1) use of a culture donor HCEC adhered on a bionic construct [Ishino Y, et al. (2004) Invest Ophthalmol Vis Sci 45: 800-806], [Mimura T, et al. (2004) Invest Ophthalmol Vis Sci 45: 2992-2997], [Koizumi N, et al. (2007) Invest Ophthalmol Vis Sci 48: 4519-4526], [Koizumi N, et al. (2012) Exp Eye Res 95: 60-67], and 2) transplantation of cultured HCEC into an anterior eye chamber [Okumura N, et al. (2012) Am J Pathol 181: 268-277], [Mimura T, et al. (2003) Exp Eye Res 76: 745-751], [Mimura T, et al. (2005) Invest Ophthalmol Vis Sci 46: 3128-3135], [Patel SV, et al. (2009) Invest Ophthalmol Vis Sci 50: 2123-2131] . Regardless of the application of either of the two methods in accordance with clinical situations, the establishment of an effective culturing technique for HCEC is imperative and unavoidable [Peh GS, et al. (2011) Transplantation 91: 811-819]. Many researchers admit that it is extremely difficult to establish the permanent and long-term culturing of HCEC [Engelmann K, et al. (2004) Exp Eye Res 78: 573-578]. Although successful culturing of HCEC has been reported by several groups, isolation and procedures associated with the subsequent culturing protocol extremely vary between research laboratories [Peh GS, et al. (2011) Transplantation 91: 811-819]. One of the most difficult problems is that HCEC is outrageously susceptible to fibroblast-like change for each subculturing [Engelmann K, et al. (2004) Exp Eye Res 78: 573-578] . Hence, it is essential to find a means to avoid voluntary transition of CEC in order to maintain the physiological phenotype for subsequent usage for the purpose of transplantation.

The transition of an endothelial cell to a fibroblast-like cell is referred to as endothelial-mesenchymal transition. Such a transition is induced by TGF-β through a Smad2/3 pathway [Saika S (2006) Lab Invest 86: 106-115] . Endothelial-mesenchymal transition causes loss of a single layer of contact inhibition type, and loss of apical binding protein in a plasma membrane and other characteristic endothelial phenotype loss. Furthermore, this causes induction of fibrous protein, such as type 1 collagen and fibronectin. In the present research, the inventors demonstrated that the fibroblast-like phenotype of cultured CEC would greatly lose endothelial characteristics; the expression of Na⁺/K⁺-ATPase and ZO-1 was significantly decreased, and the intracellular localization thereof was in, not the true cell membrane, but in the cytosol. Furthermore, the fibroblast-like phenotype significantly enhances the production of, not basement membrane phenotype (type IV and type VIII collagen), but fibrous ECM protein (type 1 collagen, fibronectin and integrin α5). The presence of such an undesirable cell will greatly impede the success of transplantation of cultured cells in a clinical circumstance. Hence, it is important to determine what causes the change of phenotypes, and how it interferes such an endothelial-mesenchymal transition process of cultured CEC. Based on the fact that the phosphorylation of Smad2/3 greatly enhanced in the fibroblast-like phenotype, the inventors have concluded that the fibroblast-like phenotype is mediated by TGF-β signaling in the CEC of both primates and humans. Thus, the inventors used a specific inhibitor (SB431542) to a TGF-β receptor to block the endothelial-mesenchymal transition process observed in the fibroblast-like phenotype [Inman GJ, et al. (2002) Mol Pharmacol 62: 65-74] . SB431542 completely cancels an undesirable change of cells; and when the CEC culturing of either of primate or human was treated with SB431542, the unavoidable change of the cells to fibroblast-like phenotype was completely canceled. At the same time, the characteristic intracellular position of ZO-1 and Na⁺/K⁺-ATPase returned to plasma membrane, and, the expression of these two proteins was greatly increased in both levels of mRNA and protein, which suggests that the barrier and pumping functions are intact in such culturing. Furthermore, the inventors also found that the production of fibrous ECM protein was greatly decreased. The inventors further examined an action of reversing the fibroblast-like phenotype of HCEC by a well known anti-EMT factor, BMP-7 [Zeisberg M, et al. (2003) Nat Med 9: 964-968], [Zeisberg M, et al. (2007) J Biol Chem 282: 23337-23347]. BMP-7 also reversed the fibroblast-like phenotype to a normal corneal endothelial cell having a characteristic endothelial adhesion of a single layer. In summary, both SB431542 and BMP-7 can be a powerful tool for maintaining a normal endothelial phenotype of cultured CEC, which therefore lead to the success in the subsequent transplantation.

As a conclusion, the findings by the Inventors indicated that the use of the inhibitor (SB431542) to TGF-β receptor and/or anti-EMT molecule (BMP-7) made it possible to grow HCEC while maintaining normal physiological functions (i.e., barrier and pumping functions). Although more in-depth, future research is beneficial, the inventors have not seen any clear, harmful effects in the consecutive treatments with SB431542 or BMP-7, with regard to the forms or functions, even after several passages. It may be demonstrated that the present research is to provide a protocol related to an effective culturing method *ex vivo* of HCEC to be used for regenerative medicine. In addition, this new strategy of inhibiting the fibroblast-like change during culturing can ultimately provide clinicians with novel treatment modality in regenerative medicine, not only for the treatment of corneal endothelial dysfunction, but also for various types of pathological diseases in general.

### (Example 12: Example with other inhibitors)

In the present Example, it is confirmed as to whether or not culture normalization is achieved in a similar manner with other TGF-β signal inhibiting agents. Except for exchanging agents, the experiment can be conducted in accordance with the above-mentioned Examples.

### (Material and Method)

*A83-01 (available from TOCRIS or Miltenyi Biotec) : A83-01 is a selective inhibitory substance of type I TGF-b receptor ALK5, Activin/Nodal receptor ALK4, and Nodal receptor ALK7.
*Stemolecule™ ALK5 inhibitor (available from Miltenyi Biotec): this is a selective ATP competitive inhibitory substance of a type I TGF-b receptor, activin receptor-like kinase (ALK5).
*LDN-193189 (available from Miltenyi Biotec) : this inhibits BMP Type I receptor ALK2 and ALK3.
*siRNA of Smad (synthesized using a standard method)

The above materials were used instead of SB431542 or BMP-7, which were used in the above-mentioned Examples, to conduct a similar experiment, confirming culture normalization.

In the present Example as well, it was indicated that any of the TGF-β signal inhibiting agents suppressed fibrosis and retained the activity of ZO-1 and Na⁺/K⁺-ATPase; and it is demonstrated that even if subculturing is performed, the cells can be grown while the "normalization" activity is maintained.

### (Example 12: Exemplary formulation: culture solution for preparing corneal endothelium sheets)

In the present Example, as an exemplary formulation, a culture solution for preparing corneal endothelium sheet, containing the culture normalizing agent according to the present invention, is manufactured as follows.

Using an ordinary method, the below-indicated culture solution is prepared.
SB431542 3.8439 mg
SB203580 3.7743 mg
FBS 10 mL
penicillin-streptomycin solution 1 mL
FGF basic 200 ng
DMEM appropriate amount
total amount 100 mL

FBS is available from, for example, BIOWEST (catalog number: S1820-500) or Invitrogen. Penicillin-streptomycin solution is available from Nacalai Tesque (containing 5,000 u/mL penicillin, 5,000 µg/mL streptomycin). FGF basic is available from, for example, Invitrogen (INVITROGEN, catalog number: 13256-029). SB431542 is available from TOCRIS. SB203580 is available from CALBIOCHEM. DMEM is available from Invitrogen.

### (Example 13: Exemplary formulation: cornea preservation solution containing culture normalizing agent)

In the present Example, as an exemplary formulation, a cornea preservation solution containing the culture normalizing agent according to the present invention is manufactured as follows.

The below indicated preservation solution is prepared using an ordinary method.
SB431542 3.8439 mg
SB203580 3.7743 mg
Optisol-GS (Bausch-Lomb) appropriate amount
total amount 100 mL

The respective ingredients are available in a similar manner as described in Example 12.

### (Example 14: Creation of transplantation-purpose, cultured corneal endothelial cell sheet)

In the present Example, rabbit corneal endothelial cells prepared using the technique established in Example 8 or a method equivalent thereto are used. In addition, in the present Example, a Rho kinase inhibitor or a control substance is used as a cell adhesion promoting agent prepared using a method similar to that in Example 9.

During the creation of the transplantation-purpose, cultured corneal endothelial cell sheet, a Rho kinase inhibitor, for example, Y-27632 is added, and immune cell fluorescence staining is performed using a technique similar to the above-mentioned Examples, on ZO-1 and Na⁺/K⁺ATPase, which are functional proteins of corneal endothelial cells, to confirm expression.

A corneal endothelium sheet is fixed with 95% ethanol (-30°C) for 10 minutes. After PBS washing, the corneal endothelium sheet is treated with 0.5% TritonX-100/PBS for 5 minutes. Thereafter, treatment is performed with 1% BSA/PBS for 1 hour. Thereafter, anti-ZO-1 antibodies or anti-Na⁺/K⁺ATPase antibodies are treated overnight. After PBS washing, Alexa-488 label secondary antibodies are treated for 1 hour. After PBS washing, DAPI-containing mounting agent is added dropwise, followed by encapsulating with a cover glass. Photographs are taken using a fluorescence microscope to confirm the expression of ZO-1 and Na+/K+ ATPase.

### (Example 15: Exemplary preparation of impregnating agent) Exemplary preparation of an eye lotion

Composition is shown for test substances of respective concentrations, as follows.

Y-27632 (WAKO, catalog number: 253-00513) or other Rho kinase inhibitors 0.003g, 0.01g, 0.03g, 0.05g or 0.1g (dose as a dehydrochlorination body)

| | |
|---|---|
| sodium chloride | 0.85g |
| sodium dihydrogenphosphate dihydrate | 0.1g |
| benzalkonium chloride | 0.005g |
| sodium hydroxide | appropriate amount |
| purified water | appropriate amount |

The eye lotion may be diluted with a base.

### Composition of the base is as follows.

| | |
|---|---|
| sodium chloride | 0.85g |
| sodium dihydrogenphosphate dihydrate | 0.1g |
| benzalkonium chloride | 0.005g |
| sodium hydroxide | appropriate amount |
| purified water | appropriate amount |
| total amount | 100 mg (pH 7.0) |

### [Industrial Applicability]

The present invention provides a method for normalized culturing of corneal endothelial cell, and provides a technique which can be used in an industry (such as cell culturing industry and pharmaceutical industry) involved in the techniques related to corneal transplantation.

### [Sequence Listing Free Text]

SEQ ID NO: 1: forward primer of Na⁺/K⁺-ATPase; CTTCCTCCGCATTTATGCTCATTTTCTCACCC
SEQ ID NO: 2: reverse primer of Na⁺/K⁺-ATPase; GGATGATCATAAACTTAGCCTTGATGAACTC
SEQ ID NO: 3: forward primer of ZO-1; GGACGAGGCATCATCCCTAA
SEQ ID NO: 4: reverse primer of ZO-1; CCAGCTTCTCGAAGAACCAC
SEQ ID NO: 5: forward primer of GAPDH: GAGTCAACGGATTTGGTCGT
SEQ ID NO: 6: reverse primer of GAPDH: TTGATTTTGGAGGGATCTCG
SEQ ID NO: 7: forward primer of Collagen 1: TCGGCGAGAGCATGACCGATGGAT
SEQ ID NO: 8: reverse primer of Collagen 1: GACGCTGTAGGT GAAGCGGCTGTT
SEQ ID NO: 9: forward primer of Collagen 4: AGCAAGGTGTTACAGGATTGGT
SEQ ID NO: 10: reverse primer of Collagen 4: AGAAGGACACTGTGGGTCATCT
SEQ ID NO: 11: forward primer of Collagen 8: ATGTGATGGCTGTGCTGCTGCTGCCT
SEQ ID NO: 12: reverse primer of Collagen 8: CTCTTGGGCCAGGCTCTCCA
SEQ ID NO: 13: forward primer of Fibronectin: AGATGAGTGGGAACGAATGTCT
SEQ ID NO: 14: reverse primer of Fibronectin: GAGGGTCACACTTGAATTCTCC
SEQ ID NO: 15: forward primer of integrin α5: TCCTCAGCAAGAATCTCAACAA
SEQ ID NO: 16: reverse primer of integrin α5: GTTGAGTCCCGTAACTCTGGTC
SEQ ID NO: 17: forward primer of integrin β1: GCTGAAGACTATCCCATTGACC
SEQ ID NO: 18: reverse primer of integrin β1: ATTTCCAGATATGCGCTGTTTT

## Claims

1. Use of fibrosis inhibitor for the normalization of culture of corneal endothelial cells, wherein said fibrosis inhibitor comprises a transforming growth factor (TGF) β signal inhibiting agent.

2. The use according to claim 1, wherein said normalization of culture comprises a cellular function being normal, the cellular function being selected from the group consisting of ZO-1 and Na⁺/K⁺-ATPase.

3. The use according to claim 1 or 2, wherein said normalization of culture is for manufacturing a cell for transplantation which adapts to corneal transplantation.

4. The use according to claim 3, wherein said cell for transplantation is a cell of a primate, preferably human.

5. The use according to any one of claims 1-4, wherein said TGF-β signal inhibiting agent is an antagonist of a TGF-β, an antagonist of a TGF-β receptor, or an inhibitor of Smad3.

6. The use according to any one of claims 1-5, wherein said TGF-β signal inhibiting agent comprises at least one of 4-[4-(1,3-benzodioxol-5-yl)-5-(2-pyridinyl)-1H-imidazol-2-yl]benzamide (SB431542), BMP-7, anti-TGF-β antibody, anti-TGF-β receptor antibody, siRNA of a TGF-β, siRNA of a TGF-β receptor, an antisense oligonucleotide of a TGF-β, 6,7-dimethoxy-2-((2E)-3-(1-methyl-2-phenyl-1H-pyrrolo[2,3-b]pyridine-3-yl-prop-2-enoyl))-1,2,3,4-tetrahydroisoquinolone, 3-(6-methyl-2-pyridinyl)-N-phenyl-4-(4-quinolinyl)-1H-pyrazole-1-carbothioamide (A83-01), 2-(3-(6-methylpyridine-2-yl)-1H-pyrazole-4-yl)-1,5-naphthyridine, 6-(4-(piperidine-1-yl)ethoxy)phenyl)-3-(pyridine-4-yl)pyrazolo[1,5-a]pyrimidine (LDN-193189), 2-(5-chloro-2-fluorophenyl)-4-[(4-pyridinyl)amino]pteridine (SD-208), 4-[3-(2-pyridinyl)-1H-pyrazole-4-yl]-quinoline (LY364947), a pharmaceutically acceptable salt or a solvate thereof, or a solvate of a pharmaceutically acceptable salt thereof, preferably 4-[4-(1,3-benzodioxol-5-yl)-5-(2-pyridinyl)-1H-imidazol-2-yl]benzamide (SB431542) or a pharmaceutically acceptable salt thereof.

7. The use according to claims 1-6, wherein said fibrosis inhibitor further comprises a p38 MAP kinase inhibiting agent, preferably 4-[4-(4-fluorophenyl)-2-(4-methylsulfinylphenyl)-1H-imidazole-5-yl]pyridine (SB203580) or a pharmaceutically acceptable salt thereof.

8. The use according to claim 1, further comprising 4-[4-(1,3-benzodioxol-5-yl)-5-(2-pyridinyl)-1H-imidazol-2-yl]benzamide (SB431542) or a pharmaceutically acceptable salt thereof, and 4-[4-(4-fluorophenyl)-2-(4-methylsulfinylphenyl)-1H-imidazole-5-yl]pyridine) (SB203580) or a pharmaceutically acceptable salt thereof.

9. The use according to claim 1, further comprising a cell adhesion promoting agent, preferably (R)-(+)-trans-(4-pyridyl)-4-(1-aminoethyl)-cyclohexanecarboxamide (Y-27632) or a pharmaceutically acceptable salt thereof.

10. The use according to claim 9, wherein said fibrosis inhibitor is allowed to be present at all times during the culturing of said corneal endothelial cell, while said adhesion promoting agent is allowed to be present for a certain period of time, and then the adhesion promoting agent is once deleted, and the cell adhesion promoting agent is once again allowed to be present for a certain period of time.

11. The use according to claim 9, wherein both of said fibrosis inhibitor and said cell adhesion promoting agent are allowed to be present at all times during the culturing of said corneal endothelial cell.

12. The use according to claim 3, wherein said cell for transplantation is for the prevention or treatment of corneal endothelial damage.

13. Use of a culture medium for the normalization of culture of corneal endothelial cells, said culture medium comprising the fibrosis inhibitor as defined in any one of claims 1-12 and a culturing ingredient of corneal endothelium.

14. A method for the normalization of culture of corneal endothelial cells, comprising the step of culturing a corneal endothelial cell using the fibrosis inhibitor as defined in any one of claims 1-12.

15. Use of a preservation solution for preserving a corneal endothelial cell *in vitro,* said preservation solution comprising the fibrosis inhibitor as defined in any one of claims 1-12.

16. A method for manufacturing corneal endothelial cells suitable for treating or preventing a corneal endothelial disease, damage or condition, preferably of a primate, more preferably of a human, preferably bullous keratopathy or corneal endotheliitis, the corneal endothelial cell being preferably derived from a primate, more preferably from a human, the method being **characterized by** comprising a step of culturing corneal endothelial cells using the fibrosis inhibitor for normalization of the cell culture as defined in any one of claims 1-12.

17. The method according to claim 16, wherein said corneal endothelial cells are in the form of a sheet or a suspended substance.

18. The method according to claim 16 or 17, further comprising a cell adhesion promoting agent, preferably (R)-(+)-trans-(4-pyridyl)-4-(1-aminoethyl)-cyclohexanecarboxamide or a pharmaceutically acceptable salt thereof.

## Patentansprüche

1. Verwendung eines Fibroseinhibitors zur Normalisierung einer Kultivierung von Hornhautendothelzellen, wobei der Fibroseinhibitor ein transformierenden Wachstumsfaktor (TGF)-β-Signal-inhibierendes Mittel ist.

2. Verwendung nach Anspruch 1, wobei die Normalisierung der Kultivierung umfasst, dass eine Zellfunktion normal ist, wobei die Zellfunktion ausgewählt ist aus der Gruppe, bestehend aus ZO-1 und Na⁺/K⁺-ATPase.

3. Verwendung nach Anspruch 1 oder 2, wobei die Normalisierung der Kultivierung zum Herstellen einer Zelle zur Transplantation dient, welche für eine Hornhauttransplantation angepasst ist.

4. Verwendung nach Anspruch 3, wobei die Zelle zur Transplantation eine Primatenzelle, bevorzugt eine menschliche Zelle ist.

5. Verwendung nach einem der Ansprüche 1-4, wobei das TGF-β-Signal-inhibierende Mittel ein Antagonist eines TGF-β, ein Antagonist eines TGF-β-Rezeptors oder ein Inhibitor von Smad3 ist.

6. Verwendung nach einem der Ansprüche 1-5, wobei das TGF-β-Signal-inhibierende Mittel wenigstens eines umfasst von 4-[4-(1,3-Benzodioxol-5-yl)-5-(2-pyridinyl)-1H-imidazol-2-yl]benzamid (SB431542), BMP-7, anti-TGF-β-Antikörper, anti-TGF-β-Rezeptor-Antikörper, siRNA eines TGF-β, siRNA eines TGF-β-Rezeptors, ein Antisense-Oligonucleotid eines TGF-β, 6,7-Dimethoxy-2-((2E)-3-(1-methyl-2-phenyl-1H-pyrrolo[2,3-b]pyridin-3-yl-prop-2-enoyl))-1,2,3,4-tetrahydroisochinolon, 3-(6-Methyl-2-pyridinyl)-N-phenyl-4-(4-chinolinyl)-1H-pyrazol-1-carbothioamid (A83-01), 2-(3-(6-Methylpyridin-2-yl)-1H-pyrazol-4-yl)-1,5-naphthyridin, 6-(4-(Piperidin-1-yl)ethoxy)phenyl)-3-(pyridin-4-yl)pyrazolo[1,5-a]pyrimidin (LDN-193189), 2-(5-Chlor-2-fluorophenyl)-4-[(4-pyridinyl)amino]pteridin (SD-208), 4-[3-(2-Pyridinyl)-1H-pyrazol-4-yl]-chinolin (LY364947), ein pharmazeutisch akzeptables Salz oder Solvat davon, oder ein Solvat eines pharmazeutisch akzeptablen Salzes davon, bevorzugt 4-[4-(1,3-Benzodioxol-5-yl)-5-(2-pyridinyl)-1H-imidazol-2-yl]benzamid (SB431542) oder ein pharmazeutisch akzeptables Salz davon.

7. Verwendung nach Ansprüchen 1-6, wobei der Fibroseinhibitor weiterhin ein p38-MAP-Kinase-inhibierendes Mittel umfasst, bevorzugt 4-[4-(4-Fluorophenyl)-2-(4-methylsulfinylphenyl)-1H-imidazol-5-yl]pyridin (SB203580) oder ein pharmazeutisch akzeptables Salz davon.

8. Verwendung nach Anspruch 1, weiterhin umfassend 4-[4-(1,3-Benzodioxol-5-yl)-5-(2-pyridinyl)-1H-imidazol-2-yl]benzamid (SB431542) oder ein pharmazeutisch akzeptables Salz davon, und 4-[4-(4-Fluorophenyl)-2-(4-methylsulfinylphenyl)-1H-imidazol-5-yl]pyridin) (SB203580) oder ein pharmazeutisch akzeptables Salz davon.

9. Verwendung nach Anspruch 1, weiterhin umfassend ein zelladhäsionsförderndes Mittel, bevorzugt (R)-(+)-trans-(4-Pyridyl)-4-(1-aminoethyl)-cyclohexanecarboxamid (Y-27632) oder ein pharmazeutisch akzeptables Salz davon.

10. Verwendung nach Anspruch 9, wobei ermöglicht wird, dass der Fibroseinhibitor während der Kultivierung der Hornhautendothelzelle jederzeit vorliegt, während gleichzeitig ermöglicht wird, dass das adhäsionsfördernde Mittel für eine bestimmte Zeitspanne vorliegt und dann das adhäsionsfördernde Mittel einmal nicht vorhanden ist und dann erneut ermöglicht wird, dass das zelladhäsionsfördernde Mittel für eine bestimmte Zeitspanne vorliegt.

11. Verwendung nach Anspruch 9, wobei ermöglicht wird, dass sowohl der Fibroseinhibitor als auch das zelladhäsionsfördernde Mittel jederzeit während der Kultivierung der Hornhautendthelzelle vorliegt.

12. Verwendung nach Anspruch 3, wobei die Zelle zur Transplantation zur Verhinderung oder Behandlung einer Hornhautendothelschädigung dient.

13. Verwendung eines Kulturmediums zur Normalisierung der Kultivierung von Hornhautendothelzellen, wobei das Kulturmedium den Fibroseinhibitor, wie in einem der Ansprüche 1-12 definiert, und ein Kultivierungsinhaltsstoff von Hornhautendothel umfasst.

14. Verfahren zur Normalisierung der Kultivierung von Hornhautendothelzellen, umfassend den Schritt der Kultivierung einer Hornhautendothelzelle unter Verwendung des Fibroseinhibitors, wie in einem der Ansprüche 1-12 definiert.

15. Verwendung einer Konservierungslösung zum Konservieren einer Hornhautendothelzelle *in vitro,* wobei die Konservierungslösung den Fibroseinhibitor, wie in einem der Ansprüche 1-12 definiert, umfasst.

16. Verfahren zum Herstellen von Hornhautendothelzellen, die zur Behandlung oder Verhinderung einer Hornhautendothelerkrankung, -schädigung oder -zustands, bevorzugt eines Primaten, bevorzugter eines Menschen, geeignet sind, bevorzugt von bullöser Keratopathie oder Hornhaut-Endotheliitis, wobei die Hornhautendothelzelle bevorzugt aus einem Primaten, bevorzugter aus einem Menschen stammt, wobei das Verfahren dadurch charakterisiert ist, dass es einen Schritt der Kultivierung von Hornhautendothelzellen unter Verwendung des Fibroseinhibitors zur Normalisierung der Zellkultur, wie in einem der Ansprüche 1-12, umfasst.

17. Verfahren nach Anspruch 16, wobei die Hornhautendothelzellen in der Form eines Blattes oder einer suspendierten Substanz sind.

18. Verfahren nach Anspruch 16 oder 17, weiterhin umfassend ein zelladhäsionsförderndes Mittel, bevorzugt (R)-(+)-trans-(4-Pyridyl)-4-(1-aminoethyl)-cyclohexancarboxamid oder eines pharmazeutisch akzeptablen Salzes davon.

## Revendications

1. Utilisation d'un inhibiteur de fibrose pour la normalisation de la culture de cellules endothéliales cornéennes, dans laquelle ledit inhibiteur de fibrose comprend un agent inhibant le signal du facteur de croissance transformant (TGF) β.

2. Utilisation selon la revendication 1, dans laquelle ladite normalisation de la culture comprend une fonction cellulaire qui est normale, la fonction cellulaire étant choisie dans le groupe constitué par ZO-1 et Na⁺/K⁺-ATPase.

3. Utilisation selon la revendication 1 ou 2, dans laquelle ladite normalisation de la culture est destinée à la fabrication d'une cellule pour la transplantation qui s'adapte à la transplantation cornéenne.

4. Utilisation selon la revendication 3, dans laquelle ladite cellule pour la transplantation est une cellule d'un primate, de préférence d'un être humain.

5. Utilisation selon l'une quelconque des revendications 1 à 4, dans laquelle ledit agent inhibant le signal de TGF-β est un antagoniste d'un TGF-β, un antagoniste d'un récepteur TGF-β ou un inhibiteur de Smad3.

6. Utilisation selon l'une quelconque des revendications 1 à 5, dans laquelle ledit agent inhibant le signal de TGF-β comprend au moins l'un parmi le 4-[4-(1,3-benzodioxol-5-yl)-5-(2-pyridinyl)]-1H-imidazol-2-yl]benzamide (SB431542), BMP-7, un anticorps anti-TGF-β, un anticorps anti-récepteur TGF-β, un siARN d'un TGF-β, un siARN d'un récepteur TGF-β, un oligonucléotide antisens d'un TGF-β, la 6,7-diméthoxy-2-((2E)-3-(1-méthyl-2-phényl-1H-pyrrolo[2,3-b]pyridin-3-yl-prop-2-énoyl))-1,2,3,4-tétrahydroisoquinolone, le 3-(6-méthyl-2-pyridinyl)-N-phényl-4-(4-quinoléinyl)-1H-pyrazole-1-carbothioamide (A83-01), la 2-(3-(6-méthylpyridin-2-yl)-1H-pyrazol-4-yl)-1,5-naphthyridine, la 6-(4-(pipéridin-1-yl)éthoxy)phényl)-3-(pyridine-4-yl)pyrazolo[1,5-a]pyrimidine (LDN-193189), la 2-(5-chloro-2-fluorophényl)-4-[(4-pyridinyl)amino]ptéridine (SD-208), la 4-[3-(2-pyridinyl)-1H-pyrazol-4-yl]-quinoléine (LYS364947), un sel pharmaceutiquement acceptable ou un solvate de celle-ci, ou un solvate d'un sel pharmaceutiquement acceptable de celle-ci, de préférence le 4-[4-(1,3-benzodioxol-5-yl)-5-(2-pyridinyl)]-1H-imidazol-2-yl]benzamide (SB431542) ou un sel pharmaceutiquement acceptable de celui-ci.

7. Utilisation selon les revendications 1 à 6, dans laquelle ledit inhibiteur de fibrose comprend en outre un agent inhibant la p38 MAP kinase, de préférence la 4-[4-(4-fluorophényl)-2-(4-méthylsulfinylphényl)-1H-imidazol-5-yl]pyridine (SB203580) ou un sel pharmaceutiquement acceptable de celle-ci.

8. Utilisation selon la revendication 1, comprenant en outre le 4-[4-(1,3-benzodioxol-5-yl)-5-(2-pyridinyl)-1H-imidazol-2-yl]benzamide (SB431542) ou un sel pharmaceutiquement acceptable de celui-ci et la 4-[4-(4-fluorophényl)-2-(4-méthylsulfinylphényl)-1H-imidazol-5-yl]pyridine (SB203580) ou un sel pharmaceutiquement acceptable de celle-ci.

9. Utilisation selon la revendication 1, comprenant en outre un agent favorisant l'adhésion cellulaire, de préférence le (R)-(+)-trans-(4-pyridyl)-4-(1-aminoéthyl)-cyclohexanecarboxamide (Y-27632) ou un agent pharmaceutiquement acceptable sel de celui-ci.

10. Utilisation selon la revendication 9, dans laquelle ledit inhibiteur de fibrose est autorisé à être présent à tout moment pendant la culture de ladite cellule endothéliale cornéenne, tandis que ledit agent favorisant l'adhésion est autorisé à être présent pendant une certaine période de temps, et ensuite l'agent favorisant l'adhésion est une fois supprimé, et l'agent favorisant l'adhésion cellulaire est à nouveau autorisé à être présent pendant une certaine période de temps.

11. Utilisation selon la revendication 9, dans laquelle à la fois ledit inhibiteur de fibrose et ledit agent favorisant l'adhésion cellulaire peuvent être autorisés à être présents à tout moment pendant la culture de ladite cellule endothéliale cornéenne.

12. Utilisation selon la revendication 3, dans laquelle ladite cellule pour la transplantation est destinée à la prévention ou au traitement des lésions endothéliales cornéennes.

13. Utilisation d'un milieu de culture pour la normalisation de la culture de cellules endothéliales cornéennes, ledit milieu de culture comprenant l'inhibiteur de fibrose tel que défini dans l'une quelconque des revendications 1 à 12 et un ingrédient de culture d'endothélium cornéen.

14. Procédé de normalisation de la culture de cellules endothéliales cornéennes, comprenant l'étape de culture d'une cellule endothéliale cornéenne en utilisant l'inhibiteur de fibrose tel que défini dans l'une quelconque des revendications 1 à 12.

15. Utilisation d'une solution de conservation pour conserver une cellule endothéliale cornéenne *in vitro,* ladite solution de conservation comprenant l'inhibiteur de fibrose tel que défini dans l'une quelconque des revendications 1 à 12.

16. Procédé de fabrication d'un médicament destiné au traitement ou à la prévention d'une maladie, d'une lésion ou d'une affection endothéliale cornéenne, de préférence d'un primate, plus préférablement d'un être humain, de préférence une kératopathie bulleuse ou une endothélite cornéenne, le médicament comprenant une cellule endothéliale cornéenne dérivée de préférence d'un primate, plus préférablement d'un être humain, ladite cellule endothéliale cornéenne étant produite en utilisant un procédé de culture de manière normale d'une cellule endothéliale cornéenne, comprenant l'étape de culture d'une cellule endothéliale cornéenne en utilisant l'inhibiteur de fibrose tel que défini dans l'une quelconque des revendications 1 à 12.

17. Procédé selon la revendication 16, dans lequel lesdites cellules endothéliales cornéennes sont sous la forme d'une feuille ou d'une substance en suspension.

18. Procédé selon la revendication 16 ou 17, comprenant en outre un agent favorisant l'adhésion cellulaire, de préférence le (R)-(+)-trans-(4-pyridyl)-4-(1-aminoéthyl)-cyclohexanecarboxamide ou un sel pharmaceutiquement acceptable de celui-ci.


### SEQUENCE LISTING

<110> Kyoto Prefectural Public University corporation The Doshisha Senju Phamaceutical Co.,Ltd.
<120> Normalization of cultured corneal endothelial cells
<130> DS001PCT
<150> JP 2011-289666
   <151> 2011-12-28
<150> JP 2012-30969
   <151> 2012-02-15
<150> JP 2012-151340
   <151> 2012-07-05
<160> 18
<170> PatentIn version 3.5
<210> 1
   <211> 32
   <212> DNA
   <213> Artificial sequence
<220>
   <223> Na+K+-ATPase Fw primer
<400> 1
   cttcctccgc atttatgctc attttctcac cc 32
<210> 2
   <211> 31
   <212> DNA
   <213> Artificial sequence
<220>
   <223> Na+K+-ATPase Rv primer
<400> 2
   ggatgatcat aaacttagcc ttgatgaact c 31
<210> 3
   <211> 20
   <212> DNA
   <213> Artificial sequence
<220>
   <223> ZO-1 Fw primer
<400> 3
   ggacgaggca tcatccctaa 20
<210> 4
   <211> 20
   <212> DNA
   <213> Artificial sequence
<220>
   <223> ZO-1 Rv primer
<400> 4
   ccagcttctc gaagaaccac 20
<210> 5
   <211> 20
   <212> DNA
   <213> Artificial sequence
<220>
   <223> GAPDH Fw primer
<400> 5
   gagtcaacgg atttggtcgt 20
<210> 6
   <211> 20
   <212> DNA
   <213> Artificial sequence
<220>
   <223> GAPDH Rv primer
<400> 6
   ttgattttgg agggatctcg 20
<210> 7
   <211> 24
   <212> DNA
   <213> Artificial sequence
<220>
   <223> Collagen 1 Fw Primer
<400> 7
   tcggcgagag catgaccgat ggat 24
<210> 8
   <211> 24
   <212> DNA
   <213> Artificial sequence
<220>
   <223> Collagen 1 Rv Primer
<400> 8
   gacgctgtag gtgaagcggc tgtt 24
<210> 9
   <211> 22
   <212> DNA
   <213> Artificial sequence
<220>
   <223> Collagen 4 Fw Primer
<400> 9
   agcaaggtgt tacaggattg gt 22
<210> 10
   <211> 22
   <212> DNA
   <213> Artificial sequence
<220>
   <223> Collagen 4 Rv Primer
<400> 10
   agaaggacac tgtgggtcat ct 22
<210> 11
   <211> 26
   <212> DNA
   <213> Artificial sequence
<220>
   <223> Collagen 8 Fw Primer
<400> 11
   atgtgatggc tgtgctgctg ctgcct 26
<210> 12
   <211> 20
   <212> DNA
   <213> Artificial sequence
<220>
   <223> Collagen 8 Rv Primer
<400> 12
   ctcttgggcc aggctctcca 20
<210> 13
   <211> 22
   <212> DNA
   <213> Artificial sequence
<220>
   <223> Fibronectin Fw Primer
<400> 13
   agatgagtgg gaacgaatgt ct 22
<210> 14
   <211> 22
   <212> DNA
   <213> Artificial sequence
<220>
   <223> Fibronectin Rv Primer
<400> 14
   gagggtcaca cttgaattct cc 22
<210> 15
   <211> 22
   <212> DNA
   <213> Artificial sequence
<220>
   <223> integrin alpha 5 Fw Primer
<400> 15
   tcctcagcaa gaatctcaac aa 22
<210> 16
   <211> 22
   <212> DNA
   <213> Artificial sequence
<220>
   <223> integrin alpha 5 Rv Primer
<400> 16
   gttgagtccc gtaactctgg tc 22
<210> 17
   <211> 22
   <212> DNA
   <213> Artificial sequence
<220>
   <223> integrin beta 1 Fw Primer
<400> 17
   gctgaagact atcccattga cc 22
<210> 18
   <211> 22
   <212> DNA
   <213> Artificial sequence
<220>
   <223> integrin beta 1 Rv Primer
<400> 18
   atttccagat atgcgctgtt tt 22